(19) Europäisches Patentamt
European Patent Office
Office européen des brevets

(11) EP 1 921 152 A1

(12) EUROPEAN PATENT APPLICATION

(43) Date of publication:
**14.05.2008 Bulletin 2008/20**

(51) Int Cl.:
***C12N 15/82*** *(2006.01)*

(21) Application number: **07118354.5**

(22) Date of filing: **05.05.2004**

(84) Designated Contracting States:
**AT BE BG CH CY CZ DE DK EE ES FI FR GB GR HU IE IT LI LU MC NL PL PT RO SE SI SK TR**

(30) Priority: **05.05.2003 US 467910 P**
**15.07.2003 US 487273 P**

(62) Document number(s) of the earlier application(s) in accordance with Art. 76 EPC:
**04751446.8 / 1 620 539**

(71) Applicant: **Monsanto Technology, LLC**
**St. Louis, MO 63167 (US)**

(72) Inventors:
- **Benson, Robert**
  **Niantic, CT 06357 (US)**
- **Castiglioni, Paolo**
  **Westerly, RI 02891 (US)**
- **Bell, Erin**
  **Ladue, MO 63124 (US)**
- **Ahrens, Jaffery**
  **Manchester, MO 63021 (US)**
- **Loida, Paul**
  **Kirkwood, MO 63122 (US)**
- **Hinchey, Brendan**
  **Mystic, CT 06355 (US)**
- **Korte, John**
  **Westerly, RI 02891 (US)**

(74) Representative: **Helbing, Jörg**
**Patentanwälte**
**Von Kreisler-Selting-Werner**
**Postfach 10 22 41**
**50462 Köln (DE)**

Remarks:
This application was filed on 12 - 10 - 2007 as a divisional application to the application mentioned under INID code 62.

(54) **Transgenic plants with glycine-betaine specific promoter**

(57) Disclosed herein are transgenic plants and seed having an exogenous DNA which expresses a GB 1 protein that imparts increased glycine-betaine content in plants.

EP 1 921 152 A1

SEQUENCE LISTING

```
<110>  Monsanto Technology, LLC

<120>  Transgenic Plants with Increased Glycine-Betaine

<130>  38-15(52913)D

<150>  EP 04751446.8
<151>  2004-05-05

<150>  US 60/467910
<151>  2003-05-05

<150>  US 60/487273
<151>  2003-07-15

<160>  57

<170>  PatentIn version 3.2

<210>  1
<211>  306
<212>  PRT
<213>  Zea mays

<400>  1
Met Ile Pro Tyr Ala Thr Ala Ala Glu Ala Glu Gly Ala Leu Gly Arg
1               5                   10                  15

Thr Met Thr Trp Ala Glu Thr Ala Trp Tyr Glu Tyr Ser Ala Val Met
            20                  25                  30

Pro Asp Ser Trp Leu His Cys His Thr Thr Phe Ile Leu Phe Val Ile
        35                  40                  45


Tyr Ser Ile Ala Pro Leu Pro Leu Leu Leu Leu Glu Gln Phe Ala Pro
    50                  55                  60

Ser Val Val Leu Pro Tyr Lys Leu Gln Pro Arg Val Arg Leu Pro Pro
65                  70                  75                  80

Ala Ala Ser Leu Ser Cys Tyr Met Asp Ala Ala Cys Ile Phe Pro Leu
                85                  90                  95

Ala Val Gly Leu Gln Phe Val Ser Tyr Pro Ala Val Ala Lys Ile Leu
            100                 105                 110

Arg Thr Arg Met Gly Leu Pro Leu Pro Ser Val Arg Glu Thr Ile Ala
        115                 120                 125

Gln Leu Val Val Tyr Ser Leu Val Glu Asp Tyr Leu Ser Tyr Trp Met
    130                 135                 140

His Arg Leu Leu His Thr Gln Trp Cys Tyr Glu Lys Ile His Arg Val
145                 150                 155                 160

His His Glu Phe Thr Ala Pro Thr Gly Phe Ala Met Ser Tyr Ser His
                165                 170                 175

Trp Ala Glu Asn Val Val Leu Ser Ile Pro Ala Leu Ala Gly Pro Val
            180                 185                 190
```

```
Leu Val Pro Cys His Val Thr Thr Gln Trp Leu Trp Phe Ser Ile Arg
        195                 200                 205

Leu Ile Glu Gly Ile Asn Thr His Ser Gly Tyr His Phe Pro Phe Ser
    210                 215                 220

Pro Cys Arg Leu Ile Pro Phe Tyr Gly Gly Ala Ala Tyr His Asp Tyr
225                 230                 235                 240

His His Tyr Ala Gly Gly Arg Ser Gln Ser Asn Phe Ala Pro Leu Phe
                245                 250                 255

Thr Tyr Cys Asp Tyr Leu Tyr Arg Thr Asp Lys Gly Tyr Arg Tyr His
            260                 265                 270

Lys Leu Lys Gln Glu Lys Leu Lys Ser Leu Ala Glu Asn Ser Ala Asp
        275                 280                 285

Lys Gly Gly Asn Tyr Ser Phe Asp Glu Gly Lys Lys Asn Arg Tyr Phe
    290                 295                 300

Cys Ala
305


<210>  2
<211>  293
<212>  PRT
<213>  Zea mays

<400>  2
Met Met Pro Tyr Gly Thr Ala Ala Glu Ala Glu Ala Ala Leu Gly Arg
1               5                   10                  15

Ser Met Thr Trp Ala Glu Ala Leu Trp Phe Arg Tyr Ser Ala Gly Met
            20                  25                  30

Pro Asp Leu Cys Leu Thr Trp His Val Ser Leu Val Tyr Leu Val Leu
        35                  40                  45

Tyr Ala Leu Val Pro Leu Pro Val Met Val Ile Gln Lys Leu Ala Pro
    50                  55                  60

Gly Tyr Ala Leu Arg His Lys Leu Gln Pro Gly Val Pro Glu Pro Ser
65                  70                  75                  80

Pro Val Ser Thr Tyr Val Glu Tyr Ile Arg Asp Ser Arg Gly Val Thr
                85                  90                  95

Leu Ala Ala Leu Gly Pro Phe Pro Leu Ile Tyr Ser Ile Ala Phe Lys
            100                 105                 110

Leu Phe Gly Val Arg Thr Gly Leu Pro Leu Pro Ser Val Trp Glu Thr
        115                 120                 125

Ala Thr His Leu Ala Val Tyr Ser Leu Val Glu Asp Tyr Thr Ser Tyr
    130                 135                 140

Trp Leu His Arg Phe Leu His Thr Arg Trp Gly Tyr Glu Lys Ile His
145                 150                 155                 160

Arg Val His His Glu Lys Thr Ala Pro Ser Gly Phe Ala Ala Ala Tyr
```

```
                165                 170                 175

Ala Thr Gly Thr Glu Leu Ser Leu Tyr Leu Thr Thr Leu Phe Leu Gly
            180                 185                 190

Pro Ala Ile Val Pro Ser His Val Thr Thr His Trp Leu Leu Phe Ser
        195                 200                 205

Ile Arg Ile Met Glu Ala Phe Asp Thr His Ser Gly Tyr His Phe Pro
    210                 215                 220

Phe Ser Leu Ala Arg Phe Ile Pro Phe Tyr Gly Gly Ala Glu Phe His
225                 230                 235                 240

Asp Tyr His His Tyr Ala Gly Glu Lys Thr Arg Ser Asn Phe Ser Ser
                245                 250                 255

Val Phe Thr Tyr Cys Asp Tyr Ile Tyr Gly Thr Asn Lys Gly Tyr Met
            260                 265                 270

Tyr His Lys Arg Ser Leu Ala Glu Leu Lys Thr Lys Glu Ala Glu His
        275                 280                 285

Ser Gly Lys Glu Asp
    290


<210>  3
<211>  284
<212>  PRT
<213>  Oryza sativa

<400>  3
Met Leu Pro Tyr Ala Thr Ala Ala Glu Ala Glu Ala Ala Val Gly Arg
1               5                   10                  15

Gly Leu Thr Trp Ala Glu Ala Ala Trp Phe Arg Tyr Ser Ala Ala Ile
            20                  25                  30

Pro Asp Tyr Cys Leu Tyr Cys His Asn Val Pro Ile Leu Leu Leu Val
        35                  40                  45

Tyr Thr Leu Ala Pro Leu Pro Leu Ala Leu Leu Glu Leu Arg Arg His
    50                  55                  60

Leu Pro Leu Pro His Lys Leu Gln Pro Gly Val Arg His Pro Pro Ala
65                  70                  75                  80

Ala Phe Leu Arg Cys Tyr Ala Ala Thr Ala Arg Val Leu Leu Leu Ala
                85                  90                  95

Val Gly Pro Val Gln Leu Ala Ser Phe Pro Ala Val Arg Ala Val Gly
            100                 105                 110

Ile Arg Thr Gly Leu Pro Leu Pro Ser Ala Gly Glu Thr Ala Ala Gln
        115                 120                 125

Val Ala Val Tyr Leu Leu Val Glu Asp Tyr Leu Gly Tyr Trp Ile His
    130                 135                 140

Arg Leu Leu His Thr Pro Trp Ala Tyr His His Ile His Arg Val His
145                 150                 155                 160
```

```
His Glu Phe Thr Ala Pro Met Gly Tyr Ala Ala Pro Tyr Ala His Trp
                165                 170                 175

Ala Glu Ile Leu Ile Leu Gly Phe Pro Ala Phe Ala Gly Pro Ala Ile
            180                 185                 190

Val Pro Cys His Met Thr Thr Phe Trp Leu Trp Phe Val Leu Arg His
        195                 200                 205

Leu Glu Ala Ile His Ile His Ser Gly Phe Lys Leu Pro Phe Asp Pro
    210                 215                 220

Thr Lys Tyr Ile Pro Leu Tyr Gly Gly Val Glu Tyr His Asp Tyr His
225                 230                 235                 240

His Phe Val Gly Gly His Ser Gln Ser Asn Phe Ser Ser Val Phe Thr
                245                 250                 255

Phe Cys Asp Tyr Ile Tyr Gly Thr Asp Arg Gly Tyr Arg Tyr His Lys
            260                 265                 270

Ala Ser Leu Ser Lys Met Arg Ile Phe Val Arg Ala
        275                 280


<210>  4
<211>  301
<212>  PRT
<213>  Hordeum vulgare

<220>
<221>  misc_feature
<222>  (185)..(185)
<223>  Xaa can be any naturally occurring amino acid

<400>  4
Met Leu Pro Tyr Ala Thr Thr Gly Asp Ala Glu Ala Ala Leu Gly Arg
1               5                   10                  15

Ala Leu Thr Trp Ala Glu Ala Ala Trp Leu Arg Tyr Ser Ala Ser Val
            20                  25                  30

Pro Asp Arg Tyr Leu His Trp Pro Asn Ile Ala Ile Thr Leu Val Val
        35                  40                  45

Tyr Thr Leu Ala Pro Leu Pro Leu Ala Leu Phe Asp Leu Ala Ala Pro
    50                  55                  60

Ala Val Ala Ala Pro Tyr Lys Leu Gln Pro Lys Val Gln His Pro Pro
65                  70                  75                  80

Ala Thr Phe Phe Arg Cys Tyr Met Asp Ala Val Arg Val Ser Leu Leu
                85                  90                  95

Ile Ile Gly Pro Tyr Gln Leu Ile Ser Tyr Pro Ala Ala Lys Ile Met
            100                 105                 110

Asp Ile Arg Thr Gly Leu Pro Leu Pro Ser Met Gly Glu Ile Ala Ala
        115                 120                 125

Gln Leu Thr Val Tyr Phe Leu Val Glu Asp Tyr Leu Asn Tyr Trp Leu
    130                 135                 140
```

```
His Arg Leu Leu His Thr Lys Trp Cys Tyr Glu Lys Ile His His Val
145                 150                 155                 160

His His Glu Phe Thr Ala Pro Met Ala Tyr Ala Ala Trp Tyr Gly His
                165                 170                 175

Trp Ala Glu Met Leu Ile Leu Ala Xaa Pro Ser Leu Ala Gly Pro Ala
            180                 185                 190

Leu Val Pro Cys His Val Thr Thr Leu Trp Ile Trp Phe Ala Ala Arg
        195                 200                 205

Leu Val Glu Ser Leu Asn Ile His Ser Gly Phe Lys Leu Pro Phe Asn
    210                 215                 220

Ala Glu Lys Tyr Ile Pro Phe Tyr Gly Gly Ala Glu His His Asp Tyr
225                 230                 235                 240

His His Tyr Ile Gly Gly Gln Ser Lys Ser Asn Phe Ala Pro Val Phe
                245                 250                 255

Thr Tyr Cys Asp Tyr Ile Tyr Gly Thr Asp Lys Gly Tyr Arg Tyr His
            260                 265                 270

Lys Ala Thr Leu Ala Lys Leu Lys Glu Leu Ala Gly Asn Glu Val Gln
        275                 280                 285

Lys Gly Val Asp Asn Gly Phe Asn Ser Gly Lys Gln Glu
    290                 295                 300


<210>  5
<211>  301
<212>  PRT
<213>  Zea mays

<400>  5
Met Leu Pro Tyr Ala Thr Ala Ala Glu Ala Glu Ala Ala Leu Gly Arg
1               5                   10                  15

Pro Met Thr Pro Ala Glu Ala Leu Trp Phe Arg Tyr Thr Ala Gly Val
            20                  25                  30

Ser Asp Tyr His Leu Tyr Cys Cys Asn Ile Leu Phe Leu Phe Val Val
        35                  40                  45

Phe Thr Val Ala Pro Leu Pro Ile Ala Leu Leu Glu Leu Arg Ala Pro
    50                  55                  60

Ala Ala Val Ser Pro Tyr Lys Leu Gln Pro Arg Val Arg Leu Ser Arg
65                  70                  75                  80

Ala Glu Phe Val Arg Cys Tyr Lys Asp Val Leu Arg Ile Phe Phe Leu
                85                  90                  95

Val Ile Gly Pro Leu Gln Leu Val Ser Tyr Pro Ala Val Lys Phe Val
            100                 105                 110

Gly Ile His Thr Lys Leu Pro Leu Pro Ser Leu Ala Glu Leu Ala Ala
        115                 120                 125

Gln Leu Leu Val Tyr Phe Leu Val Glu Asp Tyr Leu Asn Tyr Trp Ile
    130                 135                 140
```

```
His Arg Phe Leu His Gly Glu Trp Gly Tyr Gln Asn Ile His Arg Val
145                 150                 155                 160

His His Glu Phe Thr Ala Pro Ile Gly Phe Ala Ala Pro Tyr Ala His
                165                 170                 175

Trp Ala Glu Val Leu Ile Leu Gly Ile Pro Ser Phe Val Gly Pro Ala
            180                 185                 190

Ile Val Pro Gly His Met Ile Thr Phe Trp Leu Trp Ile Ile Leu Arg
        195                 200                 205

Gln Val Glu Ala Ile Glu Thr His Ser Gly Phe Asp Phe Pro Phe Thr
    210                 215                 220

Pro Thr Lys Tyr Ile Pro Phe Tyr Gly Gly Ala Glu Tyr His Asp Tyr
225                 230                 235                 240

His His Tyr Val Gly Gly Gln Ser Gln Ser Asn Phe Ala Ser Val Phe
                245                 250                 255

Thr Tyr Cys Asp Tyr Leu Tyr Gly Thr Asp Lys Gly Tyr Arg Phe His
            260                 265                 270

Lys Thr Tyr Leu Ala Lys Leu Lys Asp Leu Gly His Asn Asp Gly Gln
        275                 280                 285

Lys Gly Asp Gly Ser Gly Pro Ser Tyr Val Lys Leu Asp
    290                 295                 300


<210>  6
<211>  299
<212>  PRT
<213>  Allium porrum

<400>  6
Met Ile Pro Tyr Pro Ser Leu Thr Ala Ala Glu Ala Ala Leu Asn Arg
1               5                   10                  15

Pro Leu Thr Tyr Ala Glu Thr Ile Trp Phe Asn Tyr Ser Ala Thr Ile
            20                  25                  30

Pro Asp Pro Leu Leu Tyr Tyr His Asn Thr Ile Phe Leu Phe Val Ile
        35                  40                  45

Phe Thr Leu Val Pro Leu Pro Leu Ala Leu Leu Glu Leu Tyr Trp Pro
    50                  55                  60

Ser Val Leu Lys Pro Phe Lys Ile Gln Pro Lys Val Tyr Leu Ser Lys
65                  70                  75                  80

Ser Glu Phe Leu Glu Cys Tyr Lys Asn Val Ile Lys Val Phe Phe Leu
                85                  90                  95

Val Val Cys Pro Leu Gln Leu Leu Ser Tyr Pro Thr Val Lys Phe Val
            100                 105                 110

Gly Ile Arg Thr Gly Leu Pro Leu Pro Ser Val Trp Glu Val Ala Ser
        115                 120                 125

Gln Leu Ala Val Tyr Phe Leu Leu Glu Asp Phe Gly Asn Tyr Trp Ile
```

```
        130                 135                 140

His Arg Trp Leu His Gly Lys Trp Gly Tyr Glu Lys Ile His Lys Val
145                 150                 155                 160

His His Glu Tyr Thr Ala Pro Ile Gly Phe Ala Ala Pro Tyr Ala His
                165                 170                 175

Trp Ala Glu Val Leu Ile Leu Gly Ile Pro Ser Phe Leu Gly Pro Ala
            180                 185                 190

Ile Val Pro Gly His Met Ile Thr Leu Trp Leu Trp Ile Ala Leu Arg
        195                 200                 205

Gln Ile Glu Ala Leu Asp Thr His Ser Gly Tyr Asp Phe Pro Leu Ser
    210                 215                 220

Phe Thr Lys Phe Ile Pro Phe Tyr Gly Gly Ala Glu Tyr His Asp Tyr
225                 230                 235                 240

His His Tyr Val Gly Gly Gln Ser Gln Ser Asn Phe Ala Ser Val Phe
                245                 250                 255

Thr Tyr Cys Asp Tyr Val Tyr Gly Thr Asp Lys Gly Tyr Arg Tyr Arg
            260                 265                 270

Lys Ala Cys Leu Ser Met Met Lys Glu Glu Ser Glu Asn Gln Asn Gly
        275                 280                 285

Val Glu Asn Ser Phe Gln Asn Gln Lys Ser Asp
    290                 295


<210>  7
<211>  298
<212>  PRT
<213>  Arabidopsis thaliana

<400>  7
Met Ile Pro Tyr Ala Thr Val Glu Glu Ala Ser Ile Ala Leu Gly Arg
1               5                   10                  15

Asn Leu Thr Arg Leu Glu Thr Leu Trp Phe Asp Tyr Ser Ala Thr Lys
            20                  25                  30

Ser Asp Tyr Tyr Leu Tyr Cys His Asn Ile Leu Phe Leu Phe Leu Val
        35                  40                  45

Phe Ser Leu Val Pro Leu Pro Leu Val Phe Val Glu Leu Ala Arg Ser
    50                  55                  60

Ala Ser Gly Leu Phe Asn Arg Tyr Lys Ile Gln Pro Lys Val Asn Tyr
65                  70                  75                  80

Ser Leu Ser Asp Met Phe Lys Cys Tyr Lys Asp Val Met Thr Met Phe
                85                  90                  95

Ile Leu Val Val Gly Pro Leu Gln Leu Val Ser Tyr Pro Ser Ile Gln
            100                 105                 110

Met Ile Glu Ile Arg Ser Gly Leu Pro Leu Pro Thr Ile Thr Glu Met
        115                 120                 125
```

```
Leu Ser Gln Leu Val Val Tyr Phe Leu Ile Glu Asp Tyr Thr Asn Tyr
    130                 135                 140

Trp Val His Arg Phe Phe His Ser Lys Trp Gly Tyr Asp Lys Ile His
145                 150                 155                 160

Arg Val His His Glu Tyr Thr Ala Pro Ile Gly Tyr Ala Ala Pro Tyr
                165                 170                 175

Ala His Trp Ala Glu Val Leu Leu Leu Gly Ile Pro Thr Phe Met Gly
            180                 185                 190

Pro Ala Ile Ala Pro Gly His Met Ile Thr Phe Trp Leu Trp Ile Ala
        195                 200                 205

Leu Arg Gln Met Glu Ala Ile Glu Thr His Ser Gly Tyr Asp Phe Pro
    210                 215                 220

Trp Ser Pro Thr Lys Tyr Ile Pro Phe Tyr Gly Gly Ala Glu Tyr His
225                 230                 235                 240

Asp Tyr His His Tyr Val Gly Gly Gln Ser Gln Ser Asn Phe Ala Ser
                245                 250                 255

Val Phe Thr Tyr Cys Asp Tyr Ile Tyr Gly Thr Asp Lys Gly Tyr Arg
            260                 265                 270

Phe Gln Lys Lys Leu Leu Glu Gln Ile Lys Glu Ser Ser Lys Lys Ser
        275                 280                 285

Asn Lys His Asn Gly Gly Ile Lys Ser Asp
    290                 295


<210>  8
<211>  297
<212>  PRT
<213>  Arabidopsis thaliana

<400>  8
Met Ile Pro Tyr Ala Thr Ile Glu Glu Ala Ser Ile Ala Leu Ser Arg
1               5                   10                  15

Asn Leu Thr Trp Leu Glu Thr Leu Trp Phe Asp Tyr Ser Ala Thr Lys
            20                  25                  30

Ser Asp Tyr Tyr Leu Tyr Cys His Asn Ile Leu Phe Leu Phe Leu Ile
        35                  40                  45

Phe Ser Leu Val Pro Leu Pro Leu Val Phe Ile Glu Ser Ser Gln Ser
    50                  55                  60

Thr Ser Asp Leu Phe Asn Arg Tyr Lys Ile Gln Pro Lys Val Lys Asn
65                  70                  75                  80

Ser Phe Ser Ser Met Phe Lys Cys Tyr Lys Asp Val Met Lys Met Phe
                85                  90                  95

Ile Leu Val Val Gly Pro Leu Gln Leu Val Ser Tyr Pro Ser Ile Gln
            100                 105                 110

Val Asp Phe Val Phe Arg Val Leu Lys Gln Met Ile Glu Ile Arg Ser
        115                 120                 125
```

```
Gly Leu Pro Leu Pro Ser Cys Met Glu Ile Val Ala Gln Leu Val Val
    130                 135                 140

Tyr Phe Leu Val Glu Asp Tyr Thr Asn Tyr Trp Val His Arg Phe Phe
145                 150                 155                 160

His Cys Lys Trp Gly Tyr Glu Lys Phe His His Ile His His Glu Tyr
                165                 170                 175

Thr Ala Pro Ile Gly Tyr Ala Ala Pro Tyr Ala His Trp Ala Glu Val
            180                 185                 190

Leu Leu Leu Gly Ile Pro Thr Phe Leu Gly Pro Ala Ile Ala Pro Gly
        195                 200                 205

His Met Ile Thr Phe Trp Leu Trp Ile Ala Leu Arg Gln Ile Glu Ala
    210                 215                 220

Ile Glu Thr His Ser Gly Tyr Asp Phe Pro Trp Ser Leu Thr Lys Tyr
225                 230                 235                 240

Ile Pro Phe Tyr Gly Gly Ala Glu Tyr His Asp Tyr His His Tyr Val
                245                 250                 255

Gly Gly Gln Ser Gln Ser Asn Phe Ala Ser Val Phe Thr Tyr Cys Asp
            260                 265                 270

Tyr Ile Tyr Gly Thr Asp Lys Gly Tyr Arg Phe Gln Lys Lys Leu Leu
        275                 280                 285

Gln Gln Val Asn Lys Tyr Ser Ile Asn
    290                 295


<210>  9
<211>  291
<212>  PRT
<213>  Arabidopsis thaliana

<400>  9
Met Ile Pro Tyr Pro Thr Val Glu Asp Ala Ser Val Ala Leu Gly Arg
1               5                   10                  15

Asn Leu Thr Trp Phe Glu Thr Val Trp Phe Asp Tyr Ser Ala Thr Lys
            20                  25                  30

Ser Asn Phe His Val Tyr Cys His Thr Ile Leu Val Leu Phe Leu Val
        35                  40                  45

Phe Ser Leu Ala Pro Phe Pro Leu Val Ile Val Glu Trp Thr Gly Trp
    50                  55                  60

Phe Asp Gln Phe Lys Ile Gln Lys Lys Val Lys Tyr Ser Leu Ser Asp
65                  70                  75                  80

Met Phe Gln Cys Tyr Lys Glu Val Met Lys Leu Phe Leu Leu Val Val
                85                  90                  95

Gly Thr Leu Gln Ile Val Ser Tyr Pro Ser Ile Gln Met Val Gly Ile
            100                 105                 110

Arg Ser Gly Leu Pro Leu Pro Ser Leu Met Glu Ile Val Ala Gln Leu
```

```
        115                 120                 125

Val Val Tyr Phe Leu Ile Glu Asp Tyr Thr Asn Tyr Trp Ile His Arg
    130                 135                 140

Trp Met His Cys Lys Trp Gly Tyr Glu Lys Ile His Arg Ile His His
145                 150                 155                 160

Glu Tyr Thr Ser Pro Ile Gly Tyr Ala Ser Pro Tyr Ala His Trp Ala
                165                 170                 175

Glu Ile Leu Ile Leu Gly Ile Pro Thr Phe Leu Gly Pro Ala Ile Ala
            180                 185                 190

Pro Gly His Ile Met Thr Phe Trp Leu Trp Ile Ser Leu Arg Gln Phe
        195                 200                 205

Glu Ala Ile Glu Thr His Ser Gly Tyr Asp Phe Pro Trp Ser Val Thr
    210                 215                 220

Lys Leu Ile Pro Phe Tyr Gly Gly Pro Glu Tyr His Asp Tyr His His
225                 230                 235                 240

Tyr Val Gly Gly Gln Ser Gln Ser Asn Phe Ala Ser Val Phe Thr Tyr
                245                 250                 255

Cys Asp Tyr Ile Tyr Gly Thr Asp Lys Gly Tyr Arg Ile His Lys Lys
            260                 265                 270

Leu Leu His His Gln Ile Lys Glu Glu Ala Glu Glu Lys Arg Val Arg
        275                 280                 285

Lys His Asp
    290


<210>  10
<211>  299
<212>  PRT
<213>  Arabidopsis thaliana

<400>  10
Met Ile Pro Tyr Ala Thr Ile Glu Glu Ala Ser Ile Ala Leu Ser Arg
1               5                   10                  15

Asn Leu Thr Trp Leu Glu Thr Leu Trp Phe Asp Tyr Ser Ala Thr Lys
            20                  25                  30

Ser Asp Tyr Tyr Leu Tyr Cys His Asn Ile Leu Phe Leu Phe Leu Ile
        35                  40                  45

Phe Ser Leu Val Pro Leu Pro Leu Val Leu Ile Glu Ser Ala Gln Ser

    50                  55                  60

Thr Ser Asp Leu Phe Asn Arg Tyr Lys Ile Gln Pro Lys Val Lys Asn
65                  70                  75                  80

Ser Phe Ser Ser Met Leu Lys Cys Tyr Lys Asp Val Met Lys Met Phe
                85                  90                  95

Ile Leu Val Val Gly Pro Leu Gln Leu Val Ser Tyr Pro Ser Ile Gln
            100                 105                 110
```

```
Met Ile Glu Ile Arg Ser Gly Leu Pro Leu Pro Ser Cys Met Glu Ile
        115                 120                 125

Val Ala Gln Phe Val Val Tyr Phe Leu Val Glu Asp Tyr Thr Asn Tyr
    130                 135                 140

Trp Val His Arg Phe Phe His Cys Lys Trp Gly Tyr Glu Lys Phe His
145                 150                 155                 160

His Ile His His Glu Tyr Thr Ala Pro Ile Gly Tyr Ala Ala Pro Tyr
                165                 170                 175

Ala His Trp Ala Glu Val Leu Leu Leu Gly Ile Pro Thr Phe Leu Gly
            180                 185                 190

Pro Ala Ile Ala Pro Gly His Met Ile Thr Phe Trp Leu Trp Ile Ala
        195                 200                 205

Leu Arg Gln Ile Glu Ala Ile Glu Thr His Ser Gly Tyr Asp Phe Pro
    210                 215                 220

Trp Ser Leu Thr Lys Tyr Ile Pro Phe Tyr Gly Gly Ala Glu Tyr His
225                 230                 235                 240

Asp Tyr His His Tyr Val Gly Gly Gln Ser Gln Ser Asn Phe Ala Ser
                245                 250                 255

Val Phe Thr Tyr Cys Asp Tyr Ile Tyr Gly Thr Asp Lys Gly Tyr Arg
            260                 265                 270

Phe Gln Lys Lys Leu Leu Gln Gln Met Lys Glu Lys Ser Lys Lys Ser
        275                 280                 285

Asn Lys Leu Val Asn Gly Gly Glu Lys Phe Asp
    290                 295


<210>  11
<211>  298
<212>  PRT
<213>  Brassica napus

<400>  11
Met Ile Pro Tyr Ala Thr Ile Glu Glu Ala Ser Leu Ala Leu Gly Arg
1               5                   10                  15

Asn Leu Thr Thr Leu Glu Thr Leu Trp Phe Asp Tyr Ser Ala Thr Lys
            20                  25                  30

Ser Asp Tyr Tyr Leu Tyr Cys His Asn Ile Leu Phe Leu Phe Leu Ile
        35                  40                  45

Phe Ser Leu Val Pro Leu Pro Leu Val Phe Val Glu Leu Ala Arg Ser
    50                  55                  60

Ala Ser Gly Trp Phe Asp Arg Tyr Lys Ile Gln Pro Lys Val Lys Asn
65                  70                  75                  80

Ser Phe Ser Asp Met Phe Arg Cys Tyr Arg Asp Val Met Lys Met Phe
                85                  90                  95

Ile Leu Val Val Gly Pro Leu Gln Leu Val Ser Tyr Pro Ser Ile Gln
```

```
            100                 105                 110

Met Ile Glu Ile Arg Ser Gly Leu Pro Leu Pro Ser Phe Gly Glu Ile
        115                 120                 125

Ala Ala Gln Leu Val Val Tyr Phe Leu Val Glu Asp Tyr Thr Asn Tyr
    130                 135                 140

Trp Val His Arg Phe Phe His Ser Lys Trp Gly Tyr Glu Lys Ile His
145                 150                 155                 160

His Ile His His Glu Tyr Thr Ala Pro Ile Gly Tyr Ala Ala Pro Tyr
                165                 170                 175

Ala His Trp Ala Glu Val Leu Leu Leu Gly Val Pro Thr Phe Leu Gly
            180                 185                 190

Pro Ala Ile Ala Pro Gly His Met Ile Thr Phe Trp Leu Trp Ile Ala
        195                 200                 205

Leu Arg Gln Ile Glu Ala Ile Glu Thr His Ser Gly Tyr Asp Phe Pro
    210                 215                 220

Trp Thr Leu Thr Lys Phe Ile Pro Phe Tyr Gly Gly Ala Glu Tyr His
225                 230                 235                 240

Asp Tyr His His Tyr Val Gly Gly Gln Ser Gln Ser Asn Phe Ala Ser
                245                 250                 255

Val Phe Thr Tyr Cys Asp Tyr Ile Tyr Gly Thr Asp Lys Gly Tyr Arg
            260                 265                 270

Phe Gln Lys Lys Phe Leu Gln Gln Ile Lys Gln Glu Ser Lys Lys Ser
        275                 280                 285

Asn Met Gln Asn Gly Gly Asp Lys Leu Asp
    290                 295


<210>  12
<211>  297
<212>  PRT
<213>  Glycine max

<400>  12
Met Leu Pro Tyr Ala Ser Ile Pro Glu Ala Val Ala Ala Leu Gly Arg
1               5                   10                  15

Asn Leu Thr Phe Ala Glu Thr Leu Trp Phe Asn Tyr Ser Ala Ala Lys
            20                  25                  30

Ser Asp Tyr Phe Leu Tyr Cys His Asn Ile Leu Phe Leu Phe Leu Val
        35                  40                  45

Phe Ser Leu Val Pro Leu Pro Leu Val Phe Leu Glu Phe Lys Arg Phe
    50                  55                  60

Ser Phe Val Ser Ser His Lys Ile Gln Pro Lys Val Arg Leu Ser Leu
65                  70                  75                  80

Ala Glu Thr Phe Lys Cys Tyr Lys Asp Val Met Arg Met Phe Phe Leu
                85                  90                  95
```

```
Val Val Gly Pro Leu Gln Leu Ile Ser Tyr Pro Ser Ile Gln Met Ile
            100                 105                 110

Gly Ile Arg Thr Gly Leu Pro Leu Pro Ser Trp Arg Glu Ile Leu Ser
        115                 120                 125

Gln Leu Leu Val Tyr Phe Leu Val Glu Asp Tyr Thr Asn Tyr Trp Ile
    130                 135                 140

His Arg Phe Leu His Asn Asp Trp Gly Tyr Glu Lys Ile His Arg Val
145                 150                 155                 160

His His Glu Tyr His Ala Pro Ile Gly Phe Ala Ala Pro Tyr Ala His
                165                 170                 175

Trp Ala Glu Ile Leu Ile Leu Gly Ile Pro Ser Phe Leu Gly Pro Ala
            180                 185                 190

Met Val Pro Gly His Ile Ile Thr Phe Trp Leu Trp Ile Ala Leu Arg
        195                 200                 205

Gln Ile Glu Ala Ile Asp Thr His Ser Gly Tyr Asp Phe Pro Arg Ser
    210                 215                 220

Ile Thr Lys Tyr Ile Pro Phe Tyr Gly Gly Ala Glu Tyr His Asp Tyr
225                 230                 235                 240

His His Tyr Val Gly Arg Gln Ser Gln Ser Asn Phe Ala Ser Val Phe
                245                 250                 255

Thr Tyr Cys Asp Tyr Ile Tyr Gly Thr Asp Lys Gly Tyr Arg Tyr Gln
            260                 265                 270

Lys Lys Ile Leu Gln Lys Leu Lys Glu Glu Leu Ala Asn Gly Val Glu
        275                 280                 285

Gln Asn Gly Gly Leu Tyr Lys Thr Asp
    290                 295


<210>  13
<211>  254
<212>  PRT
<213>  Glycine max

<220>
<221>  misc_feature
<222>  (187)..(187)
<223>  Xaa can be any naturally occurring amino acid

<220>
<221>  misc_feature
<222>  (225)..(225)
<223>  Xaa can be any naturally occurring amino acid

<400>  13
Met Leu Pro Tyr His Thr Leu Glu Gly Ala Gln Val Ala Leu Gly Arg
1               5                   10                  15

Gly Leu Thr Leu Ala Glu Thr Ile Trp Phe Lys Tyr Ser Ala Asn Lys
            20                  25                  30

Pro Asp Phe Val Leu His Cys His Asn Thr Leu Phe Leu Cys Leu Phe
```

```
        35                  40                  45

Tyr Ser Ile Ala Pro Ile Pro Phe Val Leu Met Glu Leu Ser Gly Tyr
    50                  55                  60

Glu Lys Leu Asn Lys His Lys Ile Gln Pro Ser Val Lys Arg Ser Phe
65                  70                  75                  80

Lys Glu Met Phe Lys Cys Tyr Lys Asp Val Met Glu Thr Phe Val Ile
                85                  90                  95

Ala Val Ser Pro Leu Gln Ile Ile Ser Tyr Pro Thr Ile Lys Trp Ile
            100                 105                 110

Gly Ile Arg Thr Gly Leu Ser Leu Pro Ser Gly Trp Glu Leu Phe Trp
        115                 120                 125

Gln Leu Phe Ile Tyr Phe Val Ile Glu Asp Phe Ser Asn Tyr Trp Ile
    130                 135                 140

His Arg Met Leu His Cys Lys Trp Ala Phe Glu Lys Ile His Lys Val
145                 150                 155                 160

His His Glu Tyr Val Ala Pro Ile Gly Leu Ser Ala Pro Tyr Ala His
                165                 170                 175

Trp Ala Glu Ile Ile Ile Leu Gly Ile Pro Xaa Phe Leu Gly Pro Ala
            180                 185                 190

Leu Val Pro Gly His Ile Thr Thr Tyr Trp Leu Trp Phe Ile Leu Arg
        195                 200                 205

Gln Leu Glu Ala Ile Glu Thr His Ser Gly Tyr Asp Phe Ser Trp Glu
    210                 215                 220

Xaa Thr Lys Tyr Ile Pro Phe Tyr Gly Gly Pro Ala Tyr His Asp Tyr
225                 230                 235                 240

His His Tyr Val Gly Gly Lys Ser Gln Ser Asn Phe Ala Ser
                245                 250


<210>  14
<211>  305
<212>  PRT
<213>  Hordeum vulgare

<400>  14
Met Leu Pro Trp Ala Thr Ala Ala Glu Ala Glu Ala Ala Leu Gly Arg
1               5                   10                  15

Pro Met Thr Pro Ala Glu Ala Leu Trp Phe Arg Trp Thr Ala Gly Thr
            20                  25                  30

Pro Asp Tyr Gly Leu Tyr Cys Leu Asn Ile Leu Phe Leu Leu Leu Val
        35                  40                  45

Phe Thr Leu Ala Pro Leu Pro Val Ala Leu Leu Glu Leu Arg Ala Pro
    50                  55                  60

Arg Ala Val Gly Pro Tyr Lys Leu Gln Pro Arg Val Arg Leu Ser Arg
65                  70                  75                  80
```

```
Ala Asp Phe Leu Lys Cys Tyr Gly Asp Val Met Arg Ile Phe Phe Leu
                85                  90                  95

Val Ile Gly Pro Leu Gln Leu Val Ser Tyr Pro Ala Val Lys Met Val
            100                 105                 110

Gly Ile His Thr Gly Leu Pro Leu Pro Ser Leu Gly Glu Met Ala Ala
        115                 120                 125

Gln Leu Val Val Tyr Phe Leu Val Glu Asp Tyr Leu Asn Tyr Trp Ile
    130                 135                 140

His Arg Leu Leu His Gly Glu Trp Gly Tyr Glu Lys Ile His Arg Ile
145                 150                 155                 160

His His Glu Tyr Thr Ala Pro Ile Gly Phe Ala Ala Pro Tyr Ala His
                165                 170                 175

Trp Ala Glu Val Leu Ile Leu Gly Ile Pro Ser Phe Ala Gly Pro Ala
            180                 185                 190

Ile Ala Pro Gly His Met Ile Thr Phe Trp Leu Trp Ile Ile Leu Arg
        195                 200                 205

Gln Met Glu Ala Ile Asp Thr His Ser Gly Phe Asp Phe Pro Phe Ser
    210                 215                 220

Leu Thr Lys Tyr Ile Pro Phe Tyr Gly Gly Ala Glu Ser His Asp Tyr
225                 230                 235                 240

His His Tyr Val Gly Gly Gln Ser Gln Ser Ile Phe Ala Ser Val Phe
                245                 250                 255

Thr Tyr Cys Asp Pro Leu Cys Gly Thr Asp Arg Gly Tyr Arg Phe His
            260                 265                 270

Arg Ala Ser Leu Pro Met Leu Arg Ala Leu Ala Pro Pro Ala Ala Lys
        275                 280                 285

Lys Asp Ala Pro Met Gly Phe Ser Ser Ala Lys Gly Asp Tyr Val Val
    290                 295                 300

Leu
305


<210>  15
<211>  301
<212>  PRT
<213>  Oryza sativa

<400>  15
Met Leu Pro Tyr Ala Thr Ala Ala Glu Ala Glu Ala Ala Leu Gly Arg
1               5                   10                  15

Ala Met Thr Ala Ala Glu Ser Leu Trp Phe Arg Tyr Ser Ala Gly Ile
            20                  25                  30

Pro Asp Tyr Val Leu Phe Trp His Asn Ile Leu Phe Leu Phe Val Val
        35                  40                  45

Phe Thr Leu Ala Pro Leu Pro Val Ala Leu Leu Glu Leu Arg Ala Pro
    50                  55                  60
```

```
Ala Ala Val Gly Pro Phe Lys Leu Gln Pro Lys Val Arg Leu Ser Arg
65                  70                  75                  80

Glu Glu Phe Phe Arg Cys Tyr Arg Asp Val Met Arg Leu Phe Phe Leu
                85                  90                  95

Val Ile Gly Pro Leu Gln Leu Val Ser Tyr Pro Thr Val Lys Met Val
            100                 105                 110

Gly Ile His Thr Gly Leu Pro Leu Pro Ser Leu Gly Glu Met Ala Ala
        115                 120                 125

Gln Leu Leu Val Tyr Phe Leu Val Glu Asp Tyr Leu Asn Tyr Trp Ile
    130                 135                 140

His Arg Leu Leu His Gly Glu Trp Gly Tyr Glu Lys Ile His Arg Val
145                 150                 155                 160

His His Glu Phe Thr Ala Pro Ile Gly Phe Ala Ala Pro Tyr Ala His
                165                 170                 175

Trp Ala Glu Val Leu Ile Leu Gly Ile Pro Ser Phe Val Gly Pro Ala
            180                 185                 190

Leu Ala Pro Gly His Met Ile Thr Phe Trp Leu Trp Ile Val Leu Arg
        195                 200                 205

Gln Met Glu Ala Ile Glu Thr His Ser Gly Phe Asp Phe Pro Phe Asn
    210                 215                 220

Leu Thr Lys Tyr Ile Pro Phe Tyr Gly Gly Ala Glu Tyr His Asp Tyr
225                 230                 235                 240

His His Tyr Val Gly Arg Gln Ser Gln Ser Asn Phe Ala Ser Val Phe
                245                 250                 255

Thr Tyr Cys Asp Tyr Leu Tyr Gly Thr Asp Lys Gly Tyr Arg Tyr His
            260                 265                 270

Lys Ala Tyr Gln Ala Lys Met Lys Ala Leu Gly Gln Thr Glu Gly Glu
        275                 280                 285

Lys Ala Asp Ser Asn Gly Leu Ser Tyr Ala Lys Leu Asp
    290                 295                 300


<210>  16
<211>  301
<212>  PRT
<213>  Triticum sp.

<400>  16
Met Leu Pro Trp Ala Thr Ala Ala Glu Ala Glu Ala Ala Leu Glu Arg
1               5                   10                  15

Ala Met Thr Ala Ala Glu Ala Leu Trp Phe Arg Trp Thr Ala Glu Ala
            20                  25                  30

Ser Asp Tyr Tyr Leu Tyr Cys Leu Asn Ile Leu Phe Leu Leu Val Val
        35                  40                  45
```

```
Phe Thr Leu Ala Pro Leu Pro Val Ala Leu Leu Glu Leu Arg Ala Pro
    50                  55                  60

Arg Ala Val Gly Pro Tyr Lys Leu Gln Pro Arg Val Arg Leu Ser Arg
65                  70                  75                  80

Ala Glu Phe Ile Lys Cys Tyr Gly Asp Val Met Arg Ile Phe Phe Leu
                85                  90                  95

Val Ile Gly Pro Leu Gln Leu Val Ser Tyr Pro Ala Val Lys Met Val
            100                 105                 110

Gly Ile His Thr Gly Leu Pro Leu Pro Ser Leu Gly Glu Met Ala Ala
        115                 120                 125

Gln Leu Leu Val Tyr Phe Leu Val Glu Asp Tyr Leu Asn Tyr Trp Ile
    130                 135                 140

His Arg Leu Leu His Gly Glu Trp Gly Tyr Glu Lys Ile His Arg Ile
145                 150                 155                 160

His His Glu Tyr Thr Ala Pro Ile Gly Phe Ala Ala Pro Tyr Ala His
                165                 170                 175

Trp Ala Glu Val Leu Ile Leu Gly Ile Pro Ser Phe Ala Gly Pro Ala
            180                 185                 190

Ile Ala Pro Gly His Met Ile Thr Phe Trp Leu Trp Ile Ile Leu Arg
        195                 200                 205

Gln Met Glu Ala Ile Asp Thr His Ser Gly Phe Asp Phe Pro Phe Ser
    210                 215                 220

Leu Thr Lys Tyr Ile Pro Phe Tyr Gly Gly Ala Glu Tyr His Asp Tyr
225                 230                 235                 240

His His Tyr Val Gly Gly Gln Ser Gln Ser Asn Phe Ala Ser Val Phe
                245                 250                 255

Thr Tyr Cys Asp Tyr Leu Tyr Gly Thr Asp Arg Gly Tyr Arg Phe His
            260                 265                 270

Lys Ala Tyr Leu Ala Lys Leu Lys Asp Leu Ala Pro Ser Asp Gly Glu
        275                 280                 285

Lys Glu Gly Ala Asp Gly Phe Ala Tyr Ala Lys Leu Asp
    290                 295                 300


<210>  17
<211>  37
<212>  PRT
<213>  Artificial

<220>
<223>  consensus of SEQ ID NOS:1-4

<220>
<221>  misc_feature
<222>  (2)..(2)
<223>  Xaa can be Leu or Ile or Met

<220>
```

```
<221>  misc_feature
<222>  (5)..(5)
<223>  Xaa can be Ala or Gly

<220>
<221>  misc_feature
<222>  (7)..(7)
<223>  Xaa can be Thr or Ala

<220>
<221>  misc_feature
<222>  (8)..(8)
<223>  Xaa can be Gly or Ala

<220>
<221>  misc_feature
<222>  (9)..(9)
<223>  Xaa can be Asp or Glu

<220>
<221>  misc_feature
<222>  (12)..(12)
<223>  Xaa can be Ala or Gly

<220>
<221>  misc_feature
<222>  (14)..(14)
<223>  Xaa can be Leu or Val

<220>
<221>  misc_feature
<222>  (17)..(17)
<223>  Xaa can be Ala or Thr or Ser or Gly

<220>
<221>  misc_feature
<222>  (18)..(18)
<223>  Xaa can be Leu or Met

<220>
<221>  misc_feature
<222>  (23)..(23)
<223>  Xaa can be Ala or Thr

<220>
<221>  misc_feature
<222>  (24)..(24)
<223>  Xaa can be Ala or Leu

<220>
<221>  misc_feature
<222>  (26)..(26)
<223>  Xaa can be Tyr or Phe

<220>
<221>  misc_feature
<222>  (27)..(27)
<223>  Xaa can be Arg or Glu

<220>
<221>  misc_feature
<222>  (31)..(31)
<223>  Xaa can be Ser or Val or Gly or Ala
```

<220>
<221> misc_feature
<222> (32)..(32)
<223> Xaa can be Val or Met or Ile

<220>
<221> misc_feature
<222> (35)..(35)
<223> Xaa can be Arg or Ser or Leu or Tyr

<220>
<221> misc_feature
<222> (36)..(36)
<223> Xaa can be Tyr or Trp or Cys

<400> 17

```
Met Xaa Pro Tyr Xaa Thr Xaa Xaa Xaa Ala Glu Xaa Ala Xaa Gly Arg
1               5                   10                  15

Xaa Xaa Thr Trp Ala Glu Xaa Xaa Trp Xaa Xaa Tyr Ser Ala Xaa Xaa
            20                  25                  30

Pro Asp Xaa Xaa Leu
        35
```

<210> 18
<211> 30
<212> PRT
<213> Artificial

<220>
<223> Consensus of SEQ ID NOS:1-6 with X defined

<220>
<221> MISC_FEATURE
<222> (2)..(2)
<223> Xaa can be Ile or Met or Leu

<220>
<221> MISC_FEATURE
<222> (4)..(4)
<223> Xaa can be Tyr or Trp

<220>
<221> MISC_FEATURE
<222> (5)..(5)
<223> Xaa can be Ala or Gly or Pro or His

<220>
<221> MISC_FEATURE
<222> (6)..(6)
<223> Xaa can be Thr or Ser

<220>
<221> MISC_FEATURE
<222> (7)..(7)
<223> Xaa can be Ala or Thr or Leu or Val or Ile

<220>
<221> MISC_FEATURE
<222> (8)..(8)

<223> Xaa can be Ala or Gly or Thr or Glu or Pro

<220>
<221> MISC_FEATURE
<222> (9)..(9)
<223> Xaa can be Glu or Asp or Ala or Gly

<220>
<221> MISC_FEATURE
<222> (11)..(11)
<223> Xaa can be Ser or Glu or Val or Gln

<220>
<221> MISC_FEATURE
<222> (12)..(12)
<223> Xaa can be Gly or Ala or Ile or Val or Leu

<220>
<221> MISC_FEATURE
<222> (14)..(14)
<223> Xaa can be Val or Leu

<220>
<221> MISC_FEATURE
<222> (15)..(15)
<223> Xaa can be Gly or Asn or Ser or Glu

<220>
<221> MISC_FEATURE
<222> (17)..(17)
<223> Xaa can be Thr or Ser or Gly or Ala or Pro or Asn

<220>
<221> MISC_FEATURE
<222> (18)..(18)
<223> Xaa can be Met or Leu

<220>
<221> MISC_FEATURE
<222> (20)..(20)
<223> Xaa can be Trp or Pro or Tyr or Arg or Thr or Phe or Leu or Ala

<220>
<221> MISC_FEATURE
<222> (21)..(21)
<223> Xaa can be Ala or Leu or Phe

<220>
<221> MISC_FEATURE
<222> (23)..(23)
<223> Xaa can be Thr or Ala or Ser

<220>
<221> MISC_FEATURE
<222> (24)..(24)
<223> Xaa can be Ala or Leu or Ile or Val

<220>
<221> MISC_FEATURE
<222> (26)..(26)
<223> Xaa can be Tyr or Phe or Leu

<220>

<221> MISC_FEATURE
<222> (27)..(27)
<223> Xaa can be Glu or Arg or Asn or Asp or Lys

<220>
<221> MISC_FEATURE
<222> (28)..(28)
<223> Xaa can be Trp or Tyr

<220>
<221> MISC_FEATURE
<222> (29)..(29)
<223> Xaa can be Ser or Thr

<400> 18
```
Met Xaa Pro Xaa Xaa Xaa Xaa Xaa Xaa Ala Xaa Xaa Ala Xaa Xaa Arg
1               5                   10                  15

Xaa Xaa Thr Xaa Xaa Glu Xaa Xaa Trp Xaa Xaa Xaa Xaa Ala
            20                  25                  30
```

<210> 19
<211> 921
<212> DNA
<213> Zea mays

<400> 19
```
atgatcccct acgcgactgc ggcggaggcg gagggagcac tggggcgcac catgacgtgg     60

gctgagacag catggtacga gtactcggcg gtgatgccag attcctggct gcactgccac    120

accacattta tcctgttcgt catctacagc atcgccccgc tgcccctgct actcctagag    180

cagttcgctc cgtccgtcgt gctgccgtac aagctgcagc cccgggtacg gctgcccccg    240

gcagcctccc tcagctgcta catggacgcg gcctgcatct ttccgctcgc cgttggcctt    300

cagttcgtct cctatcctgc ggtcgccaag atactaagga cccgaatggg actgccgttg    360

ccgtcggtga gggagaccat cgcgcagcta gtcgtatact ctctagtgga ggattacctc    420

agctactgga tgcaccgtct gctgcacacc cagtggtgct acgagaagat ccaccgcgtc    480

caccacgagt tcacggctcc tacaggcttc gccatgtcgt acagccactg ggccgagaac    540

gtcgtccttt ctatcccggc cttggccggc ccagtgctcg tgccatgcca tgtcaccacg    600

cagtggctat ggttctccat ccgcctaatt gagggcatta acacgcacag cggttaccat    660

ttcccgttca gcccttgcag gctgattcca ttctacggag ggctgcata ccatgactac    720

catcactatg caggaggccg tagccaaagc aactttgcac ccctgttcac ctactgtgat    780

tatttatata ggacagacaa aggctacaga taccacaagc taaagcaaga gaagctgaag    840

agtctagcag aaaatagtgc ggataaagga ggcaactact cattcgacga agggaaaaag    900

aacagatatt tttgtgcctg a                                              921
```

<210> 20
<211> 882

<212> DNA
<213> Zea mays

<400> 20
atgatgccct acggcacggc ggcggaggca gaggcggcgc tggggcgctc catgacctgg 60

gcagaggccc tgtggttccg gtactcagcg gggatgccgg acctgtgtct gacgtggcac 120

gtctccctcg tctacctcgt cttgtacgcg ttggttccgc tgccggtcat ggttatccag 180

aagctcgcgc cggggtacgc cctgcggcac aagctgcagc ccggggtgcc ggagccctcg 240

ccggtttcca cctacgtcga atacataagg gacagcaggg gcgtcaccct ggccgccttg 300

ggcccgttcc cgctcatcta ctccatcgca ttcaagctgt tcggggtccg gacgggactc 360

cctttgccgt cggtctggga gactgcgacg cacctggcgg tgtattcgct ggtggaggac 420

tacacgtcgt actggctcca ccgcttcctg cacaccaggt gggggtacga gaagatccac 480

cgcgtccacc acgagaagac ggcgccgtcc gggttcgccg ccgcctacgc cacgggcact 540

gagctcagct tgtacctcac cacgctcttc cttgggccgg cgatcgtgcc cagccacgtc 600

accacgcact ggctcttgtt ctccatccgc ataatggagg ccttcgacac acacagcggg 660

taccacttcc cgttcagcct cgcgaggttc atcccgttct acggtggcgc ggaattccac 720

gactaccatc actacgccgg agagaagacc aggagcaatt tcagttccgt gttcacgtac 780

tgtgattata tatatgggac aaacaaaggc tacatgtacc acaagagaag cctagccgag 840

ctgaagacga aggaggccga acacagcggg aaagaagact ga 882

<210> 21
<211> 855
<212> DNA
<213> Oryza sativa

<400> 21
atgctcccgt acgcgacggc ggcggaggcg gaggcggcgg tggggcgggg cctgacgtgg 60

gcggaggcgg cctggttccg ctactcggcg gccatcccgg actactgcct ctactgccac 120

aacgtcccca tcctcctcct cgtctacacc ctcgcgccgc tcccccctcgc gctgctcgag 180

ctccgccgcc acctgccgct gccgcacaag ctgcagcccg gcgtgcgcca cccgccggcc 240

gccttcctcc ggtgctacgc tgccaccgcg cgcgtgctgc tcctcgccgt cgggccggtc 300

cagctggcgt cgttccctgc ggtgagggcg gtggggatac ggacggggct gccgctgccg 360

tcggcggggg agacggcggc gcaggtggcg gtgtacctgc tggtggagga ctacctgggc 420

tactggatcc accgcctgct gcacacgccg tgggcctacc accacatcca ccgagtccac 480

cacgagttca ccgcgcccat gggctacgcc gccccgtacg cccactgggc cgagatcctc 540

atcctcggct tcccggcctt cgccggccca gccatcgtgc cgtgccacat gaccaccttc 600

tggctctggt tcgtgcttcg ccacctcgag gccatccaca tccacagcgg gttcaagttg 660

ccgttcgatc cgaccaagta tatcccgttg tatggaggag tggagtacca tgactaccac 720

catttcgtgg gaggacacag ccagagcaac ttctcttctg tcttcacttt ctgtgattac 780

atctacggga ctgacagagg ctacagatac cataaggcaa gcttgtcaaa gatgagaata 840

tttgttagag cttag 855

<210> 22
<211> 907
<212> DNA
<213> Hordeum vulgare

<400> 22
atgctgccgt acgcgacgac cggcgatgcg gaggcggcgc tcggccgcgc cctgacgtgg 60

gcggaggccg cgtggctccg ctactcggcg tccgtgccgg accgctacct ccactggccc 120

aacatcgcca tcacattggt cgtctacacg ctggcgccgc tgccgctcgc cctcttcgac 180

ctcgccgccc cggccgtcgc cgcgccgtac aagctgcagc ccaaggtgca gcacccgccc 240

gccaccttct tccgctgcta catggacgcc gttcgggtct cgctgctcat catcgggcca 300

taccagctca tctcctatcc cgccgcaaag ataatggaca tacggacggg acttccattg 360

ccgtcaatgg gggaaatagc agcgcaactg acggtatact tcttggtgga agactatctg 420

aactactggc tccatcggct gttgcacacc aaatggtgct atgaaaagat ccaccatgtt 480

caccatgagt tcacggcgcc catggcctat gccgcatggt atggacactg ggctgagatg 540

ctcatccttg cggggcccct ccttggccgg ccctgcactc gtcccatgcc atgttaccac 600

gctctggatc tggtttgcag cacgtttggt tgagagcctc aacatacata gcggatttaa 660

gttgccattc aacgctgaga agtacatacc attctacgga ggggcagagc accatgacta 720

ccatcactac ataggaggac agagcaagag caacttcgcc cctgttttca cctactgtga 780

ttacatatac ggaacggata aaggctacag atatcacaag gcaaccctgg caaagctgaa 840

ggagttggca ggaaacgagg ttcagaaagg agtcgacaac ggattcaaca gcggaaagca 900

ggagtag 907

<210> 23
<211> 906
<212> DNA
<213> Zea mays

<400> 23
atgctgccct acgcgacggc ggcggaggcg gaggcggcgc ttggccggcc catgacgccc 60

gccgaggcgc tgtggttccg gtacaccgcg ggggtgtccg actaccacct ctactgctgc 120

aacatcctct tcctcttcgt cgtcttcacg gtggccccgc ttcccatcgc gctcctcgag 180

ctccgggccc cggccgcggt ctcgccgtac aaactgcagc cgcgggtgcg gctctccagg 240

gccgagttcg tccggtgcta caaggacgtc ctccgcatct tcttcctcgt catcggcccg 300

ctccagctcg tctcctaccc ggcggtcaag tttgtgggaa ttcacacgaa gttgcctttg 360

ccgtcccttg cggagttggc agcacagcta ctggtgtact tccttgttga ggactacctc 420

aattactgga tccacaggtt tctccacggg gagtgggggt accagaatat ccaccgtgtt 480

caccatgagt tcactgcgcc aataggattc gcagctccat atgcacactg ggctgaggtg 540

ctgatactcg gcatcccctc cttcgtcggg ccagccattg ttccaggcca catgatcaca 600

ttctggctct ggattatact ccgtcaggtg gaggctatcg agacacatag cggctttgat 660

ttcccattca ccccgacaaa gtatattcca ttctatggag gagcagaata ccatgactat 720

catcattatg taggaggcca gagccaaagc aattttgctt ctgttttcac ttactgtgat 780

tacttatatg gcactgacaa aggttacaga ttccacaaaa catacctagc aaagctgaag 840

gatctggggc ataatgatgg ccagaaagga gacggcagcg gacccagcta tgtgaaactg 900

gattag 906

<210> 24
<211> 900
<212> DNA
<213> Allium porrum

<400> 24
atgatcccct atccatctct gacagctgca gaagcggccc tgaaccgtcc gctaacctac 60

gccgaaacca tttggttcaa ttactccgcc acaatacccg atccgttgct gtattaccac 120

aatacgattt tcctttttgt tattttcacg ctagtacctc tccctttggc tcttctcgag 180

ctctattggc cgtctgtttt gaagccgttc aagatccagc cgaaggtgta cctgtcgaaa 240

tctgagtttt tggaatgtta taagaatgtc attaaggttt tcttcttagt tgtttgcccg 300

cttcagcttc tgtcgtatcc tactgttaag ttcgtgggaa taaggactgg gctaccatta 360

ccatcagtat gggaagttgc atctcaatta gcagtgtact tcttgttgga ggattttgga 420

aattattgga ttcacagatg gctacatgga aaatgggggt acgagaagat tcacaaagtt 480

catcatgaat atactgcacc aataggtttt gctgctcctt acgcccattg ggctgaggtg 540

ttgatccttg gtattccatc gtttcttgga cctgctattg ttcctggaca catgattact 600

ctttggttat ggatagctct gaggcaaatt gaggcactgg atacccatag cgggtacgac 660

ttccctttga gttttaccaa gttcattcct ttctatggag gcgctgaata tcatgattat 720

catcattatg ttggagggca aagccagagc aatttcgctt ctgtgtttac gtattgtgac 780

tacgtatatg gaactgacaa gggttacagg tatcgaaagg catgcctctc gatgatgaag 840

gaagaatcgg aaaaccaaaa cggagttgag aattcttttc agaaccagaa atctgattga 900

<210> 25

<211> 897
<212> DNA
<213> Arabidopsis thaliana

<400> 25
atgattcctt acgctacagt cgaagaagct tcaatcgcac tgggacgaaa cctcacacgt 60

ctcgaaactc tatggttcga ttactccgcc acgaagtccg attactatct ctactgtcac 120

aacattttgt tcttgttcct cgtcttctct ctcgttcctc tccctctcgt tttcgtcgaa 180

ttggctcgat ctgcttctgg tttgtttaat cggtataaga tccagcctaa ggttaattac 240

tctttatctg atatgttcaa atgttacaaa gacgtcatga cgatgtttat cctcgtcgtt 300

ggtccattgc aactcgtttc ttatccttcg attcagatga ttgagatacg atctggatta 360

ccattaccaa caattacaga gatgctgtca cagttagtag tctacttctt gatagaagac 420

tacactaact actgggtaca tagattcttt catagtaaat ggggatacga taagattcat 480

cgagttcatc acgagtacac agctcctata ggatatgctg ctccttatgc acattgggct 540

gaagttttgc ttctcggaat cccgacgttt atgggaccag ctattgctcc tggtcatatg 600

ataacctttt ggttgtggat tgctttaagg caaatggaag ctattgagac tcacagtgga 660

tatgattttc catggagtcc aacaaaatac atccctttct acggtggtgc tgagtaccat 720

gactatcatc actacgttgg aggacaaagt caaagcaact tcgcttcagt gttcacgtac 780

tgtgattaca tttatggaac tgacaagggt tacagattcc aaaagaagct tcttgagcag 840

atcaaggagt cgtcgaagaa gagcaacaag cataacggag gaataaaatc cgattag 897

<210> 26
<211> 894
<212> DNA
<213> Arabidopsis thaliana

<400> 26
atgatcccat acgcaacaat cgaagaagcg tcgatcgcat tatctcgaaa cctcacatgg 60

ctagagactc tctggttcga ttactccgcc accaaatccg attactacct ctactgccac 120

aacattctct tcctcttcct catcttctct ctcgttcctc tccctctcgt tttcatcgaa 180

tcatctcaat ccacctcaga tttgttcaat cgctacaaaa tccaaccaaa agtgaaaaac 240

tcattctcat cgatgttcaa atgttacaaa gacgtcatga agatgttcat cctcgtcgtt 300

ggtccattac aactcgtttc ttatccttcg attcaggttg attttgtttt tcgtgtgttg 360

aaacagatga ttgagatacg aagtggatta ccattaccat catgtatgga gattgtagca 420

cagttagtgg tttacttctt ggtagaggat tatactaatt actgggttca tagattcttt 480

cattgtaaat ggggttatga gaagtttcat catattcatc atgagtatac agctcctatt 540

ggttatgctg ctccttatgc tcattgggct gaggttttgc ttcttggcat tcccacgttt 600

cttggacctg ctattgctcc tggtcatatg attacctttt ggttgtggat tgctttacga 660

cagattgagg ctatcgaaac tcatagcgga tatgatttcc catggtctct gacaaagtac 720

attccatttt atggtggagc tgagtatcat gattaccatc actacgttgg aggacaaagc 780

cagagtaact ttgcttcagt ttttacttac tgcgattaca tctatggaac tgataaaggt 840

taccgattcc agaagaagct tcttcagcag gtaaataaat actccataaa ctga 894

<210> 27
<211> 876
<212> DNA
<213> Arabidopsis thaliana

<400> 27
atgatccctt atccaaccgt agaagatgcg tccgtggcgt taggacgaaa ccttacttgg 60

ttcgagacgg tttggttcga ttactcagcc accaaatcca atttccatgt atattgccac 120

accattctgg ttctcttcct tgtcttttca ctagctcctt ttcctcttgt gattgtcgaa 180

tggaccggtt ggttcgatca gtttaagatt cagaagaagg ttaagtattc gttgtctgat 240

atgttccaat gttataaaga agtcatgaag ttgttccttc tcgtcgtcgg cacattgcaa 300

atcgtttctt atccttccat ccagatggtt gggattcgaa gtggtttgcc attaccatcg 360

ttaatggaga tagtagcaca attagtggtt tacttcttga tagaagatta cactaactac 420

tggatccata gatggatgca ttgcaaatgg ggttacgaga agattcatcg aatccatcat 480

gagtacacat cacctatcgg atacgcatcg ccgtatgcgc attgggccga gattttgatt 540

cttgggattc cgacgtttct tggaccggca attgctcctg gccatataat gacgttttgg 600

ttatggatat ctttacgaca attcgaggcg attgagaccc acagcggata tgattttcca 660

tggagtgtga caaaattaat tccattttac ggtggacctg agtatcatga ctaccatcac 720

tacgttggag gacaaagcca gagcaacttt gcttcggttt tcacttactg cgattacatt 780

tatggaactg ataaaggcta tcgaatccat aagaagcttc ttcatcatca gattaaagag 840

gaagctgaag agaagagagt aaggaaacac gattag 876

<210> 28
<211> 900
<212> DNA
<213> Arabidopsis thaliana

<400> 28
atgatcccat acgcaacaat cgaagaagcg tcgatcgcat tatctcgaaa cctcacatgg 60

ctagagactc tctggttcga ttactccgcc accaaatccg attactacct ctactgccac 120

aacattctct tcctcttcct catcttctct ctcgttcctc tccctctcgt tctcatcgaa 180

tcagctcaat ccacctcaga tttgttcaat cgctacaaaa tccaaccaaa agtgaaaaac 240

tcgttctcat cgatgttgaa atgttacaaa gacgtcatga agatgttcat cctcgtcgtt 300

ggtccattac aactcgtttc ttatccttcg attcagatga ttgagatacg aagtggatta 360

ccattaccat catgtatgga gattgtagca cagtttgtgg tttacttctt ggtagaggat 420

tatactaatt actgggttca tagattcttt cattgtaaat ggggttatga gaagtttcat 480

catattcatc atgagtatac agctcctatt ggttatgctg ctccttatgc tcattgggct 540

gaggttttgc ttcttggcat tcccacgttt cttggacctg ctattgctcc tggtcatatg 600

attacctttt ggttgtggat tgctttacga cagattgagg ctatcgaaac tcatagcgga 660

tatgatttcc catggtctct gacaaagtac attccatttt atggtggagc tgagtatcat 720

gattaccatc actacgttgg aggacaaagc cagagtaact ttgcttcagt ttttacttac 780

tgcgattaca tctatggaac tgataaaggt taccgattcc agaagaagct tcttcagcag 840

atgaaggaga agtccaagaa gagcaacaag ctggttaatg gaggagagaa attcgattag 900

<210> 29
<211> 897
<212> DNA
<213> Brassica napus

<400> 29
atgatccctt acgcaacgat cgaagaggcc tctctggcgt taggccgaaa cctcacgacc 60

ctcgagactc tctggttcga ttactccgcc acgaagtcag attactacct atactgccac 120

aacatcctct tcctcttcct catcttctcc ctcgtccccc tccctctcgt cttcgtcgaa 180

ttggcgcgat ccgcctcggg atggttcgat cggtacaaga ttcagcccaa ggtcaagaac 240

tccttctccg acatgttccg ctgctacaga gatgtaatga agatgttcat cctcgttgtc 300

ggccctttgc agctcgtgtc ctacccttca atccagatga ttgagattcg gagtgggttg 360

ccgttaccgt ctttcgggga gattgcggcg cagttagtgg tgtacttctt ggtggaggac 420

tatacgaact attgggttca tagattcttt catagcaagt ggggttacga gaagattcat 480

catatacatc atgagtacac tgctcctata gggtacgctg cgccttatgc gcattgggct 540

gaggttttgc ttcttggggt tccgacgttt cttggacctg ctattgctcc tggacacatg 600

attaccttct ggttgtggat tgctttgcgc cagattgaag ccattgagac tcacagcgga 660

tatgattttc catggacact gacgaaattc attccattct atggtggagc tgagtatcat 720

gattaccatc attacgttgg aggacaaagc caaagcaact ttgcttcagt tttcacttac 780

tgcgattaca tctatggaac tgacaaaggt taccgattcc aaaagaagtt tcttcagcag 840

atcaagcagg agtccaagaa gagcaacatg cagaatggag gagataagtt agattag 897

<210> 30
<211> 894
<212> DNA
<213> Glycine max

<400> 30
atgctcccct acgcttccat cccggaggcc gtggcggcgc tgggccgcaa cctcaccttc 60
gcggagaccc tctggttcaa ctactccgcc gccaagtccg attacttcct ctactgccac 120
aacattctgt tcctcttcct cgtcttctcc ctcgtccccc tccccctcgt cttcctcgaa 180
ttcaagcgct tctccttcgt ctcttcccac aagatccaac caaaagtccg cttgtccctg 240
gccgaaacct tcaagtgcta caaagacgtc atgcgcatgt tcttcctcgt cgtcggcccc 300
ctccaactca tctcttaccc ttccatccag atgattggga tcaggacggg cttgccatta 360
ccttcgtggc gggagatcct ctcgcagctt ctggtgtact ttctcgtaga ggattacacc 420
aattactgga tccacaggtt tctgcacaac gattgggggt acgagaagat tcaccgcgtc 480
caccacgagt accatgcgcc cattggattc gccgcgccct atgcccactg ggccgagatc 540
ttgatcctcg ggattccctc ctttcttggg cctgccatgg ttcctggcca cattatcacc 600
ttctggctct ggatagcctt gcgccagatt gaagccattg acacgcacag cgggtatgac 660
tttcctagga gtatcacaaa atatattcca ttttatggtg gtgctgagta tcatgattac 720
catcattacg ttggaagaca aagccaaagc aattttgctt cagttttcac atactgtgat 780
tacatctatg gaactgacaa ggggtatagg tatcagaaaa aaatacttca gaagttgaag 840
gaagagttgg caaatggtgt tgagcagaac ggaggattat acaagactga ctga 894

<210> 31
<211> 764
<212> DNA
<213> Glycine max

<400> 31
atgctccctt accataccct tgaaggagca caagttgcac taggcagagg actaaccctt 60
gctgagacaa tatggttcaa atactctgcc aacaaacctg attttgttct tcattgccac 120
aacactctat tcttgtgctt attttactct atagctccaa ttcctttcgt attaatggag 180
cttagtgggt atgagaagct aaacaaacac aaaattcagc cctcggttaa gagatcattc 240
aaggaaatgt tcaagtgcta caaagatgtc atggagacct ttgtcattgc agttagccca 300
ctacagataa tttcttatcc caccatcaag tggattggga tcagaactgg tttgtcattg 360
ccatcaggct gggagttatt ttggcaatta tttatttact ttgtcataga agatttttcg 420
aattattgga ttcataggat gctccattgc aagtgggcat ttgagaagat tcacaaggtc 480
catcatgaat atgtagcacc aattgggctc tcagcacctt atgcccattg ggccgagata 540
atcatattgg gtatccccct cgtttctagg cccagcactg gttcctgggc atataacaac 600
ctattggcta tggttcattt tgcgacagct agaagccatc gagactcata gcgggtatga 660
tttttcttgg gaggcccaca aaatatatac cattttatgg agggcctgca taccatgact 720

accatcacta cgttggtgga aaaagtcaaa gcaactttgc ctca 764

<210> 32
<211> 918
<212> DNA
<213> Hordeum vulgare

<400> 32
atgctcccgt gggcgacggc ggcggaggcc gaggcggcgc tgggccggcc catgacgccc 60

gcggaggcgc tctggttccg gtggaccgcc gggacgcccg actacggcct ctactgcctc 120

aacatcctct tcctcctcct cgtcttcacg ctcgcgccgc tccccgtcgc gctcctcgag 180

ctccgcgcgc cgcgggccgt cgggccgtac aagctgcagc cccgggtgcg cctctcgcgg 240

gccgacttcc tcaagtgcta cggggacgtc atgcgcatct tcttcctcgt catcggaccg 300

ctccagctcg tctcctaccc cgccgtcaag atggtgggga tccacaccgg actgccgctg 360

ccgtctctgg gggagatggc ggcgcagctg gtggtctact tcctggtcga ggactacctc 420

aactactgga tccaccggct gctgcacggt gagtggggct atgagaagat ccaccggatc 480

caccacgagt acaccgcgcc cattggcttc gcagcgccat acgctcactg ggcagaggtg 540

ctcatacttg gcatcccctc cttcgctggc ccggccattg caccaggcca catgattacc 600

ttctggctct ggattatact tcgtcagatg gaagccattg acacacacag cggttttgat 660

ttcccattca gcctgacaaa gtatattccg ttctatggag gagcagaatc ccatgattat 720

catcactacg ttggaggcca aagccagagc atttttgctt cggttttcac gtactgtgat 780

cccctgtgtg gcaccgacag aggctacaga ttccacaggg cttccttacc aatgttgagg 840

gccctggccc cccccgccgc caagaaagat gcccccatgg gtttcagttc cgcgaagggg 900

gattacgtgg tcttatag 918

<210> 33
<211> 906
<212> DNA
<213> Oryza sativa

<400> 33
atgctgccct acgcgacggc ggcggaggcg gaggcggcgc tggggagggc gatgacggcg 60

gcggagtcgc tgtggttcag gtactcggcg gggatcccgg actacgtcct cttctggcac 120

aacatcctct tcctcttcgt cgtcttcacg ctcgcgccgc tccccgtcgc gctcctcgag 180

ctccgcgcgc cggccgccgt ggggccgttc aagctgcagc ccaaggtgcg gctctcccgg 240

gaggagttct tccgctgcta cagggacgtc atgcgcctct tcttcctcgt catcggcccg 300

ctccagctcg tgtcctaccc taccgtcaag atggtgggaa tccacacagg gctgccactg 360

ccgtcgctgg gggagatggc ggcgcagctg ctggtgtact tcctggttga ggactacctc 420

aactactgga tccatcggtt gctacatggg gagtggggct atgagaagat ccaccgtgtc 480

caccatgagt tcacggcacc cattggattc gccgcgccat atgcacactg ggctgaggtg 540

ctcatcctcg gcatcccctc ctttgtcggg ccagcgcttg cacctggtca catgatcacc 600

ttctggctct ggattgtact ccgccagatg gaggccatag agacacacag cggctttgat 660

ttcccgttca acctgacaaa gtatattcca ttctatggag gcgcagaata ccatgattat 720

catcactatg ttggacgcca gagtcagagc aatttcgctt ctgttttcac gtattgtgat 780

tatctatatg gaaccgacaa aggttacaga taccataagg cgtaccaagc aaagatgaag 840

gctctggggc aaacggaagg cgagaaagca gatagcaatg gattgagcta cgcgaagttg 900

gattaa 906

<210> 34
<211> 906
<212> DNA
<213> Triticum sp.

<400> 34
atgctcccgt gggcgacggc ggcggaggcc gaggcggcgc tggagcgcgc catgacggcc 60

gcggaggcgc tctggttccg gtggaccgcg gaggcgtccg actactacct ctactgcctc 120

aacatcctct tcctcctcgt cgtcttcacg ctcgcgccgc tccccgtcgc gctcctcgag 180

ctccgcgcgc cgcgggccgt cgggccgtac aagctgcagc cccgggtgcg gctctcgcgg 240

gccgagttca tcaagtgcta cggcgacgtc atgcgcatct tcttcctcgt catcggcccg 300

cttcagctcg tctcctaccc cgccgtcaag atggtgggaa tccacaccgg actgccgctg 360

ccgtctctgg gggagatggc ggcacagctg ctggtctact tcctggttga ggactacctc 420

aactactgga tccaccggct gctgcacggt gagtggggct atgagaagat ccaccggatc 480

caccatgagt acaccgcgcc cattggcttt gccgcgccat acgcacactg ggcagaggtg 540

ctcatacttg gcatcccctc cttcgctggg ccggccattg caccaggcca catgataaca 600

ttctggctct ggattatact tcgtcagatg gaagccattg atacacacag cggttttgat 660

ttcccattca gcctgacaaa gtatattcca ttctatggag gagcagaata ccatgattat 720

catcactacg ttggaggcca aagccagagc aattttgctt ccgttttcac gtactgtgat 780

tacctatatg ggaccgacag aggttacaga ttccacaagg cttacttagc aaagttgaag 840

gatctggcgc caagcgacgg cgagaaagaa ggtgccgacg gattcgctta tgcaaagttg 900

gattag 906

<210> 35
<211> 1384
<212> DNA
<213> Oryza sativa

<400> 35
ctcgaggtca ttcatatgct tgagaagaga gtcgggatag tccaaaataa aacaaaggta 60

agattacctg gtcaaaagtg aaaacatcag ttaaaaggtg gtataaagta aaatatcggt 120

aataaaaggt ggcccaaagt gaaatttact cttttctact attataaaaa ttgaggatgt 180

ttttgtcggt actttgatac gtcatttttg tatgaattgg tttttaagtt tattcgcttt 240

tggaaatgca tatctgtatt tgagtcgggt tttaagttcg tttgcttttg taaatacaga 300

gggatttgta taagaaatat ctttaaaaaa acccatatgc taatttgaca taatttttga 360

gaaaaatata tattcaggcg aattctcaca atgaacaata ataagattaa aatagctttc 420

ccccgttgca gcgcatgggt atttttctta gtaaaaataa aagataaact tagactcaaa 480

acatttacaa aaacaacccc taaagtccta aagcccaaag tgctatccac gatccatagc 540

aagcccagcc caacccaacc caacccaacc caccccagtc cagccaactg gacaatagtc 600

tccacacccc cccactatca ccgtgagttg tccgcacgca ccgcacgtct cgcagccaaa 660

aaaaaaaaaa gaaagaaaaa aaagaaaaag aaaaaacagc aggtgggtcc gggtcgtggg 720

ggccggaaac gcgaggagga tcgcgagcca gcgacgaggc cggccctccc tccgcttcca 780

aagaaacgcc ccccatcgcc actatataca taccccccccc tctcctccca tccccccaac 840

cctaccacca ccaccaccac cacctcctcc cccctcgctg ccggacgacg agctcctccc 900

ccctccccct ccgccgccgc cggtaaccac cccgcccctc tcctctttct ttctccgttt 960

tttttttccg tctcggtctc gatctttggc cttggtagtt tgggtgggcg agaggcggct 1020

tcgtgcgcgc ccagatcggt gcgcgggagg ggcgggatct cgcggctggg gctctcgccg 1080

gcgtggatcc ggcccggatc tcgcggggaa tggggctctc ggatgtagat ctgcgatccg 1140

ccgttgttgg gggagatgat gggggggttta aaatttccgc catgctaaac aagatcagga 1200

agaggggaaa agggcactat ggtttatatt tttatatatt tctgctgctt cgtcaggctt 1260

agatgtgcta gatctttctt tcttcttttt gtgggtagaa tttgaatccc tcagcattgt 1320

tcatcggtag tttttctttt catgatttgt gacaaatgca gcctcgtgcg gagctttttt 1380

gtag 1384

<210> 36
<211> 1316
<212> DNA
<213> Zea mays

<400> 36
ctgccacatc ggcatgtact tagggcgcta gctctccccc gctagacacg tagcactctg 60

ctacacccct cattgtacac ctggatcctc ttcttacgcc tataaaagga aggaccagga 120

ccctcttaga gagggttggc cgcgcgggga cgaggacgag acaggcgctc tcttggggcc 180

gctcgcttcc ctctcccgcg tggacgcttg taactcccta ctgcaagcgc acccgacctg 240

```
ggcgcggggc gaacacaaag gccgcgggat tcccacctct ctcacgccgg tctccggccg     300
cctcgcttct ctccccttcg cgctcgccct cgcgctcgac ccatctgggc tggagcacgc     360
gacgacactc actcgtcggc ccaagggacc ccccggtctc ggaacgcgac actatctttt     420
cacacttaga agctggcaag aaggtcaaac aaataaggtc ttatcgtgta tattattttt     480
gcattgcaga tagagtggag tttgaaataa aaggtgagat agcaggagtg gaaatgggct     540
caaaaattta tactataaaa ttgaatgatc aaatcgaatt aagatcggac tttatttgta     600
ttcattcttg aactaaaatt atttaactat cataatttat tgtggataaa catttggacc     660
acgattcatt gccatcgata ggaggtgttg taagagagcc agaaagctta ggacatgtaa     720
cccgattaaa taaagagtct tttgaagtgt ccctaagggc tacgtgaaaa aaaatcaaga     780
gacatactct ttgtgaagag tctgtctcta cacaaatctc tatataagtt gtgtctcaat     840
tacattatta tctagagact cagtgttgta tcacgtagtc ttttagtggt ctcttttatt     900
tgaaatccgt tgcagagtcc cttatgtgca gagtttggac atcccacgcg gtagaagcga     960
cgtggcagtt gccacagtat actgacgtgt gggcccagaa aaccccactg tcaatggaga    1020
aagaccatcc aaagcacaga gacttctatt ttattcgtga ctcttccaga atcccgacca    1080
tcccacacag agccacggac gcgggacgcc tacgcctcgc cgcgcccggg gccccgcaca    1140
gtccacagcc tttcagaacc ttccgtcgcc ttccagaaga acagaagccc acccgtcgcc    1200
accaatataa atcgcccctc cagatcggca ctccgcacac caagaatcac atcacacagc    1260
gaaccgagaa accaacacag caacaagcaa agcagcgatc cgacatccga gagatg        1316
```

<210> 37
<211> 764
<212> DNA
<213> Zea mays

<400> 37

```
gtggagtggt ggacactagt gccgcggttc atctgacacg tgtcgccacg tgccgccatg      60
gcagcacctc agcccggccg gcgggccgac tgacgtcttg ggcaaagcgg cgagcgacgc     120
aggcggcgaa agccatccga tttgacccct cgctagaccc ttcaagaacg aacgctgtgc     180
tgctcagatc agaccgtgtc tgcctcaaag cgatgccagg acgccacgtc caagcaaagc     240
acccgatgcc attgccacct cccagcactc acgcgtgagc gtgactataa aaaacgcacc     300
ctctgcatcc gcccccgtct gcctgcccta ccgaatcttt cgccgtccca tcagcccagc     360
aattcttcgc tgttcgagga cccctcggtt tcgaccgaag cccagcaagc cgaccacaca     420
ccgctgccgt tggttccgtc ccaagagatg ggcaagtcct ggtcgctcat cagccacctc     480
cacaccgtcg ccgggtagtt tcactgttcc ctcgcagttc gttttccgat tcctcctcgt     540
ccatctattg ggctcgctcg acgctggatg cctgacgtgt gcgtttgctg cttctttgtc     600
```

ttgtctgtcc ctccctcccg tttcgcaggc caagcatcac cctgctgtac cctctgtaag 660

ttccttcatc accctaataa tagcagggac cagttttacc agtgcagcag tgaccgacca 720

cggtccacgg catacgtgag ctgagagcat cgtgctggga catg 764

<210> 38
<211> 1380
<212> DNA
<213> Zea mays

<400> 38
cgacctgcag gcggccgcga attcactagt gattactata gggcacgcgt ggtcgacggc 60

ccgggctggt aagacttaaa aatcaacaat atcttcacat gacttaatta taatgtcttg 120

cttgagacgt tgtttttgct actacataag ataaagttca aataaatgca tggtggagtt 180

cagcctaggc aaagtgatgg tccgaatgat taacacccca agcaagacat tataagtcat 240

gtgaagatct gcaagacgtg ctaagagtct ctgacacacc aacaagtgga agcccgaaca 300

aacaaaaacg aagccatcaa agttgagata aagaggtgga taaattgaaa attgtctcat 360

gattttggat atactcaaat cgacatgact tcatctctaa actatagaac ttttgatttg 420

cttttcaaaa agtccaagat caacaaaacg tgttggtggg tgcgggtttg gttcttaacc 480

caataggttt tttctcgtgt gtatgaaaag gttgtaccca tgtgtgaccg agccagacag 540

gggtacgggc aaaccgaagg gaaaccactt aggtggatcc cttggctagc ctgagactga 600

cacaccataa gtgatcggcc gcttttaact acgcctggtg ccgagccaca atagagatgt 660

cggtctgtct cccacttatg acctacgaac ccctcgtact atggctcatc tatgggtcgt 720

gtgccccttg gcttactgcg cactcatgcc ctatcaaggc taggccagag tgcgtaggcc 780

gctttcagag atcactcggt gaaaaaatca ctcggtgatg aaaccggcga actgtcgttg 840

ggtgggtggg tcttactatc aaagaaaacg tattccagca aacgtattcc actctccaca 900

aaataaacat ttctgttcgg ttacctaggt gaggcatcct gtaagaactt ggctgtgttt 960

agtcacagca aacgtatacc actctccaca aaataaaata aaaaacgggt cagtgaagct 1020

gcaattaatc ccttctcttg cttgctggtt gctgccaggg aaatggcatt agtgtttgtt 1080

cccgttccga agaccgcagc aaccccccgga atcggaaacg cctgccccct gcagcaccaa 1140

agaccgtacc aaccccccgca atcgcagttc gcaaaccaaa ctaatttgtg tacacaaacc 1200

ggccccgtct cggttctatt ctataaaacc cccgccagac cgctggcttg ttccgtcgcc 1260

tccgctgtcc gctgcacaga ctgtagtacc ggggcagggg caggggcagg ggcacaaaca 1320

gagccacacc acacacagac cccacctacg ctacgctacg cgcgtgctgg gcgagtgatg 1380

<210> 39
<211> 574

<212> DNA
<213> Zea mays

<400> 39
gtcctaattg gtactcctga gatactatac cctcctgttt taaaatagtt ggcattatcg 60

aattatcatt ttacttttta atgttttctc ttcttttaat atattttatg aattttaatg 120

tattttaaaa tgttatgcag ttcgctctgg acttttctcg tgcgcctaca cttgggtgta 180

ctgggcctaa attcagcctg accgaccgcc tgcattgaat aatggatgag caccggtaaa 240

atccgcgtac ccaactttcg agaagaaccg agacgtggcg ggccgggcca ccgacgcacg 300

gcaccagcga ctgcacacgt cccgccggcg tacgtgtacg tgctgttccc tcactggccg 360

cccaatccac tcatgcatgc ccacgtacac ccctgccgtg gcgcgcccag atcctaatcc 420

tttcgccgtt ctgcacttct gctgcctata aatggcggca tcgaccgtca cctgcttcac 480

caccggcgag ccacatcgag aacacgatcg agcacacaag cacgaagact cgtttaggag 540

aaaccacaaa ccaccaagcc gtgcaagcat catg 574

<210> 40
<211> 571
<212> DNA
<213> Zea mays

<400> 40
ctcgagggta ctcctgagat actataccct cctgttttaa aatagttggc attatcgaat 60

tatcatttta ctttttaatg ttttctcttc ttttaatata ttttatgaat tttaatgtat 120

tttaaaatgt tatgcagttc gctctggact tttctgctgc gcctacactt gggtgtactg 180

ggcctaaatt cagcctgacc gaccgcctgc attgaataat ggatgagcac cggtaaaatc 240

cgcgtaccca actttcgaga agaaccgaga cgtggcgggc cgggccaccg acgcacggca 300

ccagcgactg cacacgtccc gccggcgtac gtgtacgtgc tgttccctca ctggccgccc 360

aatccactca tgcatgccca cgtacacccc tgccgtggcg cgcccagatc ctaatccttt 420

cgccgttctg cacttctgct gcctataaat ggcggcatcg accgtcacct gcttcaccac 480

cggcgagcca catcgagaac acgatcgagc acacaagcac gaagactcgt ttaggagaaa 540

ccacaaacca ccaagccgtg caagcaccat g 571

<210> 41
<211> 733
<212> DNA
<213> Oryza sativa

<400> 41
tagcatatat aaaatcattt gtcagagtga aacaacacat ccaaattaat gacaaatata 60

aattactaat ctactttgat ccatctcatc atttttaaag aaaatactaa aatccattaa 120

aagatcattt tggaaaatta aacttttatt gaaaataaac taactcatgt aaaattatac 180

```
cgttttcctg ttacatgtac aggatataaa ttaacagcgc gccttttggc gcgctgattt    240

tctagtcgaa aagttaaacc ggggtataag tgtagcacct tcgctccact caaagaaaat    300

gtaagccgaa gacttgagaa gcttccagaa tccagagatc gcagcagaaa aggagcgaac    360

aaggcaaacc tctcaaagga aaaaagaaaa ataataaagg aggaaacctg tcaaacacca    420

ccctatgaca agtgggtccc actcgaacca accgtacggc ccccccaccc aaacccgctc    480

ccccctcgct ccgaaaatat ccacctctct agatctttct cgtcgcaaac gcccttccgc    540

ccccgcctcg ccgcgcccat tccaccacct ttccgaacct tccactccct tccagactcc    600

acccccacgt cacccctatt taaacccctc ctcccaccga gcaatcaagc gacaagatcg    660

agaagccaca aaccccagcg cgatccgagg tagaagaaga agaagaagaa gaagaagaag    720

aggcgatcga gag                                                        733
```

<210> 42
<211> 1019
<212> DNA
<213> Oryza sativa

<400> 42

```
aatataccat tcgctaaaaa atttgatttt tctatgacgg agaaagcagt agtgtaagca     60

gagcgcccgt aaacatatcc tcacttttgg ttcatctcat atttttgtaa gatggaggaa    120

acatgagtga aattagagca ccctgtaaac atatcctcat tttggttcgt ctatcagtca    180

cgtaactttg ttatttctgt cggttaccta gtactaatac ctaagatgat aatccactgt    240

aatgggaaga tgagcacggt tttatatctg aaactgaaaa tgggtctgtt ggtcataaaa    300

cttactacct ccgtttcgaa atatatcaaa ctagcttgta ttagattaga cacgatctat    360

tattcaattt ggacagagtc catatagcta tgatatgctt actatttcat attgctttca    420

tgaacttaac ttaaagtttt ggaccacaat gaaagtttca gttcatatca tatggcatac    480

tacttctatt cttttttttt tgttaaaaaa aaactggagc tctcaatttt tttaaagttt    540

gtcctgttac aattttaatc agttctttat tattcctctc cacatcaaca atttttcctc    600

gatgatccgg ttccctttttg acctcactgc actgtcccag atctctcatt aatccaaccc    660

agaaaaaaaa aacagtacaa aataaaatac acaagattca acaaagcaac ctgacctggt    720

cggtgctgta ccacgtggca tctcccctcc atgtcccaat cacttcgaga gacaaaagaa    780

acactcctcc agtggcatcc tgccatgtgt cctccattct tgtacttaat ctcttcttat    840

ttaaggcctc ataatctctt gctttccctt ccctagtaaa tcaaagaaca caaagcatcc    900

aaaacaacac caggaaactt cttttcaatc gatcactcca ctggtgagta gtgagtggct    960

agtgactggt cagttcatca cttgtgaagg ttttgcaatc aggaaaagtt cagaagatc    1019
```

```
<210>  43
<211>  1500
<212>  DNA
<213>  Oryza sativa

<400>  43
tttgatttgg gacaaaaggt tggtgaaatg gacatatttt cacatatata tatgctatat     60

ttttcttctc agtttaccga aaagatgtac ccttatatct cgtcatcgat tttgggtcag    120

gccagaaaac cattggtaac agaatatatg catagttttc tttatcaata aaattaatgt    180

tttatttaaa aatcgataaa ggaacttttt acaaaattag gctagaaatg gtctgtctat    240

tatgacaagg taaacttttg cgacattaat ttggatggca acttcaacaa ttcaaattgt    300

cgttgtccac aaatctcttg gttgtagaag acccacgcgt ctgcaacatt tttgcgccga    360

aaacttaata cataaacttg atttgttggg atacatggtg cagaagatac gatcattaat    420

aattcaaaca gtgcatttca tggtccaact gactgccacg tcattgaacc cgtaatcatt    480

cgctaagcca aatcaaattg gcctcaaatg aattttcagc acgacttttt acgccccaaa    540

aacctagtac tccctccagt tggaaatgta ccctaccaag aaacttgtgt ccgtcacgac    600

gcctgtatca tcaatctagt cctcttttgt aacaaaataa ttttagaaga tttcttttaa    660

tgccgtagaa attaaattaa tcctaatgaa aatcatgtaa aactcacccg ttataaaatg    720

tcactaaccc cctacacggt tggtgtcctc tttgtagccg aaatgcctcc tctttggcca    780

ctgcatctcc acccattttt caaacatctc caactaactt tttgttccat ttgcaaaaat    840

gcaaaatgcg aaatgttaac ttcacacaca cccccctacc actacaaaac tctcaccaac    900

cccaatctag ctatcagttc agaaagcacc ttcccttctt tccctattag agcaagtcta    960

atagtacagc tcactactag cttcaattta tctataacca atctaatagt caattcatac   1020

aatagttgct tattatacta ttaatatatg gtctcacctg tcatacacac agtgtgtctt   1080

atagtccgtg ctgcagctgg ctacatatct gtagcctgct agtcttctct ctcatcgttt   1140

atctcattaa aatatgttta tagctggcta atagcttgct aatagcatgc tattgtacct   1200

gctcttacca ccttctttcc cttttggcaa atggcaatga gtgcaaaaat gcttggaaaa   1260

ataacccccc cccccccacc cccacctgat tatttccagt agggccaaaa tccgggccca   1320

cgtccgcaac ccatgtgggc ccccacatccc ccacaccaac cctctgcacc caaaatcccc   1380

atccccccac tatatataat ccccgccgtt ggatcatcgc cctcagcaga gcagcgcatc   1440

tgcatccaaa accaaaccca aactcgtctt ctccaccgga gcagagcagc ggcggcggca   1500

<210>  44
<211>  1180
<212>  DNA
<213>  Oryza sativa
```

<400> 44
aaaacctctt ctttaacatg taaacgacct ggaggatgtc aactctgaca cgctggcgaa 60

atcatccacc tatgtctttg ccgcggtata ggatgaacat gggtagagaa aaaaatcggg 120

gtgatccaaa gtgcaataga cgtgacccaa aaagtgtaat tcactaaaaa aaacttacca 180

acgaagcaat gctttggcag tgatttttac ctttcagtca tgggcatgac ctgcattgta 240

aaataacgtg gttgtgaatt caaactcaaa tgtgtttttc tttcacaagt tgccgttaaa 300

aatatgtttc gcaagagact cactgctccc agtgaaagca gtgaattgaa gcattcccga 360

aacccactgg aatgatctag tactcactct acgatgtaca gtgaagtaat acttcaaaac 420

tggtgtaatt tggtatgcca aaaggactcc atagtttcac gacatatttc caaacggttc 480

aggatcagta ctgcccatct gcctggggcc cacactagcg ggcaattggt tctcgtagtt 540

tctcgttctc aatcaatcat tccatactcg ctatcccctc catcacagaa taaatgcaac 600

aatgagtttc cgtgtacaaa tttaatcgtt cgtcttattt aaaatatttt ttaaaaaact 660

aaaaaacaaa agtcacgcat aaagtactat tcatgtttta taatctaata acagtataaa 720

tactaatcat aaaaaaaaat tcaaataaga tggacgatta aagttgaaca ctgaaattca 780

tggctgcttt tgttttgaga ctgagggagt acacgataag atttgatcgc aatcaaagta 840

acctacatca aagaagcaag atatgtgggg gaaaaatgaa tactctagag caaattaagg 900

tgagccccgc tttgtagagg ctgatggagt actggagcga cggaagcgaa gcagatcgag 960

tgtgctgtaa agcgaaacga gcaagaacca gagaagtcca gagatttcag gacagattag 1020

ttgtgaacct ataaatatcc tgcctcattc cccaacctcc atccatcgag ccaagactga 1080

agcatttgat cgagctccaa acaaacactc gttccaaact tcctccaatc cacttcatac 1140

aaagaaacct aagcagctag cgatccacga caaaccaaca 1180

<210> 45
<211> 339
<212> DNA
<213> Oryza sativa

<400> 45
gttcgtgact tttggcaagg gatcgaatcg gaagcgaatg ggtgggccca aaacgggccg 60

gttattttac tgggactaaa gatatcggcc catctgaatt gtgcgttccg ccggataagg 120

gataactgaa ggcggcgctc agtcccgcgc cttctggaac cttcccgtgg aaggggcata 180

cagccttgca gcggcagctt ccggaagctt ctgaattctt ctccaagatt tgccgcgacg 240

ataaatcctc tcgtttctcc gctcgctgat tcattctcaa cgcaaaatcc aaaagataag 300

cacagttacg cggcgagagc gagagaggag tggagagcc 339

<210> 46

<211> 991
<212> DNA
<213> Oryza sativa

<400> 46
tgcttgccct tgtcctcatg tacacaatca gcttgcttat ctctcccata ctggtcgttt 60

gtttcccgtg gccgaaatag aagaagacag aggtaggttt tgttagagaa ttttagtggt 120

attgtagcct atttgtaatt ttgttgtact ttattgtatt aatcaataaa ggtgtttcat 180

tctattttga ctcaatgttg aatccattga tctcttggtg ttgcactcag tatgttagaa 240

tattacattc cgttgaaaca atcttggtta agggttggaa catttttatc tgttcgtgaa 300

acatccgtaa tattttcgtt gaaacaattt ttatcgacag caccgtccaa caatttacac 360

caatttggac gtgtgataca tagcagtccc caagtgaaac tgaccaccag ttgaaaggta 420

tacaaagtga acttattcat ctaaaagacc gcagagatgg gccgtgggcc gtggcctgcg 480

aaacgcagcg ttcaggccca tgagcattta ttttttaaaa aaatatttca caacaaaaaa 540

gagaacggat aaaatccatc gaaaaaaaaa actttcctac gcatcctctc ctatctccat 600

ccacggcgag cactcatcca aaccgtccat ccacgcgcac agtacacaca catagttatc 660

gtctctcccc ccgatgagtc accacccgtg tcttcgagaa acgcctcgcc cgacaccgta 720

cgtggcgcca ccgccgcgcc tgccgcctgg acacgtccgg ctcctctcca cgccgcgctg 780

gccaccgtcc accggctccc gcacacgtct ccctgtctcc ctccacccat gccgtggcaa 840

tcgagctcat ctcctcgcct cctccggctt ataaatggcg gccaccacct tcacctgctt 900

gcacaccaca gcaagagcta agtgagctag ccactgatca gaagaacacc tcgatctccg 960

agagtttttt ttcagcttta gcttaagcag g 991

<210> 47
<211> 368
<212> DNA
<213> Zea mays

<400> 47
atctctctct ctctctctaa aagaaggaac acgtatcggt agatcgattc cagacgctta 60

ccatcgcatc gttatcatag agttaaaatc gtctgtgtcg cgtaacattt ctgaaaataa 120

tttcggaaga tgaagataat gtttctgtta cgtaattttc cgtcccttgt tgttcacatg 180

tgcgtaccca cgtgtcagag tgagagatgt accagtataa agaagcgcag agcccccaag 240

agccggagct gccatgaggc ccgaacagtt gaagctacta gcgtgtagct agggaagaga 300

agcgcgcgag tagctagcag acgtacgtag gcagaagcct agctgggttt gaagggcccc 360

gatcgatg 368

<210> 48
<211> 2091

<212> DNA
<213> Zea mays

<400> 48
atccacgctc gctcgggtgt cgggtcagat cgatccagtt ggcgcacgta ataatccttt 60

tccccagaag gagtcgaacc cctcctcccc gtccaatcca atcaaagcga ccaatcgact 120

ggctgtccta cacacacaca aaaccgaccg aggcgacaca ccgcagcagt gatcattctg 180

agcatttgca gaaaaaggag aacgtcccga aatcctggtg gttgtattgt gtgattgctc 240

actcagtccg tgcagggtca gggtgaagcc aagccaacaa cccaacgctc gctgggagta 300

gggtccaccg gatttattgg cagtacatcg ctgtttggtc ctcctgccct tcgcttattt 360

tttaattcgg cagacgtgca cagacagggc accaccggac caaggaaggg cgcacaccgt 420

cgtcagtcac caggtgggtg tgatcagcag ccgcttctct tgtgctgctt tatagcgtat 480

gaaattccag tgtccctgtt ccacctgcat gcaattggtt tgactgaaca acatgatagc 540

aagtgatact atatatattt ttatagagga acacagtgaa aaaatattta gtattattac 600

gtgcatgaaa ttgtattcac agttatccct gatgcaacgc aattgttcaa tatatagcag 660

tatatattat acgaagtata tatgtatatc taattttatg agaccgggag aaggtgtatt 720

cacagtacag tgcagggcca tggccatgca gcccttgggg cctgaaaagg gtcgcgtgaa 780

gtggccaacg ctgtgcaatt gcaaccaaac aaacttttgg tggcggggtc cctgtccctg 840

gccggctttg cccacaggcc acagcgcatc acaccaccgc tttatagcgc caccccacca 900

ccctcgtctc tccccccgtc gagcacacaa cacaccctcc tcgtcctcca atccaatcaa 960

cctggtagac tcgcttcgct tctcccccca gctcggacgg agctcctcgc agcagccgcc 1020

gatcaacctg cgctcgggct cagcgctgga aggtgagagc tcagtgcctc gtcccgcccg 1080

ccccaaatct ggttcttgtg ctggctctgg ctgtgcgctg cacgaattct gcatctggtt 1140

ctttcgagac gcaattcccg gaccgtgggc tttggtttcg gagggggccg agagtaaggc 1200

gttaggactt tctccgagct gcaaggccgc tcgtcgttgc ggcatttttc gtttcgcttg 1260

tcctgtgatg agagatgtgc atttcccttt ggcgggctta ccgttccctg ctcgtctgta 1320

tgtgtgtatg tttgtgtgac ctttccctca acgccaggct cttctcccct cttgctgttt 1380

ctttcagcag tacagacgcg catctgtaca gcgcctttct tcggtcctgg gttatgattg 1440

atccgttaac agttggtcac caagtgctgg ctgtttaata tgtactataa gcttcttggt 1500

gccgctgcct ctgcctatac gactttatgc gctgcctgca caagtctcag ccatctgtgg 1560

gaacgtgtgt ctctcaccta cctttcatat tgcactagct ggattgaatc attctgcttt 1620

ggagagatgt ccggtcattt ttttttaaat cattttcatc tcgcgtacta gtttttgttt 1680

tgttttgcga gagagtaatt tttttttaat atttactgtc tcctgtccca tttgctgttt 1740

ctttacccag aaatttccac cagattcagt caaacgaaac tcctgtgctc ttttttttct 1800

ccctttcaaa agggtgtgta accgactacc gactcagata atataagtgc ggtcacatat 1860

cacatgatat catctcgcct ctctcccttc tcctgtgttt tattttcctt ttttctaacc 1920

acagcgtgat gaacttcttt tttttttggg gggggggggg ggggtaacta cagcttagcg 1980

aacatgaatg ggtagtttta caactaatgc aacggctggt tcactgaaca actgtaggtg 2040

ttggaagaga atagcctgaa ggttcacagt aaccttcatc tgtcggaagc c 2091

<210> 49
<211> 1106
<212> DNA
<213> Zea mays

<400> 49
atccacgctc gctcgggtgt cgggtcagat cgatccagtt ggcgcacgta ataatccttt 60

tccccagaag gagtcgaacc cctcctcccc gtccaatcca atcaaagcga ccaatcgact 120

ggctgtccta cacacacaca aaaccgaccg aggcgacaca ccgcagcagt gatcattctg 180

agcatttgca gaaaaaggag aacgtcccga aatcctggtg gttgtattgt gtgattgctc 240

actcagtccg tgcagggtca gggtgaagcc aagccaacaa cccaacgctc gctgggagta 300

gggtccaccg gatttattgg cagtacatcg ctgtttggtc ctcctgccct tcgcttattt 360

tttaattcgg cagacgtgca cagacagggc accaccggac caaggaaggg cgcacaccgt 420

cgtcagtcac caggtgggtg tgatcagcag ccgcttctct tgtgctgctt tatagcgtat 480

gaaattccag tgtccctgtt ccacctgcat gcaattggtt tgactgaaca acatgatagc 540

aagtgatact atatatattt ttatagagga acacagtgaa aaaatattta gtattattac 600

gtgcatgaaa ttgtattcac agttatccct gatgcaacgc aattgttcaa tatatagcag 660

tatatattat acgaagtata tatgtatatc taattttatg agaccgggag aaggtgtatt 720

cacagtacag tgcagggcca tggccatgca gcccttgggg cctgaaaagg gtcgcgtgaa 780

gtggccaacg ctgtgcaatt gcaaccaaac aaacttttgg tggcggggtc cctgtccctg 840

gccggctttg cccacaggcc acagcgcatc acaccaccgc tttatagcgc caccccacca 900

ccctcgtctc tccccccgtc gagcacacaa cacaccctcc tcgtcctcca atccaatcaa 960

cctggtagac tcgcttcgct tctcccccca gctcggacgg agctcctcgc agcagccgcc 1020

gatcaacctg cgctcgggct cagcgctgga aggtgttgga agagaatagc ctgaaggttc 1080

acagtaacct tcatctgtcg gaagcc 1106

<210> 50
<211> 1597
<212> DNA
<213> Zea mays

<400> 50

gatccacgct cgctcgggtg tcgggtcaga tcgatccagt tggcgcacgt aataatcctt 60
ttccccagaa ggagtcgaac ccctcctccc cgtccaatcc aatcaaagcg accaatcgac 120
tggctgtcct acacacacac aaaaccgacc gaggcgacac accgcagcag tgatcattct 180
gagcatttgc agaaaaagga gaacgtcccg aaatcctggt ggttgtattg tgtgattgct 240
cactcagtcc gtgcagggtc agggtgaagc caagccaaca acccaacgct cgctgggagt 300
agggtccacc ggatttattg gcagtacatc gctgtttggt cctcctgccc ttcgcttatt 360
ttttaattcg gcagacgtgc acagacaggg caccaccgga ccaaggaagg gcgcacaccg 420
tcgtcagtca ccaggtgggt gtgatcagca gccgcttctc ttgtgctgct ttatagcgta 480
tgaaattcca gtgtccctgt tccacctgca tgcaattggt ttgactgaac aacatgatag 540
caagtgatac tatatatatt tttatagagg aacacagtga aaaaatattt agtattatta 600
cgtgcatgaa attgtattca cagttatccc tgatgcaacg caattgttca atatatagca 660
gtatatatta tacgaagtat atatgtatat ctaattttat gagaccggga gaaggtgtat 720
tcacagtaca gtgcagggcc atggccatgc agcccttggg gcctgaaaag ggtcgcgtga 780
agtggccaac gctgtgcaat tgcaaccaaa caaacttttg gtggcggggt ccctgtccct 840
ggccggcttt gcccacaggc cacagcgcat cacaccaccg ctttatagcg ccaccccacc 900
accctcgtct ctccccccgt cgagcacaca acacaccctc ctcgtcctcc aatccaatca 960
acctggtaga ctcgcttcgc ttctcccccc agctcggacg gagctcctcg cagcagccgc 1020
cgatcaacct gcgctcgggc tcagcgctgg aaggtgttgg aagagaatag cctgaaggtt 1080
cacagtaacc ttcatctgtc ggaagccctc cgccgccgcc ggtaaccacc ccgcccctct 1140
cctctttctt tctccgtttt tttttccgtc tcggtctcga tctttggcct tggtagtttg 1200
ggtgggcgag aggcggcttc gtgcgcgccc agatcggtgc gcgggagggg cgggatctcg 1260
cggctggggc tctcgccggc gtggatccgg cccggatctc gcggggaatg gggctctcgg 1320
atgtagatct gcgatccgcc gttgttgggg gagatgatgg ggggtttaaa atttccgccg 1380
tgctaaacaa gatcaggaag aggggaaaag ggcactatgg tttatatttt tatatatttc 1440
tgctgcttcg tcaggcttag atgtgctaga tctttctttc ttctttttgt gggtagaatt 1500
tgaatccctc agcattgttc atcggtagtt tttcttttca tgatttgtga caaatgcagc 1560
ctcgtgcgga gctttttgt aggtagaagt gatcaac 1597

<210> 51
<211> 907
<212> DNA
<213> Zea mays

<400> 51
atccacgctc gctcgggtgt cgggtcagat cgatccagtt ggcgcacgta ataatccttt 60

```
tccccagaag gagtcgaacc cctcctcccc gtccaatcca atcaaagcga ccaatcgact    120
ggctgtccta cacacacaca aaaccgaccg aggcgacaca ccgcagcagt gatcattctg    180
agcatttgca gaaaaaggag aacgtcccga aatcctggtg gttgtattgt gtgattgctc    240
actcagtccg tgcagggtca gggtgaagcc aagccaacaa cccaacgctc gctgggagta    300
gggtccaccg gatttattgg cagtacatcg ctgtttggtc ctcctgccct tcgcttattt    360
tttaattcgg cagacgtgca cagacagggc accaccggac caaggaaggg cgcacaccgt    420
cgtcagtcac caggtgggtg tgatcagcag ccgcttctct tgtgctgctt tatagcgtat    480
gaaattccag tgtccctgtt ccacctgcat gcaattggtt tgactgaaca acatgatagc    540
aagtgatact atatatattt ttatagagga acacagtgaa aaaatattta gtattattac    600
gtgcatgaaa ttgtattcac agttatccct gatgcaacgc aattgttcaa tatatagcag    660
tatatattat acgaagtata tatgtatatc taattttatg agaccgggag aaggtgtatt    720
cacagtacag tgcagggcca tggccatgca gcccttgggg cctgaaaagg gtcgcgtgaa    780
gtggccaacg ctgtgcaatt gcaaccaaac aaacttttgg tggcggggtc cctgtccctg    840
gccggctttg cccacaggcc acagcgcatc acaccaccgc tttatagcgc caccccacca    900
ccctcgt                                                              907
```

<210> 52
<211> 1039
<212> DNA
<213> Zea mays

<400> 52

```
gtgagagctc agtgcctcgt cccgcccgcc ccaaatctgg ttcttgtgct ggctctggct     60
gtgcgctgca cgaattctgc atctggttct ttcgagacgc aattccccgga ccgtgggctt    120
tggtttcgga gggggccgag agtaaggcgt taggactttc tccgagctgc aaggccgctc    180
gtcgttgcgg catttttcgt ttcgcttgtc ctgtgatgag agatgtgcat ttccctttgg    240
cgggcttacc gttccctgct cgtctgtatg tgtgtatgtt tgtgtgacct ttccctcaac    300
gccaggctct tctcccctct tgctgtttct ttcagcagta cagacgcgca tctgtacagc    360
gcctttcttc ggtcctgggt tatgattgat ccgttaacag ttggtcacca agtgctggct    420
gtttaatatg tactataagc ttcttggtgc cgctgcctct gcctatacga ctttatgcgc    480
tgcctgcaca agtctcagcc atctgtggga acgtgtgtct ctcacctacc tttcatattg    540
cactagctgg attgaatcat tctgctttgg agagatgtcc ggtcattttt ttttaaatca    600
ttttcatctc gcgtactagt ttttgttttg ttttgcgaga gagtaatttt tttttaatat    660
ttactgtctc ctgtcccatt tgctgtttct ttacccagaa atttccacca gattcagtca    720
```

```
aacgaaactc ctgtgctctt ttttttctcc ctttcaaaag ggtgtgtaac cgactaccga     780
ctcagataat ataagtgcgg tcacatatca catgatatca tctcgcctct ctcccttctc     840
ctgtgtttta ttttcctttt ttctaaccac agcgtgatga acttcttttt tttttggggg     900
gggggggggg ggtaactaca gcttagcgaa catgaatggg tagttttaca actaatgcaa     960
cggctggttc actgaacaac tgtaggtgtt ggaagagaat agcctgaagg ttcacagtaa    1020
ccttcatctg tcggaagcc                                                 1039

<210>  53
<211>  3530
<212>  DNA
<213>  Oryza sativa

<400>  53
ggcttcccgc tgtgagagaa gtggctgcct ctcggttctc accaagcagt cgaaaatgcc      60
agaacagcga ccagatagga tcatcgtgcc atgcaggcat gcagcctttg agaactgaaa     120
gagccggtga aagtcctgca aagcgaaaag caaatgaaca aacatctgcc tgtgctgctg     180
cctcgcctcg ctgtcctttt ccggtgggtt gccgctgcta acctctgcct ccgcgatacg     240
tgacacgtca tcctcccccc accccacccc atgcttgcac cccccccccc ccccctccct     300
cccttattac caccaccccc ctcctccatc ctcctctgct cctccaacct ggctcagttt     360
cctcctgttc ttgagagaac tgaatctgct gtccagctgc tgctccggct ggtctctgag     420
ttgaaggtaa ggttaatcgg tgtctctcag cctgaatgaa tttgtctatc tcctatggct     480
ttgtggtggt tgaattttgc gttctgggga tgttaggacc ttcttgttgg gacaatttct     540
gagattctgg cttgctttgg atgggttggg ggaagagtta ggtgcttgct ggtgtgtatt     600
tcttttgcat ttcggttgtc ctgtgaagga tgcgtgttct cggcatttcc agctcattgt     660
gtctttgccc ttcagataac agttatcctc gtgcttttcc tttttctttc agcaaaacat     720
atctggactt ctgtacaagt gctttttttt tcttctttgg acgatatttt gttttgcatt     780
ctagtgattc tgtacaagtg ccatctgata gcatatcatc attcggcaac tggtgatttc     840
ccctatgcgg tctttcatgt acttgcatga ttgaacatat ggcagtgctt ctgtcggatg     900
ctatcgatgt tttacttgcg aaaggccggg gaactttttg cagatcttga ggtttttagt     960
agtgatgcag tcattcaaaa agataacctt gtgctggtcc ttttgccttt cggctgcact    1020
tgcatgtgcg ttttctcaga gatgctgcat ctccagctca gcttctgtca tcagtcatta    1080
gtcattgcca tcttttatgt ggataaagtc aaagttaatt tagcaccgct gttttggagt    1140
tgtttggtca ttttatatat tttcatcagg tgttgtttac tgttcctggt actaaaattt    1200
cgaatttaca aatgactacc tcattctcct ttcttttttt ccttttttcct tgtggcagac   1260
cggttcctga aagaatattc ttttcatgaa atgtacttcc ggttttttaa atagaatgat    1320
```

taaattacag taggatgaat aactaaattt gtcgatatgc cttgtaccgg tactccttgt 1380
tagttcttag tgaatctatg tatactattg cttgtcaaat tgtgaatttt actatcagct 1440
gtatgtatgt ctattgaaga actctcaaca gttctaactt cctaagatgt tttaatcaat 1500
tcttgctacc aacctggata cagtattctc cgtagttttt tcttcatttt ttttttaaag 1560
agatctattg agagtccttg gtacccatct tctgtagaat tgtccatgtg aacagttgct 1620
tcaagatttc tgctgcatct gtgatacggt atcactgata ctgtagtgat cagatccaaa 1680
acacatatat agttcgccac cattctaaaa cacatgtttg tgtgatcaga tctagctcgc 1740
caccatatat agttcaggtt ttcaagttgt aatatcactt gccttttgcg atagatatga 1800
caacacactt tgtgtcaggc tgtcccaatt ttctctgaat tttctctcat atatcatgat 1860
ttagttatgg cttttgttcc ttgacatttc aatgtctaat tgtccaatgt taagtaaatc 1920
cttttcatag cctgatttat tgaatacttg caggtacttg aataacttga aggttcctag 1980
gaaccttcat ttgttggaag atgtataggg ctaagagggc tgcattatct ccaaaggtga 2040
agcgccgtgt agggaagtat gagctcgggc gcaccattgg agaaggaacc tttgcaaagg 2100
tccggtttgc gaagaacact gaaaatgacg aaccagttgc tatcaaaatc cttgacaagg 2160
agaaggttca gaagcacaga ttggttgaac agattaggcg tgaaatttgt actatgaagt 2220
tagtaaagca tcctaatgtt gttcggctgt tcgaggtcat gggaagtaaa gcaagaattt 2280
tcattgttct ggaatatgtt actggaggag agctctttga aatcattgca actaatggaa 2340
ggttgaagga ggaggaagca cgaaaatact ttcaacaact tatcaatgca gttgactact 2400
gccacagtag ggtgtgtac cacagagact tgaagttaga aaatttgctg cttgatgctt 2460
ctggaaacct gaaagtatct gactttggtt tgagtgcttt aaccgagcaa gtgaaggctg 2520
acggtttgct gcacacgaca tgtggaactc ctaattatgt tgctccagag gtgattgagg 2580
acagaggcta tgatggggca gctgcagata tctggtcttg tggggtaatc ctttatgttc 2640
tgcttgctgg gtttttacca tttgaggatg acaacatcat tgctctttat aaaaagatct 2700
ctgaagctca gtttacctgt ccctcttggt ttctactgg agctaagaag ctgatcacca 2760
gaattctgga tcccaaccct acaactagga tcaccatttc tcaaatactg gaagatcctt 2820
ggttcaaaaa gggttacaaa ccgcctgtat ttgacgagaa atatgaaact agttttgacg 2880
atgtcgatgc tgcttttgga gactccgaag accggcatgt caaagaagaa actgaagatc 2940
agcctacctc tatgaacgcg tttgaactca tttcactgaa tcaggcactg aatctggaca 3000
atttgttcga ggcaaaaaag gagtataaaa gagagacaag attcacatca caatgtcctc 3060
caaaagaaat tatcaccaag attgaagaag ctgcaaagcc acttggattt gatattcaaa 3120
agaaaaatta caagatgcgc atggagaacc tgaaagcagg tagaaaaggc aatctcaatg 3180

ttgcaactga ggttttccaa gtagctccat ccttacatgt ggttgagctc aagaaggcaa 3240

agggggacac tctggagttc caaaaggtgc cattctttga caccggaaat ttcgctattt 3300

ccaacttgct atttactgcc aagtttaacc aaaatcaatt ctgctgtgaa acaacagttc 3360

tacagaaccc tgtcgaccca gctcaaggac gtggtctgga agtgcgacgg cgaggtcgaa 3420

ggcaacggcg ccgcggcgtg aacgtggttt ttgccatggc tttcggggca ccggttcttc 3480

gtgtacatag ctgctctgcc atcatcaatg gggtgttcgc cgtagagtag 3530

<210> 54
<211> 769
<212> DNA
<213> Rice tungro bacilliform virus

<400> 54
gatctcctac aaaagggagt agtaatattt aatgagcttg aaggaggata tcaactctct 60

ccaaggttta ttggagacct ttatgctcat ggttttatta aacaaataaa cttcacaacc 120

aaggttcctg aagggctacc gccaatcata gcggaaaaac ttcaagacta taagttccct 180

ggatcaaata ccgtcttaat agaacgagag attcctcgct ggaacttcaa tgaaatgaaa 240

agagaaacac agatgaggac caacttatat atcttcaaga attatcgctg tttctatggc 300

tattcaccat taaggccata cgaacctata actcctgaag aatttgggtt tgattactac 360

agttgggaaa atatggttga tgaagacgaa ggagaagttg tatacatctc caagtatact 420

aagattatca aagtcactaa agagcatgca tgggcttggc cagaacatga tggagacaca 480

atgtcctgca ccacatcaat agaagatgaa tggatccatc gtatggacaa tgcttaaaga 540

agctttatca aaagcaactt taagtacgaa tcaataaaga aggaccagaa gatataaagc 600

gggaacatct tcacatgcta ccacatggct agcatcttta ctttagcatc tctattattg 660

taagagtgta taatgaccag tgtgcccctg gactccagta tataaggagc accagagtag 720

tgtaatagat catcgatcaa gcaagcgaga cgtcaaactt ctaagagag 769

<210> 55
<211> 697
<212> DNA
<213> Zea mays

<400> 55
cggcccgggc tggtaaatat cggaatatta gcatgtcaac ttgcactctc taaggctcct 60

ttggaaagca ggattttaga aaaaaaaatc atataaattt tttacatgaa tcagtttatt 120

ttcggattat gaaatatttt ctcataacag tataacacat attttgtata taagttatta 180

tgttattata tataaccgtt gcaacgtacg ggcattcacc tagtaaagaa agaagattaa 240

ttattctctg gtggagattg tgcccgagcc cgaaggtcat gatatggacg ttgcaaaccc 300

acttcacgag ggacaaaaa agaaataggg ttaccacttt catcagttaa agggcgtgac 360

```
atggacgtgt tgaagatccg gcacattccc tgcgaaatat acacgtcatg gtactaacga    420
ggcatgaaac tggccacatg gccatggacg cgtgaagcgt gccatgcatt ggacatgcgg    480
catccgaact tctgaagatc atatcagaga gacactgatg tacgaactgc cgtaacattc    540
tattctatat ataccctcag tccctgttcc agttctcgtt aagctagcag caccaagttg    600
tcgaacactt gcctgctctt gagctcgatc aagctatcat cagctgcgtc ttgcgcacag    660
caacagcttc ccaactgcaa ccgtagcagc cagatct                             697
```

```
<210>  56
<211>  1455
<212>  DNA
<213>  Coix lacryma-jobi

<400>  56
cggtacctcg cgaatgcatc tagatcccct ttccgcgggc aattcgatag gtagaaatcc     60
gccgggttga taccccctgca atcgtagcgt gatgagggtg tgaatgttgc cgatgtgtgg    120
acttcgagga aaatgatagc ccctggaatg ccgagatagc cgaagtcgag gtggtcgtgg    180
tcgggagaca cgcagcagta gcctattctt tggtaggggt cgatgttcaa gcgtcaacga    240
tcggctgggc gacataaaaa ttagcaccag ggtgaccttc ttgcttcttc gatcgtctgg    300
acgtcgagga gccccgcggc agcgcacgcg tctgcaccgt tatggtggcc gcgctcgcga    360
tggaatagaa ggggtaatga tggatccggc caggaaggcc acgacatcga cggatccaac    420
cggcaagacg gcgatccggt taaatagacg acggatctag ctgggaaggt agactctata    480
ttaaatgagg ttgtacatgc cctaataact ttataaatct aatttattca gaggcaaggt    540
agtagtatta tctttcccaa cggatagtta tctgatctgc cgttcagctt gatcgataac    600
tttataaatc taatttattc agaggccggc ggcagcgcac acgtctgcac cagtaatgtt    660
agccgcgcct gtggcgtaat agaaggggta acgatggatc cgaccagaaa ggcctcgaca    720
tcgacggatc cagacggcga tccggtcaaa gagacgacga atctagccga gaaggtagat    780
ctctcgagag agttcatatt aaatgatgtt gtacatgcca taataactct ataaatctaa    840
tttattcata ggcgaaggta gtagtattat ctttcccagc ggatcgttat ctgatctgcc    900
gttcagcttg atcgatccac gtcgtttgat ctcggcgagc agcacatggc ggctcttctt    960
gtgtacaggt ctcactctct gctacttcag tgcaaggcgg agtgaacgca cacaataacg   1020
tgagtattgt gggaactacc ttgtagatgc aaacgatgta aatccacctg ctccaccaag   1080
tgcccgcccg gctctatcca ttccattcgt caacatgcag gttcaagact ggcccgtgct   1140
ggaccagtga gcggtgccgg tggaccccaa tgcaagcgaa gcgagtgacc atcggggaag   1200
cctcccgtgc tgccccccaca tggcttgcct gaatgcctct ctctcgccgc agtgccctct   1260
ctctctcctc ctcctctccg tcgaagggcg tcacgagagc ccagagggca tccgaggccc   1320
```

ccaccccacc ccttcctccc gtgtatataa gcagtggcag ggtgagcgtc tctcctcaga 1380

ccaccactgc gccattggcc agctagagcc aaccagaaga gcttgcagtt actgagagtg 1440

tgtgagagag agagg 1455

<210> 57
<211> 5365
<212> DNA
<213> Artificial

<220>
<223> various organisms

<400> 57
aggatttttc ggcgctgcgc tacgtccgcg accgcgttga gggatcaagc cacagcagcc 60

cactcgacct tctagccgac ccagacgagc caagggatct tttggaatg ctgctccgtc 120

gtcaggcttt ccgacgtttg ggtggttgaa cagaagtcat tatcgcacgg aatgccaagc 180

actcccgagg ggaaccctgt ggttggcatg cacatacaaa tggacgaacg gataaacctt 240

ttcacgccct tttaaatatc cgattattct aataaacgct cttttctctt aggtttaccc 300

gccaatatat cctgtcaaac actgatagtt taaactgaag gcgggaaacg acaatctgat 360

ccccatcaag ctagcttctg caggtcctgc tcgaggtcat tcatatgctt gagaagagag 420

tcgggatagt ccaaaataaa acaaaggtaa gattacctgg tcaaaagtga aaacatcagt 480

taaaaggtgg tataaagtaa aatatcggta ataaaaggtg gcccaaagtg aaatttactc 540

ttttctacta ttataaaaat tgaggatgtt tttgtcggta ctttgatacg tcatttttgt 600

atgaattggt ttttaagttt attcgctttt ggaaatgcat atctgtattt gagtcgggtt 660

ttaagttcgt ttgcttttgt aaatacagag ggatttgtat aagaaatatc tttaaaaaaa 720

cccatatgct aatttgacat aatttttgag aaaaatatat attcaggcga attctcacaa 780

tgaacaataa taagattaaa atagctttcc cccgttgcag cgcatgggta ttttttctag 840

taaaaataaa agataaactt agactcaaaa catttacaaa aacaacccct aaagtcctaa 900

agcccaaagt gctatccacg atccatagca agcccagccc aacccaaccc aacccaaccc 960

accccagtcc agccaactgg acaatagtct ccacaccccc ccactatcac cgtgagttgt 1020

ccgcacgcac cgcacgtctc gcagccaaaa aaaaaaaaag aaagaaaaaa aagaaaaaga 1080

aaaaacagca ggtgggtccg ggtcgtgggg gccggaaacg cgaggaggat cgcgagccag 1140

cgacgaggcc ggccctccct ccgcttccaa agaaacgccc cccatcgcca ctatatacat 1200

acccccccct ctcctcccat ccccccaacc ctaccaccac caccaccacc acctcctccc 1260

ccctcgctgc cggacgacga gctcctcccc cctccccctc cgccgccgcc ggtaaccacc 1320

ccgcccctct cctctttctt tctccgtttt ttttttccgt ctcggtctcg atctttggcc 1380

ttggtagttt gggtgggcga gaggcggctt cgtgcgcgcc cagatcggtg cgcgggaggg 1440
gcgggatctc gcggctgggg ctctcgccgg cgtggatccg gcccggatct cgcggggaat 1500
ggggctctcg gatgtagatc tgcgatccgc cgttgttggg ggagatgatg gggggtttaa 1560
aatttccgcc atgctaaaca agatcaggaa gaggggaaaa gggcactatg gtttatattt 1620
ttatatattt ctgctgcttc gtcaggctta gatgtgctag atctttcttt cttctttttg 1680
tgggtagaat ttgaatccct cagcattgtt catcggtagt ttttcttttc atgatttgtg 1740
acaaatgcag cctcgtgcgg agcttttttg taggtagacc atggtcccct acgcgactgc 1800
ggcggaggcg gagggagcac tggggcgcac catgacgtgg gctgagacag catggtacga 1860
gtactcggcg gtgatgccag attcctggct gcactgccac accacattta tcctgttcgt 1920
catctacagc atcgccccgc tgcccctgct actcctagag cagttcgctc cgtccgtcgt 1980
gctgccgtac aagctgcagc cccgggtacg gctgcccccg gcagcctccc tcagctgcta 2040
catggacgcg gcctgcatct ttccgctcgc cgttggcctt cagttcgtct cctatcctgc 2100
ggtcgccaag atactaagga cccgaatggg actgccgttg ccgtcggtga gggagaccat 2160
cgcgcagcta gtcgtatact ctctagtgga ggattacctc agctactgga tgcaccgtct 2220
gctgcacacc cagtggtgct acgagaagat ccaccgcgtc caccacgagt tcacggctcc 2280
tacaggcttc gccatgtcgt acagccactg ggccgagaac gtcgtccttt ctatcccggc 2340
cttggccggc ccagtgctcg tgccatgcca tgtcaccacg cagtggctat ggttctccat 2400
ccgcctaatt gagggcatta acacgcacag cggttaccat ttcccgttca gcccttgcag 2460
gctgattcca ttctacggag ggctgcata ccatgactac catcactatg caggaggccg 2520
tagccaaagc aactttgcac ccctgttcac ctactgtgat tatttatata ggacagacaa 2580
aggctacaga taccacaagc taaagcaaga gaagctgaag agtctagcag aaaatagtgc 2640
ggataaagga ggcaactact cattcgacga agggaaaaag aacagatatt tttgtgcctg 2700
agcgtacgaa gaataatcaa ggctattact tcgtcctgtt cgaagggccc ggggggatcca 2760
ctagttctag ctatatcatc aatttatgta ttacacataa tatcgcactc agtctttcat 2820
ctacggcaat gtaccagctg atataatcag ttattgaaat atttctgaat ttaaacttgc 2880
atcaataaat ttatgttttt gcttggacta taatacctga cttgttattt tatcaataaa 2940
tatttaaact atatttcttt caagatatca ttctttacaa gtatacgtgt ttaaattgaa 3000
taccataaat ttttattttt caaatacatg taaaattatg aaatgggagt ggtggcgacc 3060
gagctcaagc acacttcaat tcctataacg gaccaaatcg caaaaattat aataacatat 3120
tatttcatcc tggattaaaa gaaagtcacc ggggattatt ttgtgacgcc gattacatac 3180
ggcgacaata aagacattgg aaatcgtagt acatattgga atacactgat tatattaatg 3240

atgaatacat actttaatat ccttacgtag gatcgatccg aattcgcgac acgcggccgc 3300
tctagaacta gtggatcccc cccttaatta agggggctgc aggaattcat aacttcgtat 3360
aatgtatgct atacgaagtt atagcttggt cgagtggaag ctagctttcc gatcctacct 3420
gtcacttcat caaaaggaca gtagaaaagg aaggtggcac ctacaaatgc catcattgcg 3480
ataaaggaaa ggctatcatt caagatgcct ctgccgacag tggtcccaaa gatggacccc 3540
cacccacgag gagcatcgtg gaaaaagaag acgttccaac cacgtcttca aagcaagtgg 3600
attgatgtga tacttccact gacgtaaggg atgacgcaca atcccactat ccttcgcaag 3660
acccttcctc tatataagga agttcatttc atttggagag gacacgctga aatcaccagt 3720
ctctctctac aagatcgggg atctctagct agacgatcgt ttcgcatgat tgaacaagat 3780
ggattgcacg caggttctcc ggccgcttgg gtggagaggc tattcggcta tgactgggca 3840
caacagacaa tcggctgctc tgatgccgcc gtgttccggc tgtcagcgca ggggcgcccg 3900
gttctttttg tcaagaccga cctgtccggt gccctgaatg aactgcagga cgaggcagcg 3960
cggctatcgt ggctggccac gacgggcgtt ccttgcgcag ctgtgctcga cgttgtcact 4020
gaagcgggaa gggactggct gctattgggc gaagtgccgg ggcaggatct cctgtcatct 4080
caccttgctc ctgccgagaa agtatccatc atggctgatg caatgcggcg gctgcatacg 4140
cttgatccgg ctacctgccc attcgaccac caagcgaaac atcgcatcga gcgagcacgt 4200
actcggatgg aagccggtct tgtcgatcag gatgatctgg acgaagagca tcaggggctc 4260
gcgccagccg aactgttcgc caggctcaag gcgcgcatgc ccgacggcga ggatctcgtc 4320
gtgacgcatg gcgatgcctg cttgccgaat atcatggtgg aaaatggccg cttttctgga 4380
ttcatcgact gtggccggct gggtgtggcg gaccgctatc aggacatagc gttggctacc 4440
cgtgatattg ctgaagagct tggcggcgaa tgggctgacc gcttcctcgt gctttacggt 4500
atcgccgctc ccgattcgca gcgcatcgcc ttctatcgcc ttcttgacga gttcttctga 4560
gcgggactct ggggttcgat ccccaattcc cgatcgttca aacatttggc aataaagttt 4620
cttaagattg aatcctgttg ccggtcttgc gatgattatc atataatttc tgttgaatta 4680
cgttaagcat gtaataatta acatgtaatg catgacgtta tttatgagat gggtttttat 4740
gattagagtc ccgcaattat acatttaata cgcgatagaa aacaaaatat agcgcgcaaa 4800
ctaggataaa ttatcgcgcg cggtgtcatc tatgttacta gatcggggat cgggccactc 4860
gaccaagcta taacttcgta taatgtatgc tatacgaagt tatcgcgcca aatcgtgaag 4920
tttctcatct aagcccccat ttggacgtga atgtagacac gtcgaaataa agatttccga 4980
attagaataa tttgtttatt gctttcgcct ataaatacga cggatcgtaa tttgtcgttt 5040
tatcaaaatg tactttcatt ttataataac gctgcggaca tctacatttt tgaattgaaa 5100
aaaaattggt aattactctt tctttttctc catattgacc atcatactca ttgctgatcc 5160

```
atgtagattt cccggacatg aagccattta caattgaata tatcctgccg ccgctgccgc   5220
tttgcacccg gtggagcttg catgttggtt tctacgcaga actgagccgg ttaggcagat   5280
aatttccatt gagaactgag ccatgtgcac cttcccccca acacggtgag cgacggggca   5340
acggagtgat ccacatggga ctttt                                         5365
```

## Description

REFERENCE TO RELATED APPLICATIONS

This application claims benefit under 35USC § 119(e) to U.S. Provisional Patent Applications Serial No. 60/467910, filed May 5, 2003 and Serial No. 60/487273, filed July 15, 2003, each of which are incorporated herein by reference in their entirety.

INCORPORATION OF SEQUENCE LISTING

A sequence listing is contained in the file named "38-15(52913)D Sequences.ST25.txt" which is 105 kilobytes (measured in MS-Windows 2000) and was created on May 4, 2004 and is located in computer readable form on a 3.5 inch diskette filed herewith and incorporated herein by reference.

BACKGROUND OF THE INVENTION

Disclosed herein are polynucleotide sequences useful for producing transgenic plants with increased glycine-betaine content and methods of using such sequences for producing transgenic plants and seed. Such sequences are useful for producing transgenic plants with increased tolerance to stresses such as water-deficit and cold.

Stress, such as water-deficit, cold, heat, nutrient deficiency and the like, can have many adverse effects on plant performance such as yield reduction, increased susceptibility to disease and pests, reduced plant growth and reproductive failure. Considering the complexity of stress response in land plants, especially during conditions that produce water-deficit or cold, relatively few genes specifically associated with this aspect of physiology have been identified. It would be of benefit to the art to increase the number and variety of genes involved in regulating water use or temperature tolerance in plants, more particularly, in maize plants, and even more particularly in maize plants experiencing water-deficit and/or cold.

Glycine-betaine (N,N,N-trimethylglycine) is an osmoprotectant metabolite. Osmoprotectant metabolites, including betaines, such as glycine-betaine, sugars, sugaralcohols, and amino acids, such as proline, are known to accumulate in plants under water-deficit and other stressful conditions such as cold conditions. Historically, applications of osmoprotectants to seeds and plants has been shown to have beneficial effects upon stress tolerance. Allard *et al.* (W0 99/01032) found that application of glycine-betaine to wheat plants increased the freezing tolerance of the plants by several degrees and Mottram (U.S. Patent No. 5,952,267) disclose the foliar application of glycine-betaine to cotton plants under water-deficit which resulted in an increased number of cotton bolls.

The pathways for the synthesis of glycine-betaine are similar in higher plants and microorganisms. In both kingdoms, a two-step oxidation of choline occurs to produce glycine-betaine via an unstable glycine-betaine aldehyde intermediate. Choline is ubiquitous in higher plants. In spinach, the first step conversion of choline to glycine-betaine aldehyde utilizes a ferredoxin dependent choline monooxygenase. In *E. coli, a* membrane bound choline dehydrogenase performs this step. The second step, conversion of the unstable aldehyde to glycine-betaine, is carried out by glycine-betaine aldehyde dehydrogenase. This enzyme has been found to share strong similarity between plant and bacterial species.

Spinach, sugar beet and some varieties of maize are examples of higher plants in which glycine-betaine is found to accumulate under water-deficit stress. In contrast, many other plants, such as tomato, tobacco, rice and some varieties of maize, do not accumulate significant amounts of glycine-betaine, regardless of growing conditions.

Hanson *et al.,* (U.S. Patent No. 6,310,271) disclose tobacco transformed with a choline monooxygenase gene which exhibited increased accumulation of glycine-betaine. The transgenic plants also demonstrated increased tolerance to irrigation with saline solution when compared to non-transgenic controls. Bulow *et al.*, (PCT Publication WO 98/26801) disclose the use of an *E. coli* choline dehydrogenase gene to impart increased freezing and choline tolerance in transformed potato plants. Allen *et al.*, (U.S. Application No. 2002/0123118A1) disclose the proposed use of choline oxidase, L-allo-threonine aldolase, phosphoserine phosphatase and sarcosine oxidase genes for altering the levels of glycine metabolism in a transformed cell. Adams *et al.,* (U.S. Patent No. 6,281,411; incorporated herein by reference in its entirety) disclose naturally occurring metabolites, such as glycine-betaine (Wyn-Jones and Storey, 1982) that are osmotically active and/or provide some direct protective effect during drought and/or desiccation.

We have discovered DNA useful for the production of a transgenic plant with increased glycine-betaine. As used herein "GB1" is the name of a protein and its homologs, e.g., a protein at least 40% identical to GB1, the expression of which results in increased glycine-betaine in plants and "gb1" is the name of the DNA coding sequence and its homologs encoding and used to express the GB1 protein. "GB" is used herein to refer to the glycine-betaine metabolite.

## SUMMARY OF THE INVENTION

One aspect of this invention provides novel DNA constructs comprising DNA sequences which express GB1 proteins which, when expressed in a transgenic plant, can increase the glycine-betaine content of a transgenic plant. Certain plants expressing such DNA constructs for enhanced levels of glycine-betaine can exhibit increased tolerance to water-deficit, cold or freezing growing conditions or increased yield. The plants expressing the DNA constructs leading to increased glycine-betaine may be inbred or hybrid, preferably soybean, cotton, canola or maize.

In one aspect, the invention provides transgenic seed and plants having in the genome an exogenous DNA comprising a gb1 coding sequence having the sequence of SEQ ID NO:19 which expresses a GB1 protein having the amino acid sequence of SEQ ID NO:1 where the transgenic plants and seeds accumulate increased glycine-betaine as compared to plants and seed of substantially the same genotype lacking this exogenous DNA. In another aspect of the invention, the transgenic seed and plants accumulating increased glycine-betaine as a result of expressing an exogenous DNA comprising a gb1 coding sequence having the sequence of SEQ ID NO:19 which expresses a GB1 protein of SEQ ID NO:1, exhibit increased tolerance to water-deficit and to cold, and exhibit increased yield under normal growing conditions, water-deficit inducing conditions and cold conditions.

An important aspect of this invention provides transgenic seed and plants having in the genome an exogenous DNA comprising a gb1 coding sequence which expresses a protein having an amino acid sequence comprising at least 25 contiguous amino acids of the consensus amino acid sequence of SEQ ID NO:17 or SEQ ID NO:18. In yet another aspect of the invention such transgenic seed or plants have in the genome an exogenous DNA construct which expresses a GB1 protein having an amino acid sequence which is at least 40% identical SEQ ID NO:1. In another aspect, the invention provides transgenic seed and plants having in the genome an exogenous DNA comprising a gb1 coding sequence which has at least 98% identity to a nucleotide sequence in the group consisting of SEQ ID NOS: 19-34, the sequences of which encode proteins having amino acid sequences of SEQ ID NOS:1-16, which result in increased accumulation of glycine-betaine in transgenic plants. The invention also provides transgenic seed and plants wherein the exogenous DNA comprising a gb1 DNA coding sequence is operably linked to a promoter which functions in plants. Operable promoters include constitutive, water-deficit-inducible, cold inducible, native, viral, tissue specific, or other promoters functional in a plant.

Still another aspect of this invention provides plants grown from such transgenic seed. The seed expressing exogenous DNA comprising gb1 coding sequence and GB1 protein leading to increased glycine-betaine may be inbred or hybrid, preferably soybean, cotton, canola or maize. Additionally, the invention provides for transgenic plants grown from the transgenic seed, for example, maize, cotton or soybean plants.

In another aspect, the invention provides for transgenic plants and seed comprising an exogenous DNA comprising a gb1 coding sequence which exhibit increased tolerance to cold temperatures. In one aspect, the transgenic plants and seed of the invention enable farmers to plant seed earlier and/or under cooler than normal temperatures for the seed type lacking the gb1 transgene, *i.e.,* at a shorter relative maturity zone or a more polar latitude, increased germination under cold conditions, increased tolerance of newly germinated seed or young seedlings to cold, and increased tolerance of mature plants to cold allowing for later harvest and/or improved harvest, *e.g.* increased yield, under cold conditions, *e.g.,* about °C -10°C. In another aspect, the invention provides transgenic plants and seed comprising an exogenous DNA comprising a gb1 coding sequence which exhibit increased germination, emergence and/or seedling survival at about 110 growing degree units (GDU) or less.

Additionally, the invention provides for a transgenic organism, *e.g.* a bacterium or plant, having in its genome an exogenous DNA construct which encodes a GB1 protein or homolog as define herein.

This invention also provides promoters for use in transgenic plants, *e.g.* a maize gb1 promoter and a coix hrgp promoter.

## BRIEF DESCRIPTION OF THE DRAWINGS

Figure 1 is an alignment of proteins of SEQ ID NOS:1-4 and a consensus sequence SEQ ID NO:17.

Figure 2 is an alignment of proteins of SEQ ID NOS:1-16 and a consensus sequence SEQ ID NO:18.

Figure 3 is a plasmid map of pMON78450, the polynucleotide sequence from right border to left border is found in SEQ ID NO:57.

DETAILED DESCRIPTION OF THE INVENTION

**Sequences of the Invention**

The following sequences are disclosed in the description of various aspects of this invention:

SEQ ID NO:1 is an amino acid sequence of a maize protein designated as GB1.
SEQ ID NO:2 is an amino acid sequence of a maize protein designated as maize GB1-2 homolog.
SEQ ID NO:3 is an amino acid sequence of a rice protein designated as rice GB1-1 homolog.
SEQ ID NO:4 is an amino acid sequence of a barley protein designated as barley GB1-1 homolog.
SEQ ID NO:5 is an amino acid sequence of a maize protein designated as maize GB1-3-1 homolog.
SEQ ID NO:6 is an amino acid sequence of a leek protein designated as leek GB1-3-1 homolog.
SEQ ID NO:7 is an amino acid sequence of an *Arabidopsis thaliana* protein designated as At GB1-3-1 homolog.
SEQ ID NO:8 is an amino acid sequence of an *Arabidopsis thaliana* protein designated as At GB1-3-2 homolog.
SEQ ID NO:9 is an amino acid sequence of an *Arabidopsis thaliana* protein designated as At GB1-3-3 homolog.
SEQ ID NO:10 is an amino acid sequence of an *Arabidopsis thaliana* protein designated as At GB1-3-4 homolog.
SEQ ID NO:11 is an amino acid sequence of a *Brassica napus* protein designated as Bn GB1-3-1 homolog.
SEQ ID NO:12 is an amino acid sequence of a soybean protein designated as soy GB1-3-1 homolog.
SEQ ID NO:13 is an amino acid sequence of a soybean protein designated as soy GB1-3-2 homolog.
SEQ ID NO:14 is an amino acid sequence of a barley protein designated as barley GB1-3-1 homolog.
SEQ ID NO:15 is an amino acid sequence of a rice protein designated as rice GB1-3-1 homolog.
SEQ ID NO:16 is an amino acid sequence of a wheat protein designated as wheat GB1-3-1 homolog.
SEQ ID NO:17 is a consensus amino acid sequence comprising amino acid residues of SEQ ID NOS:1-4.
SEQ ID NO:18 is a consensus amino acid sequence comprising amino acid residues of SEQ ID NOS:1-16.
SEQ ID NO:19 is a polynucleotide sequence of a maize gb1 coding sequence encoding the protein of SEQ ID NO:1.
SEQ ID NO:20 is a polynucleotide sequence of a maize gb1-2 homolog coding sequence encoding the protein of SEQ ID NO:2.
SEQ ID NO:21 is a polynucleotide sequence of a rice gb1-1 homolog coding sequence encoding the protein of SEQ ID NO:3.
SEQ ID NO:22 is a polynucleotide sequence of a barley gb1-1 homolog coding sequence encoding the protein of SEQ ID NO:4.
SEQ ID NO:23 is a polynucleotide sequence of a maize gb1-3-1 homolog coding sequence encoding the protein of SEQ ID NO:5.
SEQ ID NO:24 is a polynucleotide sequence of a leek gb1-3-1 homolog coding sequence encoding the protein of SEQ ID NO:6.
SEQ ID NO:25 is a polynucleotide sequence of an *Arabidopsis thaliana* gb1-3-1 homolog coding sequence encoding the protein of SEQ ID NO:7.
SEQ ID NO:26 is a polynucleotide sequence of an *Arabidopsis thaliana* gb1-3-2 homolog coding sequence encoding the protein of SEQ ID NO:8.
SEQ ID NO:27 is a polynucleotide sequence of an *Arabidopsis thaliana* gb1-3-3 homolog coding sequence encoding the protein of SEQ ID NO:9.
SEQ ID NO:28 is a polynucleotide sequence of an *Arabidopsis thaliana* gb1-3-4 homolog coding sequence encoding the protein of SEQ ID NO:10.
SEQ ID NO:29 is a polynucleotide sequence of a *Brassica napus* gb1-3-1 homolog coding sequence encoding the protein of SEQ ID NO:11.
SEQ ID NO:30 is a polynucleotide sequence of a soybean gb1-3-1 homolog coding sequence encoding the protein of SEQ ID NO:12.
SEQ ID NO:31 is a polynucleotide sequence of a soybean gb1-3-2 homolog coding sequence encoding the protein of SEQ ID NO:13.
SEQ ID NO:32 is a polynucleotide sequence of a barley gb1-3-1 homolog coding sequence encoding the protein of SEQ ID NO:14.
SEQ ID NO:33 is a polynucleotide sequence of a rice gb1-3-1 homolog coding sequence encoding the protein of SEQ ID NO:15.
SEQ ID NO:34 is a polynucleotide sequence of a wheat gb1-3-1 homolog coding sequence encoding the protein of SEQ ID NO:16.
SEQ ID NO:35 is a polynucleotide sequence of a rice actin 1 intron promoter.
SEQ ID NO:36 is a polynucleotide sequence of a maize hsp17.5 promoter.
SEQ ID NO:37 is a polynucleotide sequence of a maize hva22 promoter.

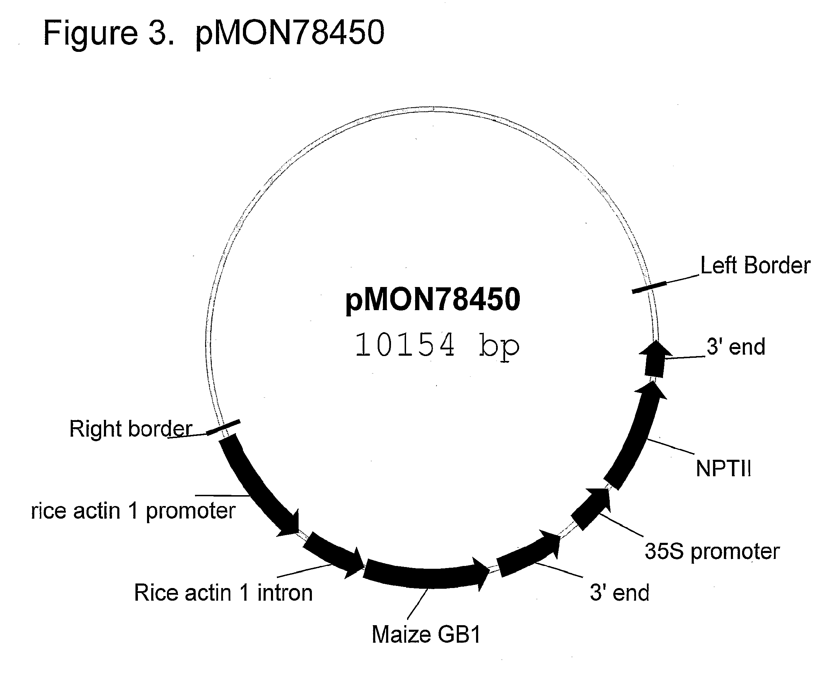

SEQ ID NO:38 is a polynucleotide sequence of a maize ca4h promoter.
SEQ ID NO:39 is a polynucleotide sequence of a maize rab-17 promoter.
SEQ ID NO:40 is a polynucleotide sequence of a maize rab-17 promoter.
SEQ ID NO:41 is a polynucleotide sequence of a rice hsp17.5 promoter.
SEQ ID NO:42 is a polynucleotide sequence of a rice hva22 promoter.
SEQ ID NO:43 is a polynucleotide sequence of a rice ca4h promoter.
SEQ ID NO:44 is a polynucleotide sequence of a rice hsp16.9 promoter.
SEQ ID NO:45 is a polynucleotide sequence of a rice hsp22 promoter.
SEQ ID NO:46 is a polynucleotide sequence of a rice rab-17 promoter.
SEQ ID NO:47 is a polynucleotide sequence of a maize gb1 promoter.
SEQ ID NO:48 is a polynucleotide sequence of a maize cvy-cik1 promoter.
SEQ ID NO:49 is a polynucleotide sequence of a maize cvy-cik1 promoter.
SEQ ID NO:50 is a polynucleotide sequence of a maize cvy-cik1 promoter.
SEQ ID NO:51 is a polynucleotide sequence of a maize cvy-cik1 promoter.
SEQ ID NO:52 is a polynucleotide sequence of a maize cvy-cik1 promoter.
SEQ ID NO:53 is a polynucleotide sequence of a rice cvy-cik1 promoter.
SEQ ID NO:54 is a polynucleotide sequence of a maize rtbv promoter.
SEQ ID NO:55 is a polynucleotide sequence of a maize nas promoter.
SEQ ID NO:56 is a polynucleotide sequence of a coix hrgp promoter.
SEQ ID NO:57 is a polynucleotide sequence from the right border to the left border, inclusive, of pMON78450 shown in Figure 3. Base pairs 1 to 357 are the right border, base pairs 390 to 1232 are the rice actin 1 promoter, base pairs 1310 to 1778 are the rice actin 1 intron, base pairs 1781 to 2698 are the maize GB1 coding sequence of SEQ ID NO:19, base pairs 2767 to 3274 are the 3' untranslated region, base pairs 3409 to 3701 are the 35S promoter, base pairs 3766 to 4560 are the NPTII marker coding sequence, base pairs 4592 to 4844 are the nos 3' untranslated region, and base pairs 4924 to 5365 are the left border.

**Traits of the Invention**

A plant or seed that shows a desired trait, *e.g.*, increased glycine-betaine, or increased tolerance or increased resistance to water-deficit condition, to cold condition, to freezing condition, or a plant with increased yield, is a plant or seed comprising a particular exogenous DNA which imparts a desired, measurable change in the trait in comparison to a control plant, *e.g.*, a plant or seed of substantially the same genotype that lacks that particular exogenous DNA. Preferably, the enhanced desired trait is measured by comparing the trait in a transgenic plant or seed with the particular exogenous DNA associated with the enhanced desired trait to the trait in a control plant or seed. As used herein, a "control plant" or a "control seed" is a plant or seed of substantially the same genotype as the plant or seed it is being compared to, but lacking a particular exogenous DNA construct. A control plant or control seed can be a natural, non-transgenic wild-type plant preferably of the same species as the transgenic plant comprising the particular exogenous DNA. A control plant or control seed can be a second transgenic plant, preferably of the same species as the transgenic plant comprising the particular exogenous DNA, but lacking that same particular exogenous DNA. Preferably, the control plant or control seed lacking the exogenous DNA is a sibling of the plant or seed comprising the particular exogenous DNA, *e.g.* a negative segregant. Such a sibling control plant or control seed may comprise other exogenous DNA.

This invention provides for a transgenic maize plant exhibiting increased glycine-betaine content. The transgenic maize plant comprises an exogenous DNA comprising a gb1 coding sequence (SEQ ID NOS:19-34) expressing a GB1 protein (SEQ ID NOS:1-16) which exhibits at least about a 2-fold, about a 5-fold, about a 10-fold, about a 20-fold, about a 50-fold or even about a 70-fold or greater increase in glycine-betaine content as compared to a non-transgenic maize plant. Increased tolerance or resistance to water-deficit or cold or freezing may be exhibited by the plant accumulating at least a 2-fold increase in glycine-betaine and may be measured in a variety of ways including increased plant height, leaf length, leaf extension rate, number of leaves, root length, root mass, shoot mass, seed set, number of seed, yield, photosynthesis, turgor pressure, osmotic potential, amount of pollen, silking, germination, chlorophyll fluorescence, necrosis, and the like.

As used herein "stress response" is a plant or seed condition occurring in response to external influences capable of affecting the physical or biochemical characteristics of a plant or seed. These external influences are "stress." Stresses include, but are not limited to, all biotic and abiotic stresses that could influence a plant or seed, from infection to environment. For example, cold, heat, water-deficit, salinity, chemicals, weather conditions, fungal or bacterial infection, insect infestation, soil nutrient deficiencies or excesses, soil compaction or density, light, shade, or soil pH, or any combination of these conditions, are types of stresses a plant or seed may experience and respond to. Those physical or biochemical characteristics of a plant or seed that may be influenced by stress include, for example, yield, height, color, vigor, root growth, shoot growth, flowering times and qualities, seed quality, pollen quality, reproductive potential, germination or development, or any combination of these or other plant characteristics.

As used herein "water-deficit" is a plant condition characterized by water potential in a plant tissue of less than about -0.5 megapascals (MPa), e.g. -0.6 MPa. Water potential in maize is conveniently measured by clamping a leaf segment in a pressurizable container so that a cut cross section of leaf is open to atmospheric pressure. Gauge pressure (above atmospheric pressure) on the contained leaf section is increased until water begins to exude from the atmospheric-pressure-exposed cross section; the gauge pressure at incipient water exudation is reported as negative water potential in the plant tissue, e.g. 0.5 MPa gauge pressure is reported as -0.5 MPa water potential. A water-deficit may be induced in plant or seed by a number of manners, including growing in a geographical location in which rainfall is usually limiting, or growing in a growth chamber or greenhouse where water is provided or withheld in a monitored manner. In addition, water-deficit condition may be brought about in a plant or seed by exposure to solutions that may cause or mimic water-deficit such as saline solutions, PEG solutions and the like. A transgenic seed or plant is said to have improved water-deficit tolerance if it is able to germinate, germinate more quickly, grow, mature, and/or reproduce under water-deficit conditions as compared to a seed or plant of substantially the same genotype but lacking that exogenous DNA construct. A seed or plant with improved water-deficit tolerance would enable farmers to plant and grow crops in less than ideal water conditions, for example, in a drier location or in a location exposed with higher saline levels than normal in the soil and/or water used for irrigation, thus expanding the locations or conditions in which the plant or seed may be grown.

As used herein "non-water-deficit" conditions describe plant conditions characterized by water potential in a plant tissue of greater than about -0.5 megapascals (MPa), e.g. -0.4 MPa and may be measured as previously described. Non-water-deficit conditions may be induced in a plant by a number of manners, including growing plants in a geographical location in which rainfall is usually not limiting, growing plants in a geographical location in which rainfall is usually limiting and providing water by irrigation methods, or growing in a growth chamber or greenhouse where water is provided in a monitored manner.

As used herein "increased yield" identifies a measurable increase in the amount of useable product from a first plant, *e.g.*, a plant comprising a particular exogenous DNA, compared to a second plant, *e.g.* a non-transgenic control plant or other control plant lacking a particular exogenous DNA, when the plants are grown under substantially identical conditions. Yield is based upon the weight of the grain produced from all the plants of a given line grown in a given plot and is measured in bushels per acre. Yield is typically measured in field trials using methods known to those of skill in the art.

As used herein, "cold tolerance" is defined as the ability of a seed, seedling, young plant, or mature plant, or parts thereof, to germinate and/or continue growth for a significant period of time after being placed at or exposed to a temperature below that normally encountered by a plant of that species at that growth stage. This invention provides a transgenic maize plant and seed with increased glycine-betaine comprising an exogenous DNA construct comprising a gb1 coding sequence (SEQ ID NOS:19-34) expressing a GB1 protein (SEQ ID NOS:1-16) that exhibits increased cold tolerance relative to a control plant or control seed.

"Germination" is defined as the beginning of growth or development in a seed, especially after a period of dormancy. Germination is often considered to begin when the seed takes up water (imbibes) and is considered to be essentially complete when the embryonic axis beings to elongate. As used herein, "cold germination" is germination occurring at temperatures below (two or more degrees Celsius below) those normal for a particular species or particular strain of plant. A transgenic seed is said to have improved cold germination if it is able to germinate more quickly in the cold temperature and/or if a greater percentage of the seed germinate in the cold temperature in a given amount of time as compared to a control seed or control plant. The temperature may be about 1°C colder than normal, about 2°C colder than normal, about 4°C, 6°C, 8°C or even about 10°C or more colder than normal.

A convenient way to measure cold stress conditions is to measure the accumulation of growing degree units (GDU) over time from the planting date. It is well known to one skilled in the art that approximately 120 GDUs are required for commercial maize hybrids to germinate and emerge from the soil. GDUs, which reflect the warming of the air, are measured on a daily basis in a cumulative manner using the following calculation:

$$\text{GDU} = \frac{(\text{Daily Max Temp}^{*} + \text{Daily Min Temp}^{**})}{2} - 50$$

where * is the daily maximum temperature up to 86°F; if the temperature exceeds 86°F then the value of 86°F is used and where ** is the daily minimum temperature down to 50°F; if the temp is lower than 50°F then the value of 50°F is used.

Under cold conditions, therefore, it takes more days to reach a given number of GDUs and, conversely, under warm conditions it takes fewer days to reach that same number of GDUs. For example, the United States National Weather Service daily high and low normal temperatures for the last 30 years indicate that for Spencer, Iowa, (latitude 42.97, Longitude 90.10, a central location within the US maize growing territories) 20 days are required to accumulate 120 GDUs if planting occurs on April 15th whereas 11 days are required if planting occurs on May 15th. Typically, it takes about 12 to about 15 days to accumulate the about 120 to 140 GDU required for maize to germinate in early spring conditions although one skilled in the art would know that this may vary slightly with respect to some variables such as planting depth and date of planting.

If it takes more than about 16, *e.g.*, about 18, or 20 or even about 24 days, to accumulate about 120 to 140 GDUs, then a cold stress is imposed on a plant or seed. A transgenic seed having in its genome an exogenous DNA comprising a gb1 coding sequence, the expression of which results in increased glycine-betaine, will demonstrate improved germination and growth as compared to a control seed or control plant when about 16, or more, *e.g.* 18,20 or 24 days are required to accumulate about 120 to 140 GDUs.

A transgenic maize seed having in its genome an exogenous DNA comprising a gb1 coding sequence resulting in increased glycine-betaine shows increased tolerance to cold conditions as compared to control plant or control seed. The transgenic maize seed germinates more quickly, emerges from the soil more rapidly and/or with more kernels germinated, and exhibits better seedling survival, in about 110 GDU, or less, *e.g.*, 100 GDU or 90 GDU, than a control seed or control plant. It is known to one skilled in the art that hot and dry conditions during the reproductive phase damage the female organs and tissues, thereby reducing the harvested yield of commercial maize. The hot and dry conditions typically begin in early July within the US maize growing territories. A transgenic maize seed that emerges from the soil more quickly and/or with more kernels germinated and exhibits better seedling survival, in about 110 GDU or less, will reach reproductive developmental stages earlier in the growing season, thus avoiding damage during hot and dry conditions and thereby enabling farmers to effectively increase the harvested yield of maize in bushel/acre.

A seed or plant may be exposed to cold conditions at many points in time and thus it is desirable to have cold tolerance at many stages of development. For example, for a seed, cold germination is a form of cold tolerance that may be exhibited during germination at temperatures below the normal germination temperature for that seed. Cold tolerance may benefit a newly germinated seed as it may experience cold temperature after the embryonic axis begins to elongate. A young plant may benefit from cold tolerance as it may experience cold temperature as new leaves are developing above the ground. A more mature plant may benefit from cold tolerance as it may experience cold temperature during the periods of fertilization, seed set, grain fill and other reproductive activities. "Freezing tolerance" is defined as the ability of a seed, seedling, young plant, or mature plant, or parts thereof, to continue growth for a significant period of time after being placed at a temperature about freezing (e.g., about 32°F) or below.

For a crop such as maize, a normal field planting is carried out when the temperature in the top two inches of soil is at least 10-12°C during the day, therefore a transgenic seed that germinates more quickly and/or to a greater percentage at about 12°C, about 10°C, 8°C, 6°C, 4°C, or about 2°C or even about 1°C as compared to a seed or plant of substantially the same genotype but lacking that exogenous DNA construct, is considered to have improved or enhanced cold germination. A transgenic seed of the invention with enhanced cold tolerance, especially improved cold germination, would enable farmers to plant and grow crops at an earlier time in the season, in a cooler location than normal, at both an earlier time in the season and at a cooler location than normal, or allow for a later harvest, thus expanding the times and/or locations in which the plant may be grown as compared to control plants or control seed.

In a field, the cold temperatures may be imposed upon seeds and plants by planting at an earlier time than is normal for a particular location and/or planting at a geographical location that is typically colder than the geographical location in which the seed is normally planted, *e.g.*, a shorter relative maturity (RM) zone. Relative maturity is a universal term of the art describing the time required for a given maize genotype to reach maturity. RM is determined during the development of a maize hybrid line by constantly assessing how many days the genotype takes to reach maturity in different environments. Most commercial hybrids fall into RM zones which range from 85 (in the more Northern areas of the US maize growing territories) to 125 (in the more Southern areas of the US maize growing territories). In other parts of the world growing maize, commercial hybrids typically have RMs of about 75-120 in Europe, about 108-138 in Africa, about 105-135 in Argentina, about 118-140 in Brazil, about 115-138 in Mexico and about 80-145 in Asia. Those skilled in the art know that maize varieties adapted to longer RM zones (100-120 or more) produce greater yield than those at shorter RM zones (85-100 or less); enabling farmers to grow a higher RM variety in a shorter RM zone would effectively increase the harvested yield of maize in bushel/acre worldwide. A transgenic seed or plant comprising an exogenous DNA comprising a gb1 DNA coding sequence of SEQ ID NOS:19-34 expressing proteins of SEQ ID NOS: 1-16 exhibiting increased glycine-betaine and increased cold tolerance, would enable farmers to plant and grow crops in a shorter RM zone as compared to control seed or control plants.

**Recombinant DNA Constructs**

The present invention contemplates the use of polynucleotides which encode a protein effective for imparting increased tolerance to water-deficit or cold in plants, increased glycine-betaine, and/or increased yield. Such polynucleotides are assembled in recombinant DNA constructs using methods known to those of ordinary skill in the art. A useful technology for building DNA constructs and vectors for transformation is the GATEWAY™ cloning technology (available from Invitrogen Life Technologies, Carlsbad, California) which uses the site-specific recombinase LR cloning reaction of the Integrase/*att* system from bacteriophage lambda for vector construction instead of restriction endonucleases and ligases. The LR cloning reaction is disclosed in U.S. Patents 5,888,732 and 6,277,608, U.S. Patent Application Publications 2001283529, 2001282319 and 20020007051, all of which are incorporated herein by reference. The GATEWAY™ Cloning Technology Instruction Manual which is supplied by Invitrogen also provides concise directions for routine cloning of any desired RNA into a vector comprising operable plant expression elements.

As used herein "exogenous DNA" refers to DNA which is not normally found next to the adjacent DNA, i.e., a sequence not normally found in the host genome in an identical context, or any two sequences adjacent to each other which are not normally or naturally adjacent to each other. Exogenous DNA may include a DNA or RNA sequence native to the host genome or may comprise the native sequence altered by the addition or deletion of one or more different regulatory elements or other sequences as discussed below. The exogenous DNA may encode a protein or non-protein product. A DNA construct comprising a coding sequence of interest, which originates or is produced outside of an organism, is also an example of an exogenous DNA.

Exogenous DNA constructs used for transforming plant cells will comprise the coding sequence of interest and usually other elements as discussed below such as, but not limited to introns, 5' and 3' untranslated regions, and enhancers. An exogenous DNA of the present invention is exemplified by a rice actin 1 promoter and intron operably linked to a gb1 coding sequence operably linked to a 3' untranslated region. As used herein "transgene" means an exogenous DNA which has been incorporated into a host genome or is capable of autonomous replication in a host cell and is capable of causing the expression of one or more cellular products. Exemplary transgenes will provide the host cell, or plants regenerated therefrom, with a novel phenotype relative to the corresponding non-transformed cell or plant. Transgenes may be directly introduced into a plant by genetic transformation, or may be inherited from a plant of any previous generation which was transformed with the exogenous DNA.

As used herein "coding sequence" means a DNA sequence from which an RNA molecule is transcribed. The RNA may be an mRNA which encodes a protein product, an RNA which functions as an anti-sense molecule, or a structural RNA molecule such as a tRNA, rRNA, or snRNA, or other RNA. As used herein "expression" refers to the combination of intracellular processes, including transcription and translation, undergone by a DNA molecule to produce a protein or an RNA molecule. As used herein, a "gene" is a hereditary unit of DNA which comprises at least coding sequence; optionally included are other sequences such as introns, promoters, untranslated regions and other signal sequences.

As used herein "promoter" means a region of DNA sequence that is essential for the initiation of transcription of RNA from DNA; this region may also be referred to as a "5' regulatory region." Promoters are located upstream of DNA to be translated and have regions that act as binding sites for RNA polymerase and have regions that work with other factors to promote RNA transcription. More specifically, basal promoters in plants comprise canonical regions associated with the initiation of transcription, such as CAAT and TATA boxes. The TATA box element is usually located approximately 20 to 35 nucleotides upstream of the site of initiation of transcription. The CAAT box element is usually located approximately 40 to 200 nucleotides upstream of the start site of transcription. The location of these basal promoter elements result in the synthesis of an RNA transcript comprising some number of nucleotides upstream of the translational ATG start site. The region of RNA upstream of the ATG is commonly referred to as a 5' untranslated region or 5' UTR. It is possible to use standard molecular biology techniques to make combinations of basal promoters, that is regions comprising sequences from the CAAT box to the translational start site, with other upstream promoter elements to enhance or otherwise alter promoter activity or specificity.

As is well known in the art, DNA constructs for use in transforming plants and expressing a coding sequence typically also comprise other regulatory elements in addition to a promoter, such as but not limited to 3' untranslated regions (such as polyadenylation sites), transit or signal peptides and marker coding sequences elements. For instance, see U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patents 5,322,938, 5,352,605, 5,359,142 and 5,530,196 which disclose a 35S promoter, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. patent application Serial No. 09/078,972 which discloses a coixin promoter, and U.S. patent application Serial No. 09/757,089 which discloses a maize chloroplast aldolase promoter, all of which are incorporated herein by reference. One skilled in the art would know that various introns, enhancers, transit peptides, targeting signal sequences, 5' and 3' untranslated regions (UTRs) useful in the design of effective plant expression vectors, such as those disclosed, for example, in U.S. Patent Application Publication 2003/01403641 (incorporated herein by reference), may be used in the promoter and coding sequence combination clones, such as, for example, those described in Table 2, to obtain and optimize expression of the gb1 coding sequence (SEQ ID NO:19) and homologs (SEQ ID NOS:20-34) of the invention.

In some aspects of the invention it is preferred that the promoter element in the exogenous DNA construct should be capable of causing sufficient expression of SEQ ID NOS:19-34 to result in the production of an effective amount of the proteins of SEQ ID NOS: 1-16 only under water-deficit conditions, cold conditions or other stress situations. By avoiding continuous high-level expression of transgenes, any undesired effects caused by continual over-expression of transgenes, or ectopic expression in various tissues or at various times, can be minimized or eliminated. Such promoters can be identified and isolated from the regulatory region of plant genes which are up-regulated in water-deficit conditions, cold or other stress conditions.

Specific water-deficit-inducible promoters for the expression of a maize gb1 coding sequence (SEQ ID NO:19) and homologs of a maize gb1 coding sequence (SEQ ID NOS:20-34) useful in the practice of this invention are derived from the 5' regulatory region of genes identified as a heat shock protein 17.5 gene (hsp17.5; SEQ ID NO: 36), an HVA22 gene (hva22; SEQ ID NO:37), and a cinnamic acid 4-hydroxylase gene (ca4h; SEQ ID NO:38) of *Zea mays.* Such water-deficit-inducible promoters are disclosed in U.S. Application Serial No. 10/739,565, incorporated herein by reference. Additional specific water-deficit-inducible promoters useful in the practice of this invention are derived from the 5' regulatory region of genes identified as a heat shock protein 17.5 gene (hsp17.5; SEQ ID NO:41), an HVA22 gene (hva22; SEQ ID NO:42), a cinnamic acid 4-hydroxylase gene (ca4h; SEQ ID NO:43), an HSP16.9 gene (hsp16.9; SEQ ID NO: 44), an HSP22 gene (hsp22; SEQ ID NO:45), and a rab-17 promoter (SEQ ID NO:47) of rice. Such water-deficit-inducible promoters are disclosed in U.S. provisional application Serial No. 60/547761, incorporated herein by reference. Additionally preferred water-deficit inducible promoters contemplated to be particularly useful in the practice of this invention include the rab-17 promoter reported by Vilardell et al., (Plant Molecular Biology, 17(5):985-993, 1990; SEQ ID NO:39) as well as a second, independently isolated rab-17 promoter (SEQ ID NO:40; disclosed in U.S. Application Serial No. 10/739,565).

It is also contemplated that a cold inducible promoter is a useful promoter for the expression of a maize gb1 coding sequence (SEQ ID NO:19) and homologs of a maize gb1 coding sequence (SEQ ID NOS:20-34). Cold inducible promoters have been isolated from a variety of plants and useful promoters include, for example, a wcs 120 promoter from wheat (Oullet, F. et al., FEBS Letters. 423 : 324-328, 1998), a ci7 promoter from potato (Kirch, H. et al., Plant Mol Biol,. Mar;33(5):897-909, 1997), an hva22 coding sequence from barley (Shen, Q., et al., Plant Mol Biol., Feb;45(3): 327-40, 2001), a cor15 promoter from *Arabidopsis* (Baker, S. et al., Plant Mol Biol. Mar;24(5):701-13, 1994), a kin1 or cor6.6 cold inducible promoter also from *Arabidopsis* (Wang H., et al., Plant Mol Biol. Jul;28(4):605-17, 1995) or the cold inducible promoters described in U.S. Patent 6,084,089. A preferred cold inducible promoter is the maize cvy-cik1 promoter (SEQ ID NOS:48-52) or its rice homolog (SEQ ID NO:53). The cvy-cik1 promoter is induced in transgenic maize plants following cold treatment and is disclosed in U.S. provisional application Serial No. 60/463,974, incorporated herein by reference in its entirety.

A useful promoter for expression a maize gb1 coding sequence is a promoter isolated from a maize gb1 gene (SEQ ID NO:47).

Tissue-specific promoters are also contemplated to be useful promoters for driving the expression of maize gb1 coding sequences and homologous sequences (SEQ ID NOS:19-34). Such promoters include, but are not limited to, a phloem specific rice tungro bacilliform virus promoter (RTBV; SEQ ID NO:54 and U.S. Patent No. 5,824,857), a maize root specific nicotianamine synthase promoter (SEQ ID NO:55), or a silk specific hydroxyproline rich glycoprotein promoter (hrgp; SEQ ID NOS:56).

During transformation, exogenous DNA may be introduced randomly, i. e. at a nonspecific location, in the plant genome. In some cases, it may be useful to target an exogenous DNA insertion in order to achieve site-specific integration, *e.g.* to replace an existing gene sequence or region in the genome. In some other cases it may be useful to target an exogenous DNA integration into the genome at a predetermined site from which it is known that gene expression occurs. Several site-specific recombination systems exist which are known to function in plants include Cre/lox as disclosed in U.S. Patent 4,959,317 and FLP/FRT as disclosed in U.S. Patent 5,527,695, both incorporated herein by reference.

Constructs and vectors may also include a transit peptide for targeting of a protein or RNA product to a plant organelle, particularly to a chloroplast, leucoplast or other plastid organelle. For a description of the use of a chloroplast transit peptide see U.S. Patent 5,188,642, incorporated herein by reference.

In practice DNA is introduced into only a small percentage of target cells in any one experiment. Marker genes are used to provide an efficient system for identification of those cells that are stably transformed by receiving and integrating an exogenous DNA construct into their genomes. Preferred marker genes provide selective markers which confer resistance to a selective agent, such as an antibiotic or herbicide. Potentially transformed cells are exposed to the selective agent. In the population of surviving cells will be those cells where, generally, the resistance-conferring coding sequence has been integrated and expressed at sufficient levels to permit cell survival. Cells may be tested further to confirm stable integration of the exogenous DNA. Useful selective marker genes include those conferring resistance to antibiotics such as kanamycin (*nptII*), hygromycin B (*aph IV*) and gentamycin (*aac3* and *aac*C4) or resistance to herbicides such as glufosinate (*bar* or *pat*) and glyphosate (EPSPS; CP4). Examples of such selectable markers are illustrated in U.S. Patents 5,550,318; 5,633,435; 5,780,708 and 6,118,047, all of which are incorporated herein by reference. Screenable markers which provide an ability to visually identify transformants can also be employed, *e.g.*, a gene expressing a colored or fluorescent protein such as a luciferase or green fluorescent protein (GFP) or a gene expressing a *beta*-glucuronidase or *uid*A gene (GUS) for which various chromogenic substrates are known.

**Protein Molecules**

Proteins of the present invention which represent whole proteins or at least a sufficient portion of the entire protein to impart the relevant biological activity of the protein, *e.g.* increased glycine-betaine content in a transgenic organism. The term "protein" also includes molecules consisting of one or more polypeptide chains. Thus, a protein useful in the present invention may constitute an entire gene product or one or more functional portions of a natural protein which provides the agronomic trait of this invention, i.e. increased glycine-betaine, increased yield despite exposure to water-deficit, increased yield despite exposure to cold, increased yield under non-water-deficit conditions or increased yield under normal growing temperatures.

Homologs of the proteins of the present invention may be identified by comparison of the amino acid sequence of the GB1 protein of SEQ ID NO:1 to amino acid sequences of proteins from the same or different plant sources, *e.g.* manually or by using known homology-based search algorithms such as those commonly known and referred to as BLAST, FASTA, and Smith-Waterman.

A further aspect of the invention provides coding sequences which encode functional homologous proteins which differ in one or more amino acids from those of a GB1 protein provided herein as the result of one or more of the well-known conservative amino acid substitutions, *e.g.* valine is a conservative substitute for alanine and threonine is a conservative substitute for serine. When such a homologous protein is expressed in a transgenic plant, the homologous protein will affect the transgenic plant in a substantially equivalent manner as the GB1 protein.

Conservative substitutions for an amino acid within the native protein sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine-valine, aspartic acid-glutamic acid, and asparagine-glutamine.

A further aspect of the invention comprises proteins which differ in one or more amino acids from those of a described GB1 protein sequence as the result of deletion or insertion of one or more amino acids in a native sequence. When such a homologous protein is expressed in a transgenic plant, the homologous protein will affect the transgenic plant in a substantially equivalent manner as the GB1 protein, *e.g.*, result in increased glycine-betaine content.

Proteins of the present invention that are variants of the proteins provided herein will generally demonstrate significant identity with the proteins provided herein. Of particular interest are proteins having at least 50% sequence identity, more preferably at least about 70% sequence identity or higher, *e.g.* at least about 80% sequence identity with a consensus amino acid sequence of SEQ ID NO:17 or SEQ ID NO:18. Of course useful proteins also include those with higher identity to a consensus sequence, *e.g.* 90%, to 100% identity. Other proteins of interest have at least 50% or more, *e.g.* at least 60% or 70% of homology with the proteins as defined by SEQ ID NO:1 through SEQ ID NO:16. Of course useful proteins also include those with higher percentage homology with the amino acids in a protein segment of SEQ ID NO:1 through SEQ ID NO:16, *e.g.*, 80%, 90%, 95%, 98% or up to 100% homology.

**Transformation Methods and Transgenic Plants**

Methods and compositions for transforming plants by introducing an exogenous DNA into a plant genome in the practice of this invention can include any of the well-known and demonstrated methods. Preferred methods of plant transformation are microprojectile bombardment as illustrated in U.S. Patents 5,015,580; 5,550,318; 5,538,880; 6,160,208; 6,399,861 and 6,403,865 and *Agrobacterium*-mediated transformation as illustrated in U.S. Patents 5,635,055; 5,824,877; 5,591,616; 5,981,840 and 6,384,301, all of which are incorporated herein by reference.

Transformation methods of this invention to provide plants with increased water-deficit, cold or other stress tolerance are preferably practiced in tissue culture on media and in a controlled environment. "Media" refers to the numerous liquid, solid, or semi-solid nutrient mixtures that are used to grow cells *in vitro,* that is, outside of the intact living organism. Recipient cell targets include, but are not limited to, meristem cells, callus, immature embryos and gametic cells such as microspores, pollen, sperm and egg cells. "Propagation" or "propagating" as used herein means the process of multiplying or breeding plant material. Therefore, propagation may involve maintaining a viable tissue on a media, e.g. a callus tissue on a solid medium, or growing a plant from seed or tissue, such as callus and cuttings.

As used herein "regeneration" means the process of growing a plant from a plant cell (*e.g.*, plant protoplast, callus or explant). It is contemplated that any cell from which a fertile plant may be regenerated is useful as a recipient cell. Callus may be initiated from tissue sources including, but not limited to, immature embryos, seedling apical meristems, microspores and the like. Those cells which are capable of proliferating as callus also are recipient cells for genetic transformation. Practical transformation methods and materials for making transgenic plants of this invention, *e.g.* various media and recipient target cells, transformation of immature embryos and subsequent regeneration of fertile transgenic plants are disclosed in U.S. Patent 6,194,636 and U.S. patent application Serial No. 09/757,089, both of which are incorporated herein by reference.

As used herein a "transgenic" organism is one whose genome has been altered by the incorporation of foreign genetic material or additional copies of native genetic material, e.g. by transformation or recombination. The transgenic organism may be a plant, mammal, fungus, bacterium or virus. As used herein "transgenic plant" means a plant or progeny plant of any subsequent generation derived therefrom, wherein the DNA of the plant or progeny thereof contains an introduced exogenous DNA not originally present in a non-transgenic plant of the same strain. The transgenic plant may additionally contain sequences which are native to the plant being transformed, but wherein the exogenous DNA has been altered in order to alter the level or pattern of expression of the coding sequence.

As used herein an "$R_o$ transgenic plant" is a plant which has been directly transformed with an exogenous DNA or has been regenerated from a cell or cell cluster which has been transformed with an exogenous DNA. As used herein "progeny" means any subsequent generation, including the seeds and plants therefrom, which is derived from a particular parental plant or set of parental plants; the resultant progeny line may be inbred or hybrid. Progeny of a transgenic plant of this invention can be, for example, self-crossed, crossed to a transgenic plant, crossed to a non-transgenic plant, and/or back crossed. Thus, a transgenic maize plant prepared according to the invention may be an Ro plant, and progeny plants may be inbred or hybrid maize plants and may be heterozygous or homozygous for the exogenous DNA insertion. As used herein "crop plants" of interest include, but are not limited to soy, cotton, canola, wheat, sunflower, sorghum, alfalfa, barley, millet, rice, tobacco, fruit and vegetable crops, and turf grass. A preferred crop plant is *Zea mays,* commonly known as maize or corn.

The seeds of this invention are harvested from fertile transgenic plants and used to grow progeny generations of plants of this invention including a hybrid plant line comprising the exogenous DNA encoding proteins of SEQ ID NOS: 1-16 which provides the benefits of increased resistance and/or tolerance to stresses such as, but not limited to, water-deficit or cold and increase yield. The seeds of the invention also comprise increased glycine-betaine content as compared to a non-transgenic seed.

## EXAMPLES

Having now generally described the invention, the same will be more readily understood through reference to the following examples which are provided by way of illustration, and are not intended to be limiting of the present invention, unless specified.

## EXAMPLE 1

*Identification of a gb1 gene from Zea mays*

Plants from a number of non-transgenic inbred lines of *Zea mays* were field-grown under water-deficit (non-irrigated) or non-water-deficit (irrigated) conditions. Leaf samples were taken from plants before the tassel stage for each condition, and RNA and metabolites were isolated. RNA from the water-deficit and non-water-deficit samples was analyzed for differences using transcriptional profiling array methods. A number of RNAs were found to show differences in accumulation, to either higher or lower levels in the plants, depending upon the water treatment.

In addition to RNA transcription profiling, the glycine-betaine (GB) content was determined in leaf tissue samples from the inbred lines grown under water-deficit and non-water-deficit conditions. The characterized inbred maize lines were grouped into two categories: "GB accumulators," comprising greater than about 0.05 mM GB, and "GB non-accumulators," comprising less than about 0.05 mM GB.

One particular transcriptional profiling array element demonstrated an increase in RNA accumulation under water-deficit conditions compared to non-water-deficit conditions. In addition, under water-deficit conditions, plants in the study designated as "GB accumulators" were shown to have 3 to 12-fold higher levels of RNA transcript of this array element when compared to "GB non-accumulator" maize plants. These correlations were significant at the $p<0.005$ level across more than 85 commercial inbred lines of *Zea mays.* This array element was designated as the GB1 array element.

The GB1 array element was used as a probe in a Northern blot analysis using RNA samples from GB accumulator maize plants which were grown under both water-deficit and non-water-deficit conditions. The Northern blot analysis showed that the GB1 array element probe hybridized to a single RNA species which accumulated to much higher levels in water-deficit plants as compared to non-water-deficit plants. In contrast, when the GB1 array element was used as a probe against RNA samples from water-deficit and non-water-deficit plant tissues from GB non-accumulator lines, no hybridization was observed.

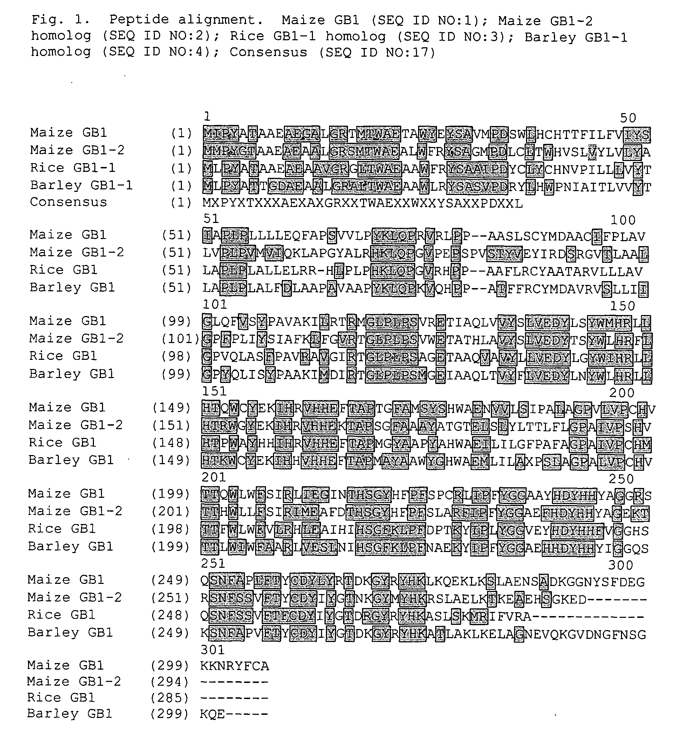

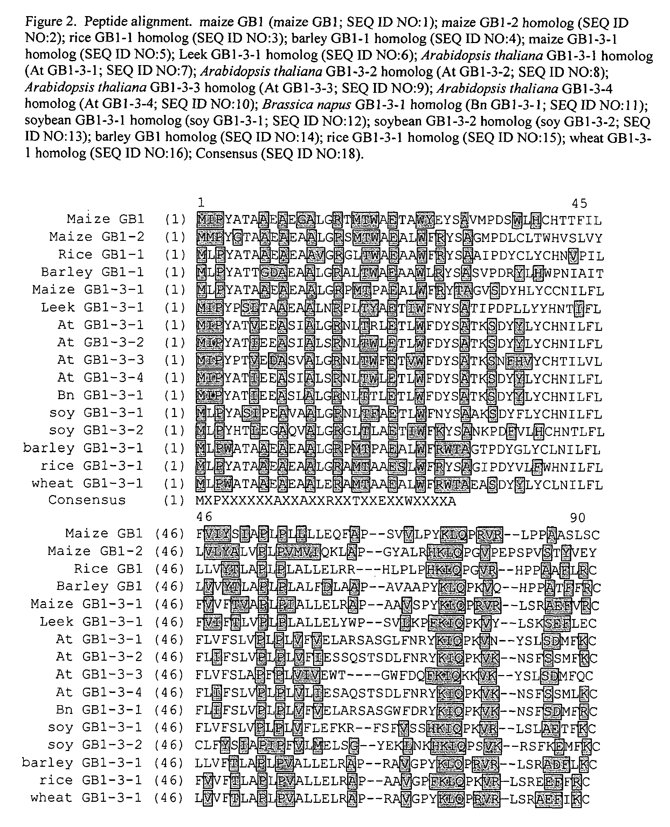

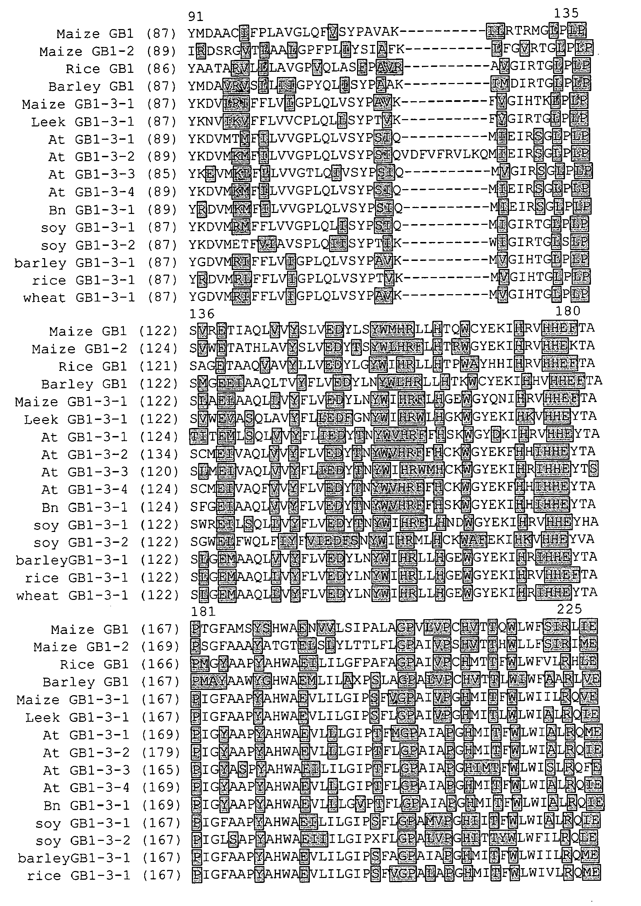

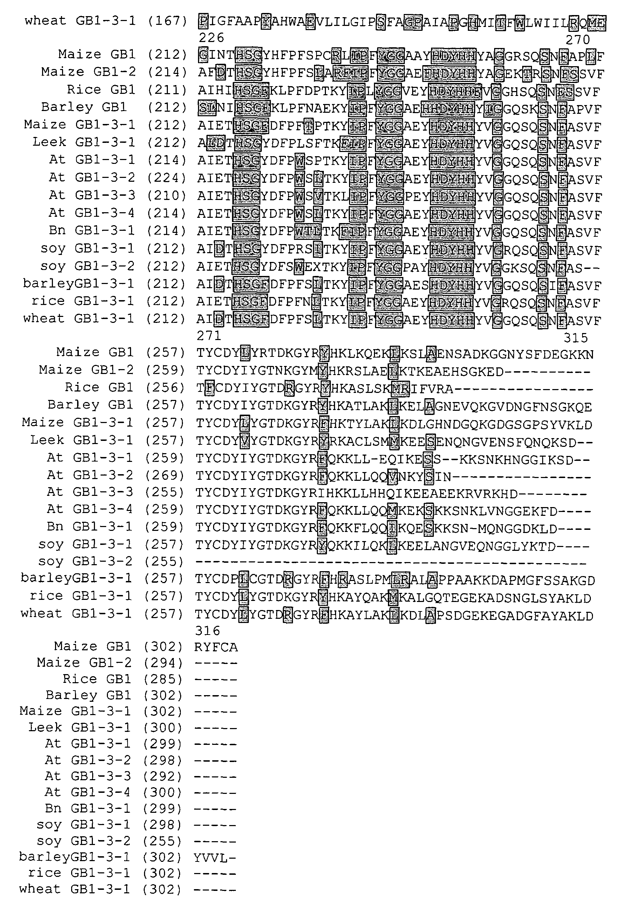

Sequence of the GB1 array element was used to identify a full-length sequence, designated the gb1 DNA, in a proprietary database of maize DNA sequences. Translation of the full-length gb1 DNA sequence (SEQ ID NO:19) indicated that the GB1 peptide sequence (SEQ ID NO:1) shares limited homology with particular histidine domains found in a sterol-4$\alpha$-methyl oxidase cDNA from *Arabidopsis thaliana* (Darnet *et al.,* 2001) and a C-4 methyl sterol oxidase from *Saccharomyces cerevisiae* (Bard *et al.,* 1996). In these systems, however, these enzymes are thought to be involved in sterol metabolism and no role has been identified for the participation of these enzymes in the synthesis of glycine-betaine. Rafalski and Famodu (U.S. Patent No. 6,479,733) propose the use of C-4 methyl sterol oxidase in the manipulation of sterol metabolism in a plant; the sequence of the present invention and that of Rafalski and Famodu are only distantly related at the polynucleotide and amino acid levels. Additionally, Lalgudi *et al.,* (U.S. Patent Application Publication No. 2001/0051335 A1) disclose short DNA and protein fragments identified only as "corn tassel-derived polynucleotides (cdps) which encode corn tassel-derived proteins (CDPs)" which show sequence similarity to the GB1 sequence identified by the current inventors. Lalgudi *et al.,* do not disclose a function for the cdps and CDPs in the synthesis of glycine-betaine, for water-deficit or cold tolerance, nor for increased yield. Alignments of proteins exhibiting homology to the maize GB1 protein of the current invention as well as alignments describing consensus regions are shown in Figure 1 and Figure 2 which are used to identify consensus amino acid sequences of SEQ ID NO:17 and SEQ ID NO:18, respectively.

EXAMPLE 2

*Over-expression of exogenous DNA constructs comprising gb1 coding sequence in transgenic Zea mays*

Transgenic *Zea mays* of a GB non-accumulator line was prepared with an exogenous DNA comprising a constitutive promoter region comprising a rice actin 1 promoter and a rice actin 1 intron operably linked to the gb1 coding sequence of SEQ ID NO:19 encoding the GB1 polynucleotide of SEQ ID NO:1 (see pMON78450 in Figure 3 and SEQ ID NO:57). $R_o$ transgenic plants comprising low copy number events (that is, about 1-2 copies based upon molecular analysis) were selected for study. Transgenic non-accumulator plants comprising the gb1 exogenous DNA and non-transgenic control plants of both a GB non-accumulator line and a GB accumulator line were grown under greenhouse conditions and leaf samples taken at approximately the V6-V8 stage. Leaf samples from a total of 45 different transgenic events were examined for GB accumulation. Leaf samples were lyophilized, ground to a fine powder and metabolites extracted into an ethanol-based extraction buffer supplemented with deuterated glycine-betaine as an internal standard metabolite. Samples were analyzed by liquid chromatography-mass spectrometry/ mass spectrometry and the amount of glycine-betaine (in mM) determined by analyzing the ratio of the deuterated and non-deuterated glycine-betaine in a sample.

Glycine-betaine was found to accumulate to significantly higher levels in the gb1 transgenic plants when compared to both the GB non-accumulator (LH59) and GB accumulator (FBLL) non-transgenic plants. On average, in the V6-V8 plants, the gb1 transgenic plants contained approximately 7.2mM GB per sample as compared to 3.0mM and 0.1mM in the non-transgenic GB accumulator FBLL and GB non-accumulator LH244 lines, respectively. This represents an approximately 70-fold increase in GB in the transgenic plants compared to the non-transgenic GB non-accumulator lines and an approximately 2.4-fold increase compared to the GB non-transgenic accumulator line. As can been seen from Table 1, the range of accumulated GB in the transgenic plants was from 0.1mM to 22.6mM. $R_o$ transgenic plants were outcrossed and progeny seed prepared for propagation of $F_1$, $F_2$ and other generations of progeny plants and seeds and additional analysis of glycine-betaine indicated that the metabolite continued to accumulate to increased levels in the progeny plants (see for example, Tables 3 and 4 in Example 5).

Studies also indicated that the amount of GB in tissue increased with the age of the plant. For example, older VT leaves of a non-transgenic FBLL x LH59 hybrid accumulated more of the metabolite than younger V5 leaves.

**Table 1.** Glycine-betaine accumulation in $R_0$ gb1 transgenic plants.

| Line | Accumulator Type* | n | Mean GB(mM) | Std Dev | Min | Max |
|---|---|---|---|---|---|---|
| LH59/GB1 | Non-accumulator with transgene | 45[a] | 7.2 | 4.70 | 0.1 | 22.6 |
| LH59 | Non-accumulator | 11[b] | 0.1 | 0.14 | 0.008 | 0.4 |
| FBLL | Accumulator | 4[b] | 3.0 | 0.59 | 2.1 | 3.66 |
| *Indicates characterization of maize line without transgene.<br>a = represents 45 different events, one plant from each event.<br>b = represents the number of individual plants of each non-transgenic line | | | | | | |

EXAMPLE 3

*Expression of exogenous DNA constructs comprising gb1 coding sequence and homologs in transgenic Zea mays*

In substantially the same manner as in Example 2, a variety of exogenous DNA constructs comprising a gb1 coding sequence are transformed into the GB non-accumulating maize line (LH59) and a GB accumulating maize line (FBLL MAB , U.S Patent Application Publication 20040016030, incorporated herein by reference). The gb1-containing DNA constructs are substantially similar to the construct illustrated in Figure 3 except for combinations of promoter and gb1 coding sequence as described Table 2 where the promoter identified by "promoter sequence" replaces the rice actin 1 promoter and the gb1 coding sequence identified by "gb1 DNA sequence" replaces the maize gb1 coding sequence. The rice actin 1 intron is retained or deleted or replaced with other introns in the various constructs.

**Table 2.** Coding sequence and promoter combinations for the expression of gb1 coding sequences.

| Promoter | Promoter sequence | gb1 | gb1 coding sequence |
|---|---|---|---|
| hsp17.5 promoter | SEQ ID NO:36 | maize gb1 | SEQ ID NO:19 |
| hva22 promoter | SEQ ID NO:37 | maize gb1 | SEQ ID NO:19 |
| ca4h promoter | SEQ ID NO:38 | maize gb1 | SEQ ID NO:19 |
| rab-17 promoter | SEQ ID NO:39 | maize gb1 | SEQ ID NO:19 |
| rab-17 promoter | SEQ ID NO:40 | maize gb1 | SEQ ID NO:19 |
| hsp17.5 promoter | SEQ ID NO:41 | maize gb1 | SEQ ID NO:19 |
| hva22 promoter | SEQ ID NO:42 | maize gb1 | SEQ ID NO:19 |
| ca4h promoter | SEQ ID NO:43 | maize gb1 | SEQ ID NO:19 |
| hsp16.9 promoter | SEQ ID NO:44 | maize gb1 | SEQ ID NO:19 |
| hsp22 promoter | SEQ ID NO:45 | maize gb1 | SEQ ID NO:19 |
| rab-17 promoter | SEQ ID NO:46 | maize gb1 | SEQ ID NO:19 |
| maize gb1 promoter | SEQ ID NO:47 | maize gb1 | SEQ ID NO:19 |
| maize cvy-cik1 promoter | SEQ ID NO:48 | maize gb1 | SEQ ID NO:19 |
| maize cvy-cik1 promoter | SEQ ID NO:49 | maize gb1 | SEQ ID NO:19 |
| maize cvy-cik1 promoter | SEQ ID NO:50 | maize gb1 | SEQ ID NO:19 |
| maize cvy-cik1 promoter | SEQ ID NO:51 | maize gb1 | SEQ ID NO:19 |
| maize cvy-cik1 promoter | SEQ ID NO:52 | maize gb1 | SEQ ID NO:19 |
| rice cvy-cik1 promoter | SEQ ID NO:53 | maize gb1 | SEQ ID NO:19 |
| rtbv promoter | SEQ ID NO:54 | maize gb1 | SEQ ID NO:19 |
| maize nas promoter | SEQ ID NO:55 | maize gb1 | SEQ ID NO:19 |
| coix hrgp promoter | SEQ ID NO:56 | maize gb1 | SEQ ID NO:19 |
| | | | |

(continued)

| Promoter | Promoter sequence | gb1 | gb1 coding sequence |
|---|---|---|---|
| rice actin 1 promoter and intron | SEQ ID NO:35 | maize gb1-2 | SEQ ID NO:20 |
| hsp17.5 promoter | SEQ ID NO:36 | maize gb1-2 | SEQ ID NO:20 |
| hva22 promoter | SEQ ID NO:37 | maize gb1-2 | SEQ ID NO:20 |
| ca4h promoter | SEQ ID NO:38 | maize gb1-2 | SEQ ID NO:20 |
| rab-17 promoter | SEQ ID NO:39 | maize gb1-2 | SEQ ID NO:20 |
| rab-17 promoter | SEQ ID NO:40 | maize gb1-2 | SEQ ID NO:20 |
| hsp17.5 promoter | SEQ ID NO:41 | maize gb1-2 | SEQ ID NO:20 |
| hva22 promoter | SEQ ID NO:42 | maize gb1-2 | SEQ ID NO:20 |
| ca4h promoter | SEQ ID NO:43 | maize gb1-2 | SEQ ID NO:20 |
| hsp16.9 promoter | SEQ ID NO:44 | maize gb1-2 | SEQ ID NO:20 |
| hsp22 promoter | SEQ ID NO:45 | maize gb1-2 | SEQ ID NO:20 |
| rab-17 promoter | SEQ ID NO:46 | maize gb1-2 | SEQ ID NO:20 |
| maize gb1 promoter | SEQ ID NO:47 | maize gb1-2 | SEQ ID NO:20 |
| maize cvy-cik1 promoter | SEQ ID NO:48 | maize gb1-2 | SEQ ID NO:20 |
| maize cvy-cik1 promoter | SEQ ID NO:49 | maize gb1-2 | SEQ ID NO:20 |
| maize cvy-cik1 promoter | SEQ ID NO:50 | maize gb1-2 | SEQ ID NO:20 |
| maize cvy-cik1 promoter | SEQ ID NO:51 | maize gb1-2 | SEQ ID NO:20 |
| maize cvy-cik1 promoter | SEQ ID NO:52 | maize gb1-2 | SEQ ID NO:20 |
| rice cvy-cik1 promoter | SEQ ID NO:53 | maize gb1-2 | SEQ ID NO:20 |
| rtbv promoter | SEQ ID NO:54 | maize gb1-2 | SEQ ID NO:20 |
| maize nas promoter | SEQ ID NO:55 | maize gb1-2 | SEQ ID NO:20 |
| coix hrgp promoter | SEQ ID NO:56 | maize gb1-2 | SEQ ID NO:20 |
| | | | |
| rice actin 1 promoter and intron | SEQ ID NO:35 | rice gb1-1 | SEQ ID NO:21 |
| hsp17.5 promoter | SEQ ID NO:36 | rice gb1-1 | SEQ ID NO:21 |
| hva22 promoter | SEQ ID NO:37 | rice gb1-1 | SEQ ID NO:21 |
| ca4h promoter | SEQ ID NO:38 | rice gb1-1 | SEQ ID NO:21 |
| rab-17 promoter | SEQ ID NO:39 | rice gb1-1 | SEQ ID NO:21 |
| rab-17 promoter | SEQ ID NO:40 | rice gb1-1 | SEQ ID NO:21 |
| hsp17.5 promoter | SEQ ID NO:41 | rice gb1-1 | SEQ ID NO:21 |
| hva22 promoter | SEQ ID NO:42 | rice gb1-1 | SEQ ID NO:21 |
| ca4h promoter | SEQ ID NO:43 | rice gb1-1 | SEQ ID NO:21 |
| hsp16.9 promoter | SEQ ID NO:44 | rice gb1-1 | SEQ ID NO:21 |
| hsp22 promoter | SEQ ID NO:45 | rice gb1-1 | SEQ ID NO:21 |
| rab-17 promoter | SEQ ID NO:46 | rice gb1-1 | SEQ ID NO:21 |
| maize gb1 promoter | SEQ ID NO:47 | rice gb1-1 | SEQ ID NO:21 |
| maize cvy-cik1 promoter | SEQ ID NO:48 | ricegb1-1 | SEQ ID NO:21 |
| maize cvy-cik1 promoter | SEQ ID NO:49 | rice gb1-1 | SEQ ID NO:21 |

(continued)

| Promoter | Promoter sequence | gb1 | gb1 coding sequence |
|---|---|---|---|
| maize cvy-cik1 promoter | SEQ ID NO:50 | rice gb1-1 | SEQ ID NO:21 |
| maize cvy-cik1 promoter | SEQ ID NO:51 | rice gb1-1 | SEQ ID NO:21 |
| maize cvy-cik1 promoter | SEQ ID NO:52 | rice gb1-1 | SEQ ID NO:21 |
| rice cvy-cik1 promoter | SEQ ID NO:53 | rice gb1-1 | SEQ ID NO:21 |
| rtbv promoter | SEQ ID NO:54 | rice gb1-1 | SEQ ID NO:21 |
| maize nas promoter | SEQ ID NO:55 | rice gb1-1 | SEQ ID NO:21 |
| coix hrgp promoter | SEQ ID NO:56 | rice gb1-1 | SEQ ID NO:21 |
| | | | |
| rice actin 1 promoter and intron | SEQ ID NO:35 | barley gb1-1 | SEQ ID NO:22 |
| hsp17.5 promoter | SEQ ID NO:36 | barley gb1-1 | SEQ ID NO:22 |
| hva22 promoter | SEQ ID NO:37 | barley gb1-1 | SEQ ID NO:22 |
| ca4h promoter | SEQ ID NO:38 | barley gb1-1 | SEQ ID NO:22 |
| rab-17 promoter | SEQ ID NO:39 | barley gb1-1 | SEQ ID NO:22 |
| rab-17 promoter | SEQ ID NO:40 | barley gb1-1 | SEQ ID NO:22 |
| hsp17.5 promoter | SEQ ID NO:41 | barley gb1-1 | SEQ ID NO:22 |
| hva22 promoter | SEQ ID NO:42 | barley gb1-1 | SEQ ID NO:22 |
| ca4h promoter | SEQ ID NO:43 | barley gb1-1 | SEQ ID NO:22 |
| hsp16.9 promoter | SEQ ID NO:44 | barley gb1-1 | SEQ ID NO:22 |
| hsp22 promoter | SEQ ID NO:45 | barley gb1-1 | SEQ ID NO:22 |
| rab-17 promoter | SEQ ID NO:46 | barley gbl-1 | SEQ ID NO:22 |
| maize gb1 promoter | SEQ ID NO:47 | barley gb1-1 | SEQ ID NO:22 |
| maize cvy-cik1 promoter | SEQ ID NO:48 | barley gb1-1 | SEQ ID NO:22 |
| maize cvy-cik1 promoter | SEQ ID NO:49 | barley gb1-1 | SEQ ID NO:22 |
| maize cvy-cik1 promoter | SEQ ID NO:50 | barley gb1-1 | SEQ ID NO:22 |
| maize cvy-cik1 promoter | SEQ ID NO:51 | barley gb1-1 | SEQ ID NO:22 |
| maize cvy-cik1 promoter | SEQ ID NO:52 | barley gb1-1 | SEQ ID NO:22 |
| rice cvy-cik1 promoter | SEQ ID NO:53 | barley gb1-1 | SEQ ID NO:22 |
| rtbv promoter | SEQ ID NO:54 | barley gb1-1 | SEQ ID NO:22 |
| maize nas promoter | SEQ ID NO:55 | barley gb1-1 | SEQ ID NO:22 |
| coix hrgp promoter | SEQ ID NO:56 | barley gb1-1 | SEQ ID NO:22 |
| | | | |
| rice actin 1 promoter and intron | SEQ ID NO:35 | maize gb1-3-1 | SEQ ID NO:23 |
| hsp17.5 promoter | SEQ ID NO:36 | maize gb1-3-1 | SEQ ID NO:23 |
| hva22 promoter | SEQ ID NO:37 | maize gb1-3-1 | SEQ ID NO:23 |
| ca4h promoter | SEQ ID NO:38 | maize gb1-3-1 | SEQ ID NO:23 |
| rab-17 promoter | SEQ ID NO:39 | maize gb1-3-1 | SEQ ID NO:23 |
| rab-17 promoter | SEQ ID NO:40 | maize gbl-3-1 | SEQ ID NO:23 |
| hsp17.5 promoter | SEQ ID NO:41 | maize gb1-3-1 | SEQ ID NO:23 |

(continued)

| Promoter | Promoter sequence | gb1 | gb1 coding sequence |
|---|---|---|---|
| hva22 promoter | SEQ ID NO:42 | maize gb1-3-1 | SEQ ID NO:23 |
| ca4h promoter | SEQ ID NO:43 | maize gb1-3-1 | SEQ ID NO:23 |
| hsp16.9 promoter | SEQ ID NO:44 | maize gb1-3-1 | SEQ ID NO:23 |
| hsp22 promoter | SEQ ID NO:45 | maize gb1-3-1 | SEQ ID NO:23 |
| rab-17 promoter | SEQ ID NO:46 | maize gb1-3-1 | SEQ ID NO:23 |
| maize gb1 promoter | SEQ ID NO:47 | maize gb1-3-1 | SEQ ID NO:23 |
| maize cvy-cik1 promoter | SEQ ID NO:48 | maize gb1-3-1 | SEQ ID NO:23 |
| maize cvy-cik1 promoter | SEQ ID NO:49 | maize gb1-3-1 | SEQ ID NO:23 |
| maize cvy-cik1 promoter | SEQ ID NO:50 | maize gb1-3-1 | SEQ ID NO:23 |
| maize cvy-cik1 promoter | SEQ ID NO:51 | maize gb1-3-1 | SEQ ID NO:23 |
| maize cvy-cik1 promoter | SEQ ID NO:52 | maize gb1-3-1 | SEQ ID NO:23 |
| rice cvy-cik1 promoter | SEQ ID NO:53 | maize gb1-3-1 | SEQ ID NO:23 |
| rtbv promoter | SEQ ID NO:54 | maize gb1-3-1 | SEQ ID NO:23 |
| maize nas promoter | SEQ ID NO:55 | maize gb1-3-1 | SEQ ID NO:23 |
| coix hrgp promoter | SEQ ID NO:56 | maize gb1-3-1 | SEQ ID NO:23 |
| | | | |
| rice actin 1 promoter and intron | SEQ ID NO:35 | leek gb1-3-1 | SEQ ID NO:24 |
| hsp17.5 promoter | SEQ ID NO:36 | leek gb1-3-1 | SEQ ID NO:24 |
| hva22 promoter | SEQ ID NO:37 | leek gb1-3-1 | SEQ ID NO:24 |
| ca4h promoter | SEQ ID NO:38 | leek gb1-3-1 | SEQ ID NO:24 |
| rab-17 promoter | SEQ ID NO:39 | leek gb1-3-1 | SEQ ID NO:24 |
| rab-17 promoter | SEQ ID NO:40 | leek gb1-3-1 | SEQ ID NO:24 |
| hsp17.5 promoter | SEQ ID NO:41 | leek gb1-3-1 | SEQ ID NO:24 |
| hva22 promoter | SEQ ID NO:42 | leek gb1-3-1 | SEQ ID NO:24 |
| ca4h promoter | SEQ ID NO:43 | leek gb1-3-1 | SEQ ID NO:24 |
| hsp16.9 promoter | SEQ ID NO:44 | leek gb1-3-1 | SEQ ID NO:24 |
| hsp22 promoter | SEQ ID NO:45 | leek gb1-3-1 | SEQ ID NO:24 |
| rab-17 promoter | SEQ ID NO:46 | leek gb1-3-1 | SEQ ID NO:24 |
| maize gb1 promoter | SEQ ID NO:47 | leek gb1-3-1 | SEQ ID NO:24 |
| maize cvy-cik1 promoter | SEQ ID NO:48 | leek gb1-3-1 | SEQ ID NO:24 |
| maize cvy-cik1 promoter | SEQ ID NO:49 | leek gb1-3-1 | SEQ ID NO:24 |
| maize cvy-cik1 promoter | SEQ ID NO:50 | leek gb1-3-1 | SEQ ID NO:24 |
| maize cvy-cik1 promoter | SEQ ID NO:51 | leek gb1-3-1 | SEQ ID NO:24 |
| maize cvy-cik1 promoter | SEQ ID NO:52 | leek gb1-3-1 | SEQ ID NO:24 |
| rice cvy-cik1 promoter | SEQ ID NO:53 | leek gb1-3-1 | SEQ ID NO:24 |
| rtbv promoter | SEQ ID NO:54 | leek gb1-3-1 | SEQ ID NO:24 |
| maize nas promoter | SEQ ID NO:55 | leek gb1-3-1 | SEQ ID NO:24 |
| coix hrgp promoter | SEQ ID NO:56 | leek gb1-3-1 | SEQ ID NO:24 |

(continued)

| Promoter | Promoter sequence | gb1 | gb1 coding sequence |
|---|---|---|---|
| | | | |
| rice actin 1 promoter and intron | SEQ ID NO:35 | At gb1-3-1 | SEQ ID NO:25 |
| hsp17.5 promoter | SEQ ID NO:36 | At gb1-3-1 | SEQ ID NO:25 |
| hva22 promoter | SEQ ID NO:37 | At gb1-3-1 | SEQ ID NO:25 |
| ca4h promoter | SEQ ID NO:38 | At gb1-3-1 | SEQ ID NO:25 |
| rab-17 promoter | SEQ ID NO:39 | At gb1-3-1 | SEQ ID NO:25 |
| rab-17 promoter | SEQ ID NO:40 | At gbl-3-1 | SEQ ID NO:25 |
| hsp17.5 promoter | SEQ ID NO:41 | At gb1-3-1 | SEQ ID NO:25 |
| hva22 promoter | SEQ ID NO:42 | At gb1-3-1 | SEQ ID NO:25 |
| ca4h promoter | SEQ ID NO:43 | At gb1-3-1 | SEQ ID NO:25 |
| hsp16.9 promoter | SEQ ID NO:44 | At gb1-3-1 | SEQ ID NO:25 |
| hsp22 promoter | SEQ ID NO:45 | At gb1-3-1 | SEQ ID NO:25 |
| rab-17 promoter | SEQ ID NO:46 | At gb1-3-1 | SEQ ID NO:25 |
| maize gb1 promoter | SEQ ID NO:47 | At gb1-3-1 | SEQ ID NO:25 |
| maize cvy-cik1 promoter | SEQ ID NO:48 | At gb1-3-1 | SEQ ID NO:25 |
| maize cvy-cik1 promoter | SEQ ID NO:49 | At gb1-3-1 | SEQ ID NO:25 |
| maize cvy-cik1 promoter | SEQ ID NO:50 | At gb1-3-1 | SEQ ID NO:25 |
| maize cvy-cik1 promoter | SEQ ID NO:51 | At gb1-3-1 | SEQ ID NO:25 |
| maize cvy-cik1 promoter | SEQ ID NO:52 | At gb1-3-1 | SEQ ID NO:25 |
| rice cvy-cik1 promoter | SEQ ID NO:53 | At gb1-3-1 | SEQ ID NO:25 |
| rtbv promoter | SEQ ID NO:54 | At gb1-3-1 | SEQ ID NO:25 |
| maize nas promoter | SEQ ID NO:55 | At gb1-3-1 | SEQ ID NO:25 |
| coix hrgp promoter | SEQ ID NO:56 | At gb1-3-1 | SEQ ID NO:25 |
| | | | |
| rice actin 1 promoter and intron | SEQ ID NO:35 | At gb1-3-2 | SEQ ID NO:26 |
| hsp17.5 promoter | SEQ ID NO:36 | At gb1-3-2 | SEQ ID NO:26 |
| hva22 promoter | SEQ ID NO:37 | At gb1-3-2 | SEQ ID NO:26 |
| ca4h promoter | SEQ ID NO:38 | At gb1-3-2 | SEQ ID NO:26 |
| rab-17 promoter | SEQ ID NO:39 | At gb1-3-2 | SEQ ID NO:26 |
| rab-17 promoter | SEQ ID NO:40 | At gb1-3-2 | SEQ ID NO:26 |
| hsp17.5 promoter | SEQ ID NO:41 | At gb1-3-2 | SEQ ID NO:26 |
| hva22 promoter | SEQ ID NO:42 | At gb1-3-2 | SEQ ID NO:26 |
| ca4h promoter | SEQ ID NO:43 | At gb1-3-2 | SEQ ID NO:26 |
| hsp16.9 promoter | SEQ ID NO:44 | At gb1-3-2 | SEQ ID NO:26 |
| hsp22 promoter | SEQ ID NO:45 | At gb1-3-2 | SEQ ID NO:26 |
| rab-17 promoter | SEQ ID NO:46 | At gb1-3-2 | SEQ ID NO:26 |
| maize gb1 promoter | SEQ ID NO:47 | At gb1-3-2 | SEQ ID NO:26 |
| maize cvy-cik1 promoter | SEQ ID NO:48 | At gb1-3-2 | SEQ ID NO:26 |

(continued)

| Promoter | Promoter sequence | gb1 | gb1 coding sequence |
|---|---|---|---|
| maize cvy-cik1 promoter | SEQ ID NO:49 | At gb1-3-2 | SEQ ID NO:26 |
| maize cvy-cik1 promoter | SEQ ID NO:50 | At gb1-3-2 | SEQ ID NO:26 |
| maize cvy-cik1 promoter | SEQ ID NO:51 | At gb1-3-2 | SEQ ID NO:26 |
| maize cvy-cik1 promoter | SEQ ID NO:52 | At gb1-3-2 | SEQ ID NO:26 |
| rice cvy-cik1 promoter | SEQ ID NO:53 | At gb1-3-2 | SEQ ID NO:26 |
| rtbv promoter | SEQ ID NO:54 | At gb1-3-2 | SEQ ID NO:26 |
| maize nas promoter | SEQ ID NO:55 | At gb1-3-2 | SEQ ID NO:26 |
| coix hrgp promoter | SEQ ID NO:56 | At gb1-3-2 | SEQ ID NO:26 |
| | | | |
| rice actin 1 promoter and intron | SEQ ID NO:35 | At gb1-3-3 | SEQ ID NO:27 |
| hsp17.5 promoter | SEQ ID NO:36 | At gb1-3-3 | SEQ ID NO:27 |
| hva22 promoter | SEQ ID NO:37 | At gb1-3-3 | SEQ ID NO:27 |
| ca4h promoter | SEQ ID NO:38 | At gb1-3-3 | SEQ ID NO:27 |
| rab-17 promoter | SEQ ID NO:39 | At gb1-3-3 | SEQ ID NO:27 |
| rab-17 promoter | SEQ ID NO:40 | At gb1-3-3 | SEQ ID NO:27 |
| hsp17.5 promoter | SEQ ID NO:41 | At gb1-3-3 | SEQ ID NO:27 |
| hva22 promoter | SEQ ID NO:42 | At gb1-3-3 | SEQ ID NO:27 |
| ca4h promoter | SEQ ID NO:43 | At gb1-3-3 | SEQ ID NO:27 |
| hsp16.9 promoter | SEQ ID NO:44 | At gb1-3-3 | SEQ ID NO:27 |
| hsp22 promoter | SEQ ID NO:45 | At gb1-3-3 | SEQ ID NO:27 |
| rab-17 promoter | SEQ ID NO:46 | At gb1-3-3 | SEQ ID NO:27 |
| maize gb1 promoter | SEQ ID NO:47 | At gb1-3-3 | SEQ ID NO:27 |
| maize cvy-cik1 promoter | SEQ ID NO:48 | At gb1-3-3 | SEQ ID NO:27 |
| maize cvy-cik1 promoter | SEQ ID NO:49 | At gb1-3-3 | SEQ ID NO:27 |
| maize cvy-cik1 promoter | SEQ ID NO:50 | At gb1-3-3 | SEQ ID NO:27 |
| maize cvy-cik1 promoter | SEQ ID NO:51 | At gb1-3-3 | SEQ ID NO:27 |
| maize cvy-cik1 promoter | SEQ ID NO:52 | At gb1-3-3 | SEQ ID NO:27 |
| rice cvy-cik1 promoter | SEQ ID NO:53 | At gb1-3-3 | SEQ ID NO:27 |
| rtbv promoter | SEQ ID NO:54 | At gb1-3-3 | SEQ ID NO:27 |
| maize nas promoter | SEQ ID NO:55 | At gb1-3-3 | SEQ ID NO:27 |
| coix hrgp promoter | SEQ ID NO:56 | At gb1-3-3 | SEQ ID NO:27 |
| | | | |
| rice actin 1 promoter and intron | SEQ ID NO:35 | At gb1-3-4 | SEQ ID NO:28 |
| hsp17.5 promoter | SEQ ID NO:36 | At gb1-3-4 | SEQ ID NO:28 |
| hva22 promoter | SEQ ID NO:37 | At gb1-3-4 | SEQ ID NO:28 |
| ca4h promoter | SEQ ID NO:38 | At gb1-3-4 | SEQ ID NO:28 |
| rab-17 promoter | SEQ ID NO:39 | At gb1-3-4 | SEQ ID NO:28 |
| rab-17 promoter | SEQ ID NO:40 | At gb1-3-4 | SEQ ID NO:28 |

(continued)

| Promoter | Promoter sequence | gb1 | gb1 coding sequence |
|---|---|---|---|
| hsp17.5 promoter | SEQ ID NO:41 | At gb1-3-4 | SEQ ID NO:28 |
| hva22 promoter | SEQ ID NO:42 | At gb1-3-4 | SEQ ID NO:28 |
| ca4h promoter | SEQ ID NO:43 | At gb1-3-4 | SEQ ID NO:28 |
| hsp16.9 promoter | SEQ ID NO:44 | At gb1-3-4 | SEQ ID NO:28 |
| hsp22 promoter | SEQ ID NO:45 | At gb1-3-4 | SEQ ID NO:28 |
| rab-17 promoter | SEQ ID NO:46 | At gb1-3-4 | SEQ ID NO:28 |
| maize gb1 promoter | SEQ ID NO:47 | At gb1-3-4 | SEQ ID NO:28 |
| maize cvy-cik1 promoter | SEQ ID NO:48 | At gb1-3-4 | SEQ ID NO:28 |
| maize cvy-cik1 promoter | SEQ ID NO:49 | At gb1-3-4 | SEQ ID NO:28 |
| maize cvy-cik1 promoter | SEQ ID NO:50 | At gb1-3-4 | SEQ ID NO:28 |
| maize cvy-cik1 promoter | SEQ ID NO:51 | At gb1-3-4 | SEQ ID NO:28 |
| maize cvy-cik1 promoter | SEQ ID NO:52 | At gb1-3-4 | SEQ ID NO:28 |
| rice cvy-cik1 promoter | SEQ ID NO:53 | At gb1-3-4 | SEQ ID NO:28 |
| rtbv promoter | SEQ ID NO:54 | At gb1-3-4 | SEQ ID NO:28 |
| maize nas promoter | SEQ ID NO:55 | At gb1-3-4 | SEQ ID NO:28 |
| coix hrgp promoter | SEQ ID NO:56 | At gb1-3-4 | SEQ ID NO:28 |
| | | | |
| rice actin 1 promoter and intron | SEQ ID NO:35 | Bn gb1-3-1 | SEQ ID NO:29 |
| hsp17.5 promoter | SEQ ID NO:36 | Bn gb1-3-1 | SEQ ID NO:29 |
| hva22 promoter | SEQ ID NO:37 | Bn gb1-3-1 | SEQ ID NO:29 |
| ca4h promoter | SEQ ID NO:38 | Bn gb1-3-1 | SEQ ID NO:29 |
| rab-17 promoter | SEQ ID NO:39 | Bn gb1-3-1 | SEQ ID NO:29 |
| rab-17 promoter | SEQ ID NO:40 | Bn gb1-3-1 | SEQ ID NO:29 |
| hsp17.5 promoter | SEQ ID NO:41 | Bn gb1-3-1 | SEQ ID NO:29 |
| hva22 promoter | SEQ ID NO:42 | Bn gb1-3-1 | SEQ ID NO:29 |
| ca4h promoter | SEQ ID NO:43 | Bn gb1-3-1 | SEQ ID NO:29 |
| hsp16.9 promoter | SEQ ID NO:44 | Bn gb1-3-1 | SEQ ID NO:29 |
| hsp22 promoter | SEQ ID NO:45 | Bn gb1-3-1 | SEQ ID NO:29 |
| rab-17 promoter | SEQ ID NO:46 | Bn gb1-3-1 | SEQ ID NO:29 |
| maize gb1 promoter | SEQ ID NO:47 | Bn gb1-3-1 | SEQ ID NO:29 |
| maize cvy-cik1 promoter | SEQ ID NO:48 | Bn gb1-3-1 | SEQ ID NO:29 |
| maize cvy-cik1 promoter | SEQ ID NO:49 | Bn gb1-3-1 | SEQ ID NO:29 |
| maize cvy-cik1 promoter | SEQ ID NO:50 | Bn gb1-3-1 | SEQ ID NO:29 |
| maize cvy-cik1 promoter | SEQ ID NO:51 | Bn gb1-3-1 | SEQ ID NO:29 |
| maize cvy-cik1 promoter | SEQ ID NO:52 | Bn gb1-3-1 | SEQ ID NO:29 |
| rice cvy-cik1 promoter | SEQ ID NO:53 | Bn gb1-3-1 | SEQ ID NO:29 |
| rtbv promoter | SEQ ID NO:54 | Bn gb1-3-1 | SEQ ID NO:29 |
| maize nas promoter | SEQ ID NO:55 | Bn gb1-3-1 | SEQ ID NO:29 |

(continued)

| Promoter | Promoter sequence | gb1 | gb1 coding sequence |
|---|---|---|---|
| coix hrgp promoter | SEQ ID NO:56 | Bn gb1-3-1 | SEQ ID NO:29 |
| | | | |
| rice actin 1 promoter and intron | SEQ ID NO:35 | soybean gb1-3-1 | SEQ ID NO:30 |
| hsp17.5 promoter | SEQ ID NO:36 | soybean gb1-3-1 | SEQ ID NO:30 |
| hva22 promoter | SEQ ID NO:37 | soybean gb1-3-1 | SEQ ID NO:30 |
| ca4h promoter | SEQ ID NO:38 | soybean gb1-3-1 | SEQ ID NO:30 |
| rab-17 promoter | SEQ ID NO:39 | soybean gb1-3-1 | SEQ ID NO:30 |
| rab-17 promoter | SEQ ID NO:40 | soybean gb1-3-1 | SEQ ID NO:30 |
| hsp17.5 promoter | SEQ ID NO:41 | soybean gb1-3-1 | SEQ ID NO:30 |
| hva22 promoter | SEQ ID NO:42 | soybean gb1-3-1 | SEQ ID NO:30 |
| ca4h promoter | SEQ ID NO:43 | soybean gb1-3-1 | SEQ ID NO:30 |
| hsp16.9 promoter | SEQ ID NO:44 | soybean gb1-3-1 | SEQ ID NO:30 |
| hsp22 promoter | SEQ ID NO:45 | soybean gb1-3-1 | SEQ ID NO:30 |
| rab-17 promoter | SEQ ID NO:46 | soybean gb1-3-1 | SEQ ID NO:30 |
| maize gb1 promoter | SEQ ID NO:47 | soybean gb1-3-1 | SEQ ID NO:30 |
| maize cvy-cik1 promoter | SEQ ID NO:48 | soybean gb1-3-1 | SEQ ID NO:30 |
| maize cvy-cik1 promoter | SEQ ID NO:49 | soybean gb1-3-1 | SEQ ID NO:30 |
| maize cvy-cik1 promoter | SEQ ID NO:50 | soybean gb1-3-1 | SEQ ID NO:30 |
| maize cvy-cik1 promoter | SEQ ID NO:51 | soybean gb1-3-1 | SEQ ID NO:30 |
| maize cvy-cik1 promoter | SEQ ID NO:52 | soybean gb1-3-1 | SEQ ID NO:30 |
| rice cvy-cik1 promoter | SEQ ID NO:53 | soybean gb1-3-1 | SEQ ID NO:30 |
| rtbv promoter | SEQ ID NO:54 | soybean gb1-3-1 | SEQ ID NO:30 |
| maize nas promoter | SEQ ID NO:55 | soybean gb1-3-1 | SEQ ID NO:30 |
| coix hrgp promoter | SEQ ID NO:56 | soybean gb1-3-1 | SEQ ID NO:30 |
| | | | |
| rice actin 1 promoter and intron | SEQ ID NO:35 | soybean gb1-3-2 | SEQ ID NO:31 |
| hsp17.5 promoter | SEQ ID NO:36 | soybean gb1-3-2 | SEQ ID NO:31 |
| hva22 promoter | SEQ ID NO:37 | soybean gb1-3-2 | SEQ ID NO:31 |
| ca4h promoter | SEQ ID NO:38 | soybean gb1-3-2 | SEQ ID NO:31 |
| rab-17 promoter | SEQ ID NO:39 | soybean gb1-3-2 | SEQ ID NO:31 |
| rab-17 promoter | SEQ ID NO:40 | soybean gb1-3-2 | SEQ ID NO:31 |
| hsp17.5 promoter | SEQ ID NO:41 | soybean gb1-3-2 | SEQ ID NO:31 |
| hva22 promoter | SEQ ID NO:42 | soybean gb1-3-2 | SEQ ID NO:31 |
| ca4h promoter | SEQ ID NO:43 | soybean gb1-3-2 | SEQ ID NO:31 |
| hsp16.9 promoter | SEQ ID NO:44 | soybean gb1-3-2 | SEQ ID NO:31 |
| hsp22 promoter | SEQ ID NO:45 | soybean gb1-3-2 | SEQ ID NO:31 |
| rab-17 promoter | SEQ ID NO:46 | soybean gb1-3-2 | SEQ ID NO:31 |
| maize gb1 promoter | SEQ ID NO:47 | soybean gb1-3-2 | SEQ ID NO:31 |

(continued)

| Promoter | Promoter sequence | gb1 | gb1 coding sequence |
|---|---|---|---|
| maize cvy-cik1 promoter | SEQ ID NO:48 | soybean gb1-3-2 | SEQ ID NO:31 |
| maize cvy-cik1 promoter | SEQ ID NO:49 | soybean gb1-3-2 | SEQ ID NO:31 |
| maize cvy-cik1 promoter | SEQ ID NO:50 | soybean gb1-3-2 | SEQ ID NO:31 |
| maize cvy-cik1 promoter | SEQ ID NO:51 | soybean gb1-3-2 | SEQ ID NO:31 |
| maize cvy-cik1 promoter | SEQ ID NO:52 | soybean gb1-3-2 | SEQ ID NO:31 |
| rice cvy-cik1 promoter | SEQ ID NO:53 | soybean gb1-3-2 | SEQ ID NO:31 |
| rtbv promoter | SEQ ID NO:54 | soybean gb1-3-2 | SEQ ID NO:31 |
| maize nas promoter | SEQ ID NO:55 | soybean gb1-3-2 | SEQ ID NO:31 |
| coix hrgp promoter | SEQ ID NO:56 | soybean gb1-3-2 | SEQ ID NO:31 |
| | | | |
| rice actin 1 promoter and intron | SEQ ID NO:35 | barley gb1-3-1 | SEQ ID NO:32 |
| hsp17.5 promoter | SEQ ID NO:36 | barley gb1-3-1 | SEQ ID NO:32 |
| hva22 promoter | SEQ ID NO:37 | barley gb1-3-1 | SEQ ID NO:32 |
| ca4h promoter | SEQ ID NO:38 | barley gb1-3-1 | SEQ ID NO:32 |
| rab-17 promoter | SEQ ID NO:39 | barley gb1-3-1 | SEQ ID NO:32 |
| rab-17 promoter | SEQ ID NO:40 | barley gb1-3-1 | SEQ ID NO:32 |
| hsp17.5 promoter | SEQ ID NO:41 | barley gb1-3-1 | SEQ ID NO:32 |
| hva22 promoter | SEQ ID NO:42 | barley gb1-3-1 | SEQ ID NO:32 |
| ca4h promoter | SEQ ID NO:43 | barley gb1-3-1 | SEQ ID NO:32 |
| hsp16.9 promoter | SEQ ID NO:44 | barley gb1-3-1 | SEQ ID NO:32 |
| hsp22 promoter | SEQ ID NO:45 | barley gb1-3-1 | SEQ ID NO:32 |
| rab-17 promoter | SEQ ID NO:46 | barley gb1-3-1 | SEQ ID NO:32 |
| maize gb1 promoter | SEQ ID NO:47 | barley gb1-3-1 | SEQ ID NO:32 |
| maize cvy-cik1 promoter | SEQ ID NO:48 | barley gb1-3-1 | SEQ ID NO:32 |
| maize cvy-cik1 promoter | SEQ ID NO:49 | barley gb1-3-1 | SEQ ID NO:32 |
| maize cvy-cik1 promoter | SEQ ID NO:50 | barley gb1-3-1 | SEQ ID NO:32 |
| maize cvy-cik1 promoter | SEQ ID NO:51 | barley gb1-3-1 | SEQ ID NO:32 |
| maize cvy-cik1 promoter | SEQ ID NO:52 | barley gb1-3-1 | SEQ ID NO:32 |
| rice cvy-cik1 promoter | SEQ ID NO:53 | barley gb1-3-1 | SEQ ID NO:32 |
| rtbv promoter | SEQ ID NO:54 | barley gb1-3-1 | SEQ ID NO:32 |
| maize nas promoter | SEQ ID NO:55 | barley gb1-3-1 | SEQ ID NO:32 |
| coix hrgp promoter | SEQ ID NO:56 | barley gb1-3-1 | SEQ ID NO:32 |
| | | | |
| rice actin 1 promoter and intron | SEQ ID NO:35 | rice gb1-3-1 | SEQ ID NO:33 |
| hsp17.5 promoter | SEQ ID NO:36 | rice gb1-3-1 | SEQ ID NO:33 |
| hva22 promoter | SEQ ID NO:37 | rice gb1-3-1 | SEQ ID NO:33 |
| ca4h promoter | SEQ ID NO:38 | rice gb1-3-1 | SEQ ID NO:33 |
| rab-17 promoter | SEQ ID NO:39 | rice gb1-3-1 | SEQ ID NO:33 |

(continued)

| Promoter | Promoter sequence | gb1 | gb1 coding sequence |
|---|---|---|---|
| rab-17 promoter | SEQ ID NO:40 | rice gb1-3-1 | SEQ ID NO:33 |
| hsp17.5 promoter | SEQ ID NO:41 | rice gb1-3-1 | SEQ ID NO:33 |
| hva22 promoter | SEQ ID NO:42 | rice gb1-3-1 | SEQ ID NO:33 |
| ca4h promoter | SEQ ID NO:43 | rice gb1-3-1 | SEQ ID NO:33 |
| hsp16.9 promoter | SEQ ID NO:44 | rice gb1-3-1 | SEQ ID NO:33 |
| hsp22 promoter | SEQ ID NO:45 | rice gb1-3-1 | SEQ ID NO:33 |
| rab-17 promoter | SEQ ID NO:46 | rice gb1-3-1 | SEQ ID NO:33 |
| maize gb1 promoter | SEQ ID NO:47 | rice gb1-3-1 | SEQ ID NO:33 |
| maize cvy-cik1 promoter | SEQ ID NO:48 | rice gb1-3-1 | SEQ ID NO:33 |
| maize cvy-cik1 promoter | SEQ ID NO:49 | rice gb1-3-1 | SEQ ID NO:33 |
| maize cvy-cik1 promoter | SEQ ID NO:50 | rice gb1-3-1 | SEQ ID NO:33 |
| maize cvy-cik1 promoter | SEQ ID NO:51 | rice gb1-3-1 | SEQ ID NO:33 |
| maize cvy-cik1 promoter | SEQ ID NO:52 | rice gb1-3-1 | SEQ ID NO:33 |
| rice cvy-cik1 promoter | SEQ ID NO:53 | rice gb1-3-1 | SEQ ID NO:33 |
| rtbv promoter | SEQ ID NO:54 | rice gb1-3-1 | SEQ ID NO:33 |
| maize nas promoter | SEQ ID NO:55 | rice gb1-3-1 | SEQ ID NO:33 |
| coix hrgp promoter | SEQ ID NO:56 | rice gb1-3-1 | SEQ ID NO:33 |
| | | | |
| rice actin 1 promoter and intron | SEQ ID NO:35 | wheat gb1-3-1 | SEQ ID NO:34 |
| hsp17.5 promoter | SEQ ID NO:36 | wheat gb1-3-1 | SEQ ID NO:34 |
| hva22 promoter | SEQ ID NO:37 | wheat gb1-3-1 | SEQ ID NO:34 |
| ca4h promoter | SEQ ID NO:38 | wheat gb1-3-1 | SEQ ID NO:34 |
| rab-17 promoter | SEQ ID NO:39 | wheat gb1-3-1 | SEQ ID NO:34 |
| rab-17 promoter | SEQ ID NO:40 | wheat gb1-3-1 | SEQ ID NO:34 |
| hsp17.5 promoter | SEQ ID NO:41 | wheat gb1-3-1 | SEQ ID NO:34 |
| hva22 promoter | SEQ ID NO:42 | wheat gb1-3-1 | SEQ ID NO:34 |
| ca4h promoter | SEQ ID NO:43 | wheat gb1-3-1 | SEQ ID NO:34 |
| hsp16.9 promoter | SEQ ID NO:44 | wheat gb1-3-1 | SEQ ID NO:34 |
| hsp22 promoter | SEQ ID NO:45 | wheat gb1-3-1 | SEQ ID NO:34 |
| rab-17 promoter | SEQ ID NO:46 | wheat gb1-3-1 | SEQ ID NO:34 |
| maize gb1 promoter | SEQ ID NO:47 | wheat gb1-3-1 | SEQ ID NO:34 |
| maize cvy-cik1 promoter | SEQ ID NO:49 | wheat gb1-3-1 | SEQ ID NO:34 |
| maize cvy-cilk1 promoter | SEQ ID NO:49 | wheat gbl-3-1 | SEQ ID NO:34 |
| maize cvy-cik1 promoter | SEQ ID NO:50 | wheat gb1-3-1 | SEQ ID NO:34 |
| maize cvy-cik1 promoter | SEQ ID NO:51 | wheat gb1-3-1 | SEQ ID NO:34 |
| maize cvy-cik1 promoter | SEQ ID NO:52 | wheat gb1-3-1 | SEQ ID NO:34 |
| rice cvy-cik1 promoter | SEQ ID NO:53 | wheat gb1-3-1 | SEQ ID NO:34 |
| rtbv promoter | SEQ ID NO:54 | wheat gb1-3-1 | SEQ ID NO:34 |

(continued)

| Promoter | Promoter sequence | gb1 | gb1 coding sequence |
|---|---|---|---|
| maize nas promoter | SEQ ID NO:55 | wheat gb1-3-1 | SEQ ID NO:34 |
| coix hrgp promoter | SEQ ID NO:56 | wheat gb1-3-1 | SEQ ID NO:34 |

Transgenic plants produced with a water-deficit-inducible, cold inducible, other stress inducible or any other promoter operably linked to an exogenous gb1 coding sequence of the present invention (see for example, Table 2) are subjected to various growing conditions to demonstrate the effect of expressing a gb1 coding sequence in the transgenic plants. Plants are exposed to cold conditions, water-deficit conditions, heat, saline and other stresses in the field and under green house conditions. The plants are exposed to the stress condition for a period of time long enough and/or severe enough to induce the action of a stress-inducible promoter, *e.g.* withholding water for at least three days, before the collection of leaf tissue samples. Sample tissue is collected from transgenic plants expressing an exogenousgb1 coding sequence of the present invention for evaluation. Leaf tissue is collected from leaves of several ages (V2, V4, V6, V8 and VT) following water-deficit treatment or root tissue is collected at 12 hour intervals after a cold treatment. When collected tissue from the transgenic plant comprising and expressing an exogenous gb1 coding sequence described in Table 2 is analyzed for glycine-betaine, elevated levels of glycine-betaine compared to the non-transgenic maize line are measured, similar to elevated levels produced in the transgenic plant reported in the preceding Example 2 and increased stress-protection resulting from the expression of the gb1 coding sequence in the tissues is demonstrated.

EXAMPLE 4

*Tolerance to water deficit by transgenic plants and seed*

Transgenic maize and seed, prepared as described in Examples 2 and 3, are subjected to water-deficit conditions and examined for increased tolerance to water-deficit.

In a controlled environment such as a greenhouse, water-deficit is imposed upon the plants and seeds by germinating seed under water-deficit conditions, and imposing water-deficit conditions on seedlings and plants at various stage of development, such as at V2, V4, V6, V8 and VT. Water-deficit is induced by withholding or limiting water. Water-deficit is also induced by the application of saline and PEG solutions which induce water-deficit. In a less controlled environment, such as a field, water-deficit conditions are achieved by growing in a geographical location in which rainfall is usually limiting and by withholding irrigation.

Several parameters are measured to determine increased tolerance to water-deficit: plant height, leaf length, shoot mass, seed set, number of seed, yield, photosynthesis, turgor pressure, osmotic potential, leaf extension rate, and germination. In the practice of the current invention, maize plants and seeds expressing an exogenous gb1 coding sequence and producing enhanced glycine-betaine demonstrate increased tolerance to water-deficit compared to control plants lacking the transgene, *e.g.*, a non-transgenic segregant, a plant treated with GB or a plant that naturally accumulates GB. Moreover, a water-deficit tolerant maize plant and seed expressing an exogenous gb1 coding sequence has improved yield similar to, or increased upon, yield inherent in a GB accumulator maize line.

Transgenic soybean, cotton, canola and tobacco plants and seed are prepared with similar DNA constructs as described for maize, and similar water deficit studies carried out as described for maize. As compared to control plants and control seed lacking the exogenous DNA constructs, transgenic soybean cotton, canola and tobacco plants and seed with increased glycine-betaine content show increased tolerance for water-deficit conditions.

EXAMPLE 5

*Tolerance to cold by transgenic plants and seed*

Transgenic maize, prepared as described in Examples 2 and 3, are subjected to cold conditions and examined for increased tolerance to cold. Of particular interest is the ability of the seed of the transgenic maize to germinate under cold temperatures, to tolerate a period of cold temperature after germination, and the ability of a young seedling to tolerate a period of cold temperature.

Several parameters are measured to determine increased tolerance to cold such as measuring germination, plant height, leaf length, root length, root mass, shoot mass, chlorophyll fluorescence, and yield. In the practice of the current invention, transgenic maize plants and seed expressing an exogenous gb1 coding sequence and producing enhanced glycine-betaine demonstrate increased tolerance to cold conditions compared to control plants lacking the transgene, *e.g.*, a non-transgenic segregant, a plant treated with GB or a plant that naturally accumulates GB. For example, a transgenic cold tolerant maize plant or seed expressing an exogenous gb1 coding sequence has improved yield similar to, or increased upon, yield inherent in a GB accumulator maize line.

*Germination under cold condition*

Hybrid seeds were produced by crossing pMON78450 (Figure 3) transgenic $R_2$ plants prepared in Example 2 with two different tester lines: FBLL which naturally accumulates glycine-betaine, or LH244 which is naturally a non-accumulator line. The hybrid seeds from several different transgenic events comprising the exogenous gb1 coding sequence from pMON78450, as well as non-transgenic negative segregant maize kernels, were germinated under cold conditions. One hundred kernels were tested for each transgenic event and non-transgenic negative segregant. Batches of ten kernels each were germinated in Petri dishes lined with moistened filter paper in a growth chamber at approximately 9.5-9.8°C in constant darkness. Water was added to the plates throughout the test as necessary.

Each day, the number of seeds germinated per plate was counted. A seed was considered to be germinated when the root radicle reached 1cm. At the end of the test, root tip tissue was sampled from a number of seedlings per event and metabolites were extracted in order to determine the levels of glycine-betaine. For glycine-betaine measurements, samples were lyophilized, ground to a fine powder and metabolites extracted into an ethanol-based extraction buffer supplemented with deuterated glycine-betaine as an internal standard metabolite. Samples were analyzed by liquid chromatography-mass spectrometry/ mass spectrometry and the amount of glycine-betaine (in ppm) determined by analyzing the ratio of the deuterated and non-deuterated glycine-betaine in comparison to a standard curve.

A control experiment was also performed under non-cold conditions. Batches of ten kernels each were germinated in Petri dishes lined with moistened filter paper in a growth chamber set at 27°C in constant darkness. Fifty kernels were tested for each transgenic event and non-transgenic negative segregant.

Three different calculations were used to analyze the cold germination data:

1. Germination Index: This is a calculation which takes into account the time required by a given set of seeds, *e.g.*, the 100 kernels representing a transgenic event, to germinate relative to other sets of seeds in the test as well as the total number of seed which germinate in a given experiment. A higher germination index number indicates a faster germination time and better overall germination performance for a given set of seeds. The formula used is:

$$\text{Germination index} = ((T \times P1) + ((T-1) \times P2) + ((T-2) \times P3) + \ldots + (1 \times PT))/T$$

In which T = the total number of days of the test
P1, P2, P3, and PT= the percentage of seeds which germinated on that specific day of the test

2. Total Percent Germination: The percent of seeds which germinated for each set of seeds at the end of an experiment.

3. Days Until 50% Germination: This calculates the average number of days until half of the seeds being tested for a particular set of seeds have germinated. The model used to estimate the days to 50% germination is a three-parameter logistic model. This nonlinear model was fit using the statistical software package, JMP® (JMP®, version 5.1, 1989 - 2003 SAS Institute Inc. Cary, N.C.). The fitted model was found using an iterative optimization procedure.

*Germination under non-cold condition followed by cold condition: Early seedling test*

Seeds from a number of gb1 transgenic events and non-transgenic negative segregants were germinated on moistened vertical rolls of germination paper. Three rolls were set up for each selection, with 16 kernels used for each roll. For the cold assay, the seedlings were first germinated at about 23°C for three days before being transferred to a chamber at a constant 12°C for an additional 10 days. For the non-cold assay, seedlings were germinated in rolled germination paper at about 23°C for five days. At the end of the test period, root and shoot length were determined for the seeds exposed to cold and non-cold conditions.

*Germination under non-cold condition followed by cold condition: Young seedling soil test*

Seeds from a number of gb1 transgenic events and non-transgenic negative segregants were germinated in individual pots of soil at 23°C until they reached the V1 stage for testing (about 10 days; 12 hour light/dark cycle). The young seedlings were then transferred to cold condition (about 8°C during the light cycles, 5°C in the dark cycles) for 8 days, after which they were transferred back to non-cold condition (about 23°C) for recovery. On the fourth day of the cold treatment, the chlorophyll fluorescence of each of the young seedlings was measured. Three days after the young seedlings were returned to 23°C, two measurements were made: 1) leaf necrosis of each young seedling was estimated on the V2 leaf by visually estimating the percent of each V2 leaf which was still green at this stage and 2) the length of the V3 leaf (from soil to tip) was measured. This length was measured again at six days after recovery, to compare the growth rates after recovery for the transgenic and non-transgenic control young seedlings.

Tables 3 and 4 summarize the results of expressing an exogenous gb1 coding sequence having the sequence of SEQ ID NO:19 encoding a GB1 protein having an amino acid sequence of SEQ ID NO:1 which results in increased glycine-betaine on cold germination of transgenic hybrid maize seeds where one parent was a non-accumulating line (LH244; Table 3) or where one parent naturally accumulated glycine-betaine (FBLL; Table 4). The values for the Germination Index, Total Percent Germination and Days Until 50% Germination are reported as is the average amount of glycine-betaine accumulated by the transgenic or control negative segregant germinating root tip tissue.

The data indicate that of the eight gb1 transgenic events tested in the LH244 hybrid (Table 3), four events exhibited a statistically significant improvement in germination index and in total percent germination relative to the negative segregant seed. In addition, four events also demonstrated an improved germination time, as shown by the reduced number of days until 50% germination was achieved. In the early seedling test, the roots and shoots of the seedlings from one transgenic event were longer relative to the negative segregant, and in the young seedling soil test, the leaf length of one transgenic event was increased relative to the negative segregant. All events exhibiting an improvement in at least one cold germination, early seedling or young seedling characteristic accumulated at least 75 ppm glycine-betaine in the root tip tissue of the germinating seed. One event accumulating more than 75 ppm glycine-betaine did not exhibit an improvement in any of the measured parameters. On average, for all events in the LH244 hybrid plants, the improvement in germination index and total % germination in the cold were statistically significant at $P<0.0015$ and $P<0.0017$, respectively. The results from the non-cold condition germination test indicated that all of the seed used in the test were of good quality.

The data indicate that of the eight gb1 transgenic events tested in the FBLL hybrid (Table 4), three events exhibited a statistically significant improvement in germination index as well as in germination time relative to the negative segregant seed. One event demonstrated an improved total percent germination relative to the negative segregant seed. In the early seedling test, the roots and/or shoots of the seedlings from three transgenic events were longer relative to the negative segregant, and in the young seedling soil test, the leaf length of one transgenic event was increased relative to the negative segregant. On average, for all events in the FBLL hybrid plants, the improvement in germination index and total % germination in the cold were statistically significant at $P<0.0037$ and $P<0.1403$, respectively. The results from the non-cold condition germination test indicate that all of the seed used in the test were of good quality.

The results reported in Tables 3 and 4 show that over-expression of the maize gb1 transgene, and resultant increase in glycine-betaine accumulation, increases the cold germination of the non-accumulator LH244 seeds to a greater degree than that of the naturally accumulating FBLL line in these tests.

**Table 3 Effect of gb1 over-expression in LH244 (non-accumulating line)**

| | | ppm GB | Germination Index | Total % Germination | Days to 50% Germination |
|---|---|---|---|---|---|
| Event | Transgene[a] | Avg[b] | Avg[c] | Avg[c] | Avg[c] |
| M44196 | POS | 151 | 27.81 | 77 | 16.7 |
| M44196 | neg | 2 | 41.78 | 98 | 15.6 |
| M44199 | POS | 269 | 41.77 | 96 | 15.3^ |
| M44199 | neg | 2 | 38.06 | 93 | 16.2 |
| M44202 | POS | 113 | 31.82**** | 89* | 16.8^ |
| M44202 | neg | 1 | 19.82 | 77 | 19.2 |
| M45434 | POS | 76 | 31.99 | 90 | 17.3^ |
| M45434 | neg | 2 | 27.74 | 85 | 18.1 |
| M45441 | POS | 48 | 29.91 | 90 | 18.0 |
| M45441 | neg | 2 | 32.36 | 94 | 17.3 |
| M45444 | POS | 181 | 25.19**** | 87*** | 18.8^ |
| M45444 | neg | 2 | 13.34 | 57 | 19.8 |
| M46403 | POS | 184 | 29.74**** | 82**** | 17.1 |

(continued)

| | | ppm GB | Germination Index | Total % Germination | Days to 50% Germination |
|---|---|---|---|---|---|
| M46403 | neg | 1 | 17.96 | 48 | 16.6 |
| M46411 | POS | 124 | 27.91* | 83* | 17.9 |
| M46411 | neg | 2 | 22.71 | 72 | 17.9 |
| Average | POS | | 31.2** | 87.2** | |
| Average | Neg | | 27 | 79.4 | |

(a) Pos = presence of the exogenous maize gb1 coding sequence (pMON78450; SEQ ID NO:19); Neg = the absence of the gb1 transgenic coding sequence.
(b) Average is per 3 replicates of 3 pieces of root tissue measured under cold condition
(c) Average is per set of 100 kernels
(*) P<0.06, positive improvement
(**) P<0.01, positive improvement
(***) P<0.001, positive improvement
(****)P<0.0001, positive improvement
(^) high and low confidence limit values did not overlap and at least about 1 day of improvement

**Table 4 Effect of gb1 over-expression in FBLL (accumulating line)**

| | | ppm gb | Germination Index | Total% Germination | Days to 50% Germination |
|---|---|---|---|---|---|
| Event | Transgene[a] | Avg[b] | Avg[c] | Avg[c] | Avg[c] |
| M44196 | POS | 109 | 46.9** | 99 | 14.4^ |
| M44196 | neg | 12 | 40.5 | 99 | 15.7 |
| M44199 | POS | 204 | 39.9 | 97 | 15.5 |
| M44199 | neg | 3 | 43.7 | 100 | 15.4 |
| M44202 | POS | 114 | 46.4 | 98 | 14.4 |
| M44202 | neg | 4 | 43.4 | 95 | 14.8 |
| M45434 | POS | 95 | 45.6*** | 99* | 14.6^ |
| M45434 | neg | 2 | 39.4 | 94 | 15.6 |
| M45441 | POS | 45 | 44.1 * | 96 | 14.8^ |
| M45441 | neg | 2 | 38.1 | 91 | 15.9 |
| M45444 | POS | 133 | 40.6 | 94 | 15.0 |
| M45444 | neg | 7 | 39.2 | 94 | 15.7 |
| M45445 | POS | 99 | 42.2 | 97 | 14.8 |
| M45445 | neg | 1 | 42.8 | 96 | 15.1 |
| M46403 | POS | 140 | 36.9 | 94 | 16 |
| M46403 | neg | 2 | 37.9 | 90 | 15.7 |
| Average | POS | | 42.5** | 96.8 | |

(continued)

| | | ppm gb | Germination Index | Total% Germination | Days to 50% Germination |
|---|---|---|---|---|---|
| Average | Neg | | 40.2 | 95.3 | |

(a) Pos = presence of the exogenous maize gb1 coding sequence (pMON78450; SEQ ID NO:19); Neg = the absence of the gb1 transgenic coding sequence.
(b) Average is per 3 replicates of 3 pieces of root tissue measured under cold condition. Note
that by the V2 stage, the non-transgenic FBLL line accumulated about 25-30 ppm GB compared to about 2-2.5 ppm for the non-transgenic LH244 line.
(c) Average is per set of 100 kernels
(*) P<0.06, positive improvement
(**) P<0.01, positive improvement
(***) P<0.001, positive improvement
(****)P<0.0001, positive improvement
(^) high and low confidence limit values did not overlap and at least about 1 day of improvement

Transgenic soybean, cotton, canola and tobacco are prepared with similar DNA constructs as described for maize, and similar studies carried out as described for maize. As compared to plants lacking the exogenous DNA constructs, *e.g.*, non-gb1 plants, transgenic soybean, cotton, canola and tobacco with increased glycine-betaine content show increased tolerance for cold conditions.

A transgenic seed having in its genome an exogenous DNA comprising a gb1 coding sequence which expresses a protein having an amino acid sequence comprising at least 25 contiguous amino acids of the consensus amino acid sequence of SEQ ID NO:17 or SEQ ID NO:18, wherein the activity of said protein increases glycine-betaine.

The transgenic seed as defined above wherein said protein is further characterized as having at least 40% identity to the amino acid sequence of SEQ ID NO:1.

The transgenic seed as defined above wherein said protein has an amino acid sequence which is in the group consisting of SEQ ID NOS:1-16.

The transgenic seed as defined above wherein said protein has the amino acid sequence of SEQ ID NO:1.

The transgenic seed as defined above wherein said exogenous DNA comprising a gb1 coding sequence has at least 98% identity to a nucleotide sequence in the group consisting of SEQ ID NOS:19-34.

The transgenic seed as defined above wherein said exogenous DNA comprising a gb1 coding sequence has a nucleotide sequence in the group consisting of SEQ ID NOS:19-34.

The transgenic seed as defined above wherein exogenous DNA comprising a gb1 coding sequence has a nucleotide sequence of SEQ ID NO:19.

The transgenic seed as defined above, wherein said exogenous DNA comprising a gb1 coding sequence is operably linked to a promoter functional in plants.

The transgenic seed as defined above, wherein said exogenous DNA comprises a gb1 coding sequence and a promoter sequence selected from the combination indicated in Table 2.

The transgenic seed as defined above, wherein said seed produces a plant which is a crop plant selected from the group consisting of a variety of maize, soybean, rice, wheat, canola, cotton, sorghum, tobacco and turfgrass.

The transgenic seed as defined above wherein said seed is an inbred or hybrid maize seed.

A transgenic plant grown from a transgenic seed as defined above.

The transgenic plant as defined above wherein said plant is an inbred or hybrid maize plant.

The plant as defined above wherein said transgenic plant exhibits at least a 2-fold increase in glycine-betaine as compared to a control plant lacking said exogenous DNA.

The plant ss defined above wherein said transgenic plant exhibits

a) increased tolerance to water-deficit; or
b) increased yield under water-deficit growing conditions; or
c) increased yield under non-water deficit growing conditions; or
d) increased tolerance to cold; or
e) increased tolerance to freezing, as compared to a control.

The plant as defined above wherein said plant exhibits increased cold tolerance when exposed to temperatures between about 0°C and about 10°C, wherein said exposure occurs

a) within one week of planting; or

b) within two weeks of planting; or
c) within one month of planting; or
d) within one month of harvest; or
e) because of an early planting date; or
f) because of planting in a shorter relative maturity zone.

The transgenic seed as defined above wherein said seed exhibits increased cold tolerance when exposed to temperatures between about 0°C and about 10°C, wherein said exposure occurs

a) within one week of planting; or
b) within two weeks of planting; or
c) within one month of planting; or
d) because of an early planting date; or
e) because of planting in a shorter relative maturity zone.

A method comprising propagating a transgenic crop plant from the seed as defined above. The method of propagating as defined above wherein said transgenic crop plant exhibits

a) increased tolerance to water-deficit; or
b) increased yield under water-deficit growing conditions; or
c) increased yield under non-water deficit growing conditions; or
d) increased tolerance to cold; or
e) increased tolerance to freezing, as compared to a control plant lacking said exogenous DNA.

The method as defined above wherein said transgenic crop plant exhibits increased cold tolerance when exposed to temperatures between about 0°C and about 10°C, wherein said exposure occurs

a) within one week of planting; or
b) within two weeks of planting; or
c) within one month of planting; or
d) within one month of harvest; or
e) because of an early planting date; or
f) because of planting in a shorter relative maturity zone.

The method as defined above wherein said transgenic seed exhibits increased cold tolerance when exposed to temperatures between about 0°C and about 10°C, wherein said exposure occurs

a) within one week of planting; or
b) within two weeks of planting; or
c) within one month of planting; or
d) because of an early planting date; or
e) because of planting in a shorter relative maturity zone.

A transgenic plant having in its genome an exogenous DNA comprising a promoter operably linked to a heterologous DNA, wherein said promoter exhibits promoter activity and is derived from

a) about 100 to about 350 contiguous nucleotides of DNA, wherein said contiguous nucleotides of DNA have from 85% to 100% sequence identity to about 100 to about 350 contiguous nucleotides of DNA having the sequence of SEQ ID NO:47, or
b) about 100 to about 1450 contiguous nucleotides of DNA, wherein said contiguous nucleotides of DNA have from 85% to 100% sequence identity to about 100 to about 1450 contiguous nucleotides of DNA having the sequence of SEQ ID NO:56.

A DNA construct comprising a promoter operably linked to a heterologous DNA wherein said promoter is derived from

a) about 100 to about 350 contiguous nucleotides of DNA, wherein said contiguous nucleotides of DNA have from 85% to 100% sequence identity to about 100 to about 350 contiguous nucleotides of DNA having the sequence of SEQ ID NO:47, or

b) about 100 to about 1450 contiguous nucleotides of DNA, wherein said contiguous nucleotides of DNA have from 85% to 100% sequence identity to about 100 to about 1450 contiguous nucleotides of DNA having the sequence of SEQ ID NO:56.

## Claims

**1.** A DNA construct comprising a promoter operably linked to a heterologous DNA, wherein said promoter exhibits promoter activity and is derived from about 100 to about 350 contiguous nucleotides of DNA, wherein said contiguous nucleotides of DNA have from 85% to 100% sequence identity to about 100 to about 350 contiguous nucleotides of DNA having the sequence of SEQ ID NO:47.

Fig. 1. Peptide alignment. Maize GB1 (SEQ ID NO:1); Maize GB1-2 homolog (SEQ ID NO:2); Rice GB1-1 homolog (SEQ ID NO:3); Barley GB1-1 homolog (SEQ ID NO:4); Consensus (SEQ ID NO:17)

```
                       1                                                50
Maize GB1       (1)  MIPYATAAEAEGALGRTMTWAETAWYEYSAVMPDSWLHCHTTFILFVLYS
Maize GB1-2     (1)  MMPYGTAAEAEAALGRSMTWAEALWFRYSAGMPDLCLTWHVSLVYLVLYA
Rice GB1-1      (1)  MLPYATAAEAEAAVGRGLTWAEAAWFRYSAAIPDYCLYCHNVPILLLVYT
Barley GB1-1    (1)  MLPYATTGDAEAALGRALTWAEAAWLRYSASVPDRYLHWPNIAITLVVYT
Consensus       (1)  MXPYXTXXXAEXAXGRXXTWAEXXWXXYSAXXPDXXL
                     51                                               100
Maize GB1      (51)  TAPLPLLLLEQFAPSVVLPYKLQPRVRLPP--AASLSCYMDAACIFPLAV
Maize GB1-2    (51)  LVPLPVMVIQKLAPGYALRHKLQPGVPEPSPVSTYVEYIRDSRGVTLAAL
Rice GB1       (51)  LAPLPLALLELRR-HLPLPHKLQPGVRHPP--AAFLRCYAATARVLLLAV
Barley GB1     (51)  LAPLPLALFDLAAPAVAAPYKLQPKVQHPP--ATFFRCYMDAVRVSLLII
                     101                                              150
Maize GB1      (99)  GLQFVSYPAVAKILRTRMGLPLPSVRETIAQLVVYSLVEDYLSYWMHRLL
Maize GB1-2   (101)  GPFPLIYSIAFKLFGVRTGLPLPSVWETATHLAVYSLVEDYTSYWLHRFL
Rice GB1       (98)  GPVQLASFPAVRAVGIRTGLPLPSAGETAAQVAVYLLVEDYLGYWIHRLL
Barley GB1     (99)  GPYQLISYPAAKIMDIRTGLPLPSMGEIAAQLTVYFLVEDYLNYWLHRLL
                     151                                              200
Maize GB1     (149)  HTQWCYEKIHRVHHEFTAPTGFAMSYSHWAENVVLSIPALAGPVLVPCHV
Maize GB1-2   (151)  HTRWGYEKTHRVHHEKTAPSGFAAAYATGTELSLYLTTLFLGPAIVPSHV
Rice GB1      (148)  HTPWAYHHIHRVHHEFTAPMGYAAPYAHWAEILILGFPAFAGPAIVPCHM
Barley GB1    (149)  HTKWCYEKTHHVHHEFTAPMAYAAWYGHWAEMLILAXPSLAGPALVPCHV
                     201                                              250
Maize GB1     (199)  TTQWLWFSIRLIEGINTHSGYHFPFSPCRLIPFYGGAAYHDYHHYAGGRS
Maize GB1-2   (201)  TTHWLLFSIRIMEAFDTHSGYHFPFSLARFIPFYGGAEFHDYHHYAGEKT
Rice GB1      (198)  TTFWLWEVLRHLEAIHIHSGFKLPFDPTKYIPLYGGVEYHDYHHFVGGHS
Barley GB1    (199)  TTLWIWFAARLVESLNIHSGFKLPFNAEKYIPFYGGAEHHDYHHYIGGQS
                     251                                              300
Maize GB1     (249)  QSNFAPLFTYCDYLYRTDKGYRYHKLKQEKLKSLAENSADKGGNYSFDEG
Maize GB1-2   (251)  RSNFSSVFTYCDYIYGTNKGYMYHKRSLAELKTKEAEHSGKED-------
Rice GB1      (248)  QSNFSSVETFCDYIYGTDRGYRYHKASLSKMRIFVRA-------------
Barley GB1    (249)  KSNFAPVFTYCDYIYGTDKGYRYHKATLAKLKELAGNEVQKGVDNGFNSG
                     301
Maize GB1     (299)  KKNRYFCA
Maize GB1-2   (294)  --------
Rice GB1      (285)  --------
Barley GB1    (299)  KQE-----
```

Figure 2. Peptide alignment. maize GB1 (maize GB1; SEQ ID NO:1); maize GB1-2 homolog (SEQ ID NO:2); rice GB1-1 homolog (SEQ ID NO:3); barley GB1-1 homolog (SEQ ID NO:4); maize GB1-3-1 homolog (SEQ ID NO:5); Leek GB1-3-1 homolog (SEQ ID NO:6); *Arabidopsis thaliana* GB1-3-1 homolog (At GB1-3-1; SEQ ID NO:7); *Arabidopsis thaliana* GB1-3-2 homolog (At GB1-3-2; SEQ ID NO:8); *Arabidopsis thaliana* GB1-3-3 homolog (At GB1-3-3; SEQ ID NO:9); *Arabidopsis thaliana* GB1-3-4 homolog (At GB1-3-4; SEQ ID NO:10); *Brassica napus* GB1-3-1 homolog (Bn GB1-3-1; SEQ ID NO:11); soybean GB1-3-1 homolog (soy GB1-3-1; SEQ ID NO:12); soybean GB1-3-2 homolog (soy GB1-3-2; SEQ ID NO:13); barley GB1 homolog (SEQ ID NO:14); rice GB1-3-1 homolog (SEQ ID NO:15); wheat GB1-3-1 homolog (SEQ ID NO:16); Consensus (SEQ ID NO:18).

```
                      1                                           45
       Maize GB1  (1) MIPYATAAEAEGALGRTMTWAETAWYEYSAVMPDSWLHCHTTFIL
     Maize GB1-2  (1) MMPYGTAAEAEAALGRSMTWAEALWFRYSAGMPDLCLTWHVSLVY
      Rice GB1-1  (1) MLPYATAAEAEAAVGRGLTWAEAAWFRYSAAIPDYCLYCHNVPIL
    Barley GB1-1  (1) MLPYATTGDAEAALGRALTWAEAAWLRYSASVPDRYLHWPNIAIT
   Maize GB1-3-1  (1) MLPYATAAEAEAALGRPMTPAEALWFRYTAGVSDYHLYCCNILFL
    Leek GB1-3-1  (1) MIPYPSLTAAEAALNRPLTYAETIWFNYSATIPDPLLYYHNTIFL
      At GB1-3-1  (1) MIPYATVEEASIALGRNLTRLETLWFDYSATKSDYYLYCHNILFL
      At GB1-3-2  (1) MIPYATIEEASIALSRNLTWLETLWFDYSATKSDYYLYCHNILFL
      At GB1-3-3  (1) MIPYPTVEDASVALGRNLTWFETVWFDYSATKSNEHVYCHTILVL
      At GB1-3-4  (1) MIPYATIEEASIALSRNLTWLETLWFDYSATKSDYYLYCHNILFL
      Bn GB1-3-1  (1) MIPYATIEEASLALGRNLTTLETLWFDYSATKSDYYLYCHNILFL
     soy GB1-3-1  (1) MLPYASIPEAVAALGRNLTFAETLWFNYSAAKSDYFLYCHNILFL
     soy GB1-3-2  (1) MLPYHTLEGAQVALGRGLTLAETIWFKYSANKPDFVLHCHNTLFL
  barley GB1-3-1  (1) MLPWATAAEAEAALGRPMTPAEALWFRWTAGTPDYGLYCLNILFL
    rice GB1-3-1  (1) MLPYATAAEAEAALGRAMTAAESLWFRYSAGIPDYVLFWHNILFL
   wheat GB1-3-1  (1) MLPWATAAEAEAALERAMTAAEALWFRWTAEASDYYLYCLNILFL
       Consensus  (1) MXPXXXXXXAXXAXXRXXTXXEXXWXXXXA
                      46                                          90
       Maize GB1 (46) FVIYSIAPLPLLLLEQFAP--SVVLPYKLQPRVR--LPPAASLSC
     Maize GB1-2 (46) LVLYALVPLPVMVIQKLAP--GYALRHKLQPGVPEPSPVSTYVEY
        Rice GB1 (46) LLVYTLAPLPLALLELRR---HLPLPHKLQPGVR--HPPAAFLRC
      Barley GB1 (46) LVVYTLAPLPLALFDLAAP--AVAAPYKLQPKVQ--HPPATFFRC
   Maize GB1-3-1 (46) FVVFTVAPLPIALLELRAP--AAVSPYKLQPRVR--LSRAEFVRC
    Leek GB1-3-1 (46) FVIFTLVPLPLALLELYWP--SVLKPFKIQPKVY--LSKSEFLEC
      At GB1-3-1 (46) FLVFSLVPLPLVFVELARSASGLFNRYKIQPKVN--YSLSDMFKC
      At GB1-3-2 (46) FLIFSLVPLPLVFIESSQSTSDLFNRYKIQPKVK--NSFSSMFKC
      At GB1-3-3 (46) FLVFSLAPFPLVIVEWT----GWFDQFKIQKKVK--YSLSDMFQC
      At GB1-3-4 (46) FLIFSLVPLPLVLIESAQSTSDLFNRYKIQPKVK--NSFSSMLKC
      Bn GB1-3-1 (46) FLIFSLVPLPLVFVELARSASGWFDRYKIQPKVK--NSFSDMFRC
     soy GB1-3-1 (46) FLVFSLVPLPLVFLEFKR--FSFVSSHKIQPKVR--LSLAETFKC
     soy GB1-3-2 (46) CLFYSIAPIPFVLMELSG--YEKLNKHKIQPSVK--RSFKEMFKC
  barley GB1-3-1 (46) LLVFTLAPLPVALLELRAP--RAVGPYKLQPRVR--LSRADFLKC
    rice GB1-3-1 (46) FVVFTLAPLPVALLELRAP--AAVGPFKLQPKVR-LSREEFFRC
   wheat GB1-3-1 (46) LVVFTLAPLPVALLELRAP--RAVGPYKLQPRVR-LSRAEFIKC
```

```
                         91                                          135
      Maize GB1  (87)   YMDAACIFPLAVGLQFVSYPAVAK----------ILRTRMGLPLP
    Maize GB1-2  (89)   IRDSRGVTLAALGPFPLIYSIAFK----------LFGVRTGLPLP
       Rice GB1  (86)   YAATARVLELAVGPVQLASFPAVR----------AVGIRTGLPLP
     Barley GB1  (87)   YMDAVRVSLLLIGPYQLISYPAAK----------TMDIRTGLPLP
  Maize GB1-3-1  (87)   YKDVLRIFFLVIGPLQLVSYPAVK----------FVGIHTKLPLP
   Leek GB1-3-1  (87)   YKNVIKVFFLVVCPLQLLSYPTVK----------FVGIRTGLPLP
     At GB1-3-1  (89)   YKDVMTMFILVVGPLQLVSYPSIQ----------MIEIRSGLPLP
     At GB1-3-2  (89)   YKDVMKMFILVVGPLQLVSYPSIQVDFVFRVLKQMIEIRSGLPLP
     At GB1-3-3  (85)   YKEVMKLFLLVVGTLQIVSYPSIQ----------MVGIRSGLPLP
     At GB1-3-4  (89)   YKDVMKMFILVVGPLQLVSYPSIQ----------MIEIRSGLPLP
     Bn GB1-3-1  (89)   YRDVMKMFILVVGPLQLVSYPSIQ----------MIEIRSGLPLP
    soy GB1-3-1  (87)   YKDVMRMFFLVVGPLQLISYPSIQ----------MIGIRTGLPLP
    soy GB1-3-2  (87)   YKDVMETFVIAVSPLQIISYPTIK----------WIGIRTGLSLP
 barley GB1-3-1  (87)   YGDVMRIFFLVIGPLQLVSYPAVK----------MVGIHTGLPLP
   rice GB1-3-1  (87)   YRDVMRIFFLVIGPLQLVSYPTVK----------MVGIHTGLPLP
  wheat GB1-3-1  (87)   YGDVMRIFFLVIGPLQLVSYPAVK----------MVGIHTGLPLP
                         136                                         180
      Maize GB1  (122)  SVRETIAQLVVYSLVEDYLSYWMHRLLHTQWCYEKIHRVHHEFTA
    Maize GB1-2  (124)  SVWETATHLAVYSLVEDYTSYWLHRFLHTRWGYEKIHRVHHEKTA
       Rice GB1  (121)  SAGETAAQVAVYLLVEDYLGYWIHRLLHTPWAYHHIHRVHHEFTA
     Barley GB1  (122)  SMGEEIAAQLTVYFLVEDYLNYWLHRLLHTKWCYEKIHHVHHEFTA
  Maize GB1-3-1  (122)  SLAELAAQLLVYFLVEDYLNYWIHRFLHGEWGYQNIHRVHHEFTA
   Leek GB1-3-1  (122)  SVWEVASQLAVYFLLEDFGNYWIHRWLHGKWGYEKIHKVHHEYTA
     At GB1-3-1  (124)  TITEMLSQLVVYFLIEDYTNYWVHRFFHSKWGYDKIHRVHHEYTA
     At GB1-3-2  (134)  SCMEIVAQLVVYFLVEDYTNYWVHRFFHCKWGYEKFHHIHHEYTA
     At GB1-3-3  (120)  SLMEIVAQLVVYFLIEDYTNYWIHRWMHCKWGYEKIHRIHHEYTS
     At GB1-3-4  (124)  SCMEIVAQFVVYFLVEDYTNYWVHRFFHCKWGYEKFHHIHHEYTA
     Bn GB1-3-1  (124)  SFGEIAAQLVVYFLVEDYTNYWVHRFFHSKWGYEKIHHIHHEYTA
    soy GB1-3-1  (122)  SWREILSQLLVYFLVEDYTNYWIHRFLHNDWGYEKIHRVHHEYHA
    soy GB1-3-2  (122)  SGWELFWQLFIYFVIEDFSNYWIHRMLHCKWAFEKIHKVHHEYVA
  barleyGB1-3-1  (122)  SLGEMAAQLVVYFLVEDYLNYWIHRLLHGEWGYEKIHRIHHEYTA
   rice GB1-3-1  (122)  SLGEMAAQLIVYFLVEDYLNYWIHRLLHGEWGYEKIHRVHHEFTA
  wheat GB1-3-1  (122)  SLGEMAAQLLVYFLVEDYLNYWIHRLLHGEWGYEKIHRIHHEYTA
                         181                                         225
      Maize GB1  (167)  PTGFAMSYSHWAENVVLSIPALAGPVLVPCHVTTQWLWFSIRLIE
    Maize GB1-2  (169)  PSGFAAAYATGTELSLYLTTLFLGPAIVPSHVTTHWLLFSIRIME
       Rice GB1  (166)  PMGYAAPYAHWAEILILGFPAFAGPAIVPCHMTTFWLWFVLRHLE
     Barley GB1  (167)  PMAYAAWYGHWAEMLILAXPSLAGPALVPCHVTTLWIWFAARLVE
  Maize GB1-3-1  (167)  PIGFAAPYAHWAEVLILGIPSFVGPAIVPGHMITFWLWIILRQVE
   Leek GB1-3-1  (167)  PIGFAAPYAHWAEVLILGIPSFLGPAIVPGHMITLWLWIALRQIE
     At GB1-3-1  (169)  PIGYAAPYAHWAEVLLLGIPTFMGPAIAPGHMITFWLWIALRQME
     At GB1-3-2  (179)  PIGYAAPYAHWAEVLLLGIPTFLGPAIAPGHMITFWLWIALRQIE
     At GB1-3-3  (165)  PIGYASPYAHWAEILILGIPTFLGPAIAPGHIMTFWLWISLRQFE
     At GB1-3-4  (169)  PIGYAAPYAHWAEVLLLGIPTFLGPAIAPGHMITFWLWIALRQIE
     Bn GB1-3-1  (169)  PIGYAAPYAHWAEVLLLGVPTFLGPAIAPGHMITFWLWIALRQIE
    soy GB1-3-1  (167)  PIGFAAPYAHWAEILILGIPSFLGPAMVPGHIITFWLWIALRQIE
    soy GB1-3-2  (167)  PIGLSAPYAHWAEIIILGIPXFLGPALVPGHITTYWLWFILRQLE
  barleyGB1-3-1  (167)  PIGFAAPYAHWAEVLILGIPSFAGPAIAPGHMITFWLWIILRQME
   rice GB1-3-1  (167)  PIGFAAPYAHWAEVLILGIPSFVGPALAPGHMITFWLWIVLRQME
```

```
wheat GB1-3-1 (167) PIGFAAPYAHWAEVLILGIPSFAGPAIAPGHMITFWLWIILRQME
                    226                                         270
    Maize GB1 (212) GINTHSGYHFPFSPCRLIPFYGGAAYHDYHHYAGGRSQSNEAPLF
  Maize GB1-2 (214) AFDTHSGYHFPFSLARFIPFYGGAEEHDYHHYAGEKTRSNFSSVF
     Rice GB1 (211) AIHIHSGEKLPFDPTKYIPLYGGVEYHDYHHEVGGHSQSNESSVF
   Barley GB1 (212) SLNIHSGEKLPFNAEKYRPFYGGAEHHDYHHYIGGQSKSNEAPVF
Maize GB1-3-1 (212) AIETHSGEDFPFTPTKYIPFYGGAEYHDYHHYVGGQSQSNEASVF
 Leek GB1-3-1 (212) AEDTHSGYDFPLSFTKEIPFYGGAEYHDYHHYVGGQSQSNEASVF
   At GB1-3-1 (214) AIETHSGYDFPWSPTKYIPFYGGAEYHDYHHYVGGQSQSNEASVF
   At GB1-3-2 (224) AIETHSGYDFPWSLTKYIPFYGGAEYHDYHHYVGGQSQSNEASVF
   At GB1-3-3 (210) AIETHSGYDFPWSVTKLIPFYGGPEYHDYHHYVGGQSQSNEASVF
   At GB1-3-4 (214) AIETHSGYDFPWSLTKYIPFYGGAEYHDYHHYVGGQSQSNEASVF
   Bn GB1-3-1 (214) AIETHSGYDFPWTLTKFIPFYGGAEYHDYHHYVGGQSQSNEASVF
  soy GB1-3-1 (212) AIDTHSGYDFPRSITKYIPFYGGAEYHDYHHYVGRQSQSNEASVF
  soy GB1-3-2 (212) AIETHSGYDFSWEXTKYIPFYGGPAYHDYHHYVGGKSQSNEAS--
barleyGB1-3-1 (212) AIDTHSGFDFPFSLTKYIPFYGGAESHDYHHYVGGQSQSIEASVF
  rice GB1-3-1 (212) AIETHSGFDFPFNLTKYLPFYGGAEYHDYHHYVGRQSQSNEASVF
wheat GB1-3-1 (212) AIDTHSGFDFPFSLTKYIPFYGGAESHDYHHYVGGQSQSNEASVF
                    271                                         315
    Maize GB1 (257) TYCDYLYRTDKGYRYHKLKQEKLKSLAENSADKGGNYSFDEGKKN
  Maize GB1-2 (259) TYCDYIYGTNKGYMYHKRSLAEIKTKEAEHSGKED----------
     Rice GB1 (256) TFCDYIYGTDRGYRYHKASLSKMRIFVRA----------------
   Barley GB1 (257) TYCDYIYGTDKGYRYHKATLAKLKELAGNEVQKGVDNGFNSGKQE
Maize GB1-3-1 (257) TYCDYLYGTDKGYRFHKTYLAKLKDLGHNDGQKGDGSGPSYVKLD
 Leek GB1-3-1 (257) TYCDYVYGTDKGYRYRKACLSMMKEESENQNGVENSFQNQKSD--
   At GB1-3-1 (259) TYCDYIYGTDKGYRFQKKLL-EQIKESS--KKSNKHNGGIKSD--
   At GB1-3-2 (269) TYCDYIYGTDKGYRFQKKLLQQVNKYSIN----------------
   At GB1-3-3 (255) TYCDYIYGTDKGYRIHKKLLHHQIKEEAEEKRVRKHD--------
   At GB1-3-4 (259) TYCDYIYGTDKGYRFQKKLLQQMKEKSKKSNKLVNGGEKFD----
   Bn GB1-3-1 (259) TYCDYIYGTDKGYRFQKKFLQQIKQESKKSN-MQNGGDKLD----
  soy GB1-3-1 (257) TYCDYIYGTDKGYRYQKKILQKLKEELANGVEQNGGLYKTD----
  soy GB1-3-2 (255) ---------------------------------------------
barleyGB1-3-1 (257) TYCDPLCGTDRGYRFHRASLPMLRALAPPAAKKDAPMGFSSAKGD
  rice GB1-3-1 (257) TYCDYLYGTDKGYRYHKAYQAKMKALGQTEGEKADSNGLSYAKLD
wheat GB1-3-1 (257) TYCDYLYGTDRGYRFHKAYLAKLKDLAPSDGEKEGADGFAYAKLD
                    316
    Maize GB1 (302) RYFCA
  Maize GB1-2 (294) -----
     Rice GB1 (285) -----
   Barley GB1 (302) -----
Maize GB1-3-1 (302) -----
 Leek GB1-3-1 (300) -----
   At GB1-3-1 (299) -----
   At GB1-3-2 (298) -----
   At GB1-3-3 (292) -----
   At GB1-3-4 (300) -----
   Bn GB1-3-1 (299) -----
  soy GB1-3-1 (298) -----
  soy GB1-3-2 (255) -----
barleyGB1-3-1 (302) YVVL-
  rice GB1-3-1 (302) -----
wheat GB1-3-1 (302) -----
```

Figure 3. pMON78450

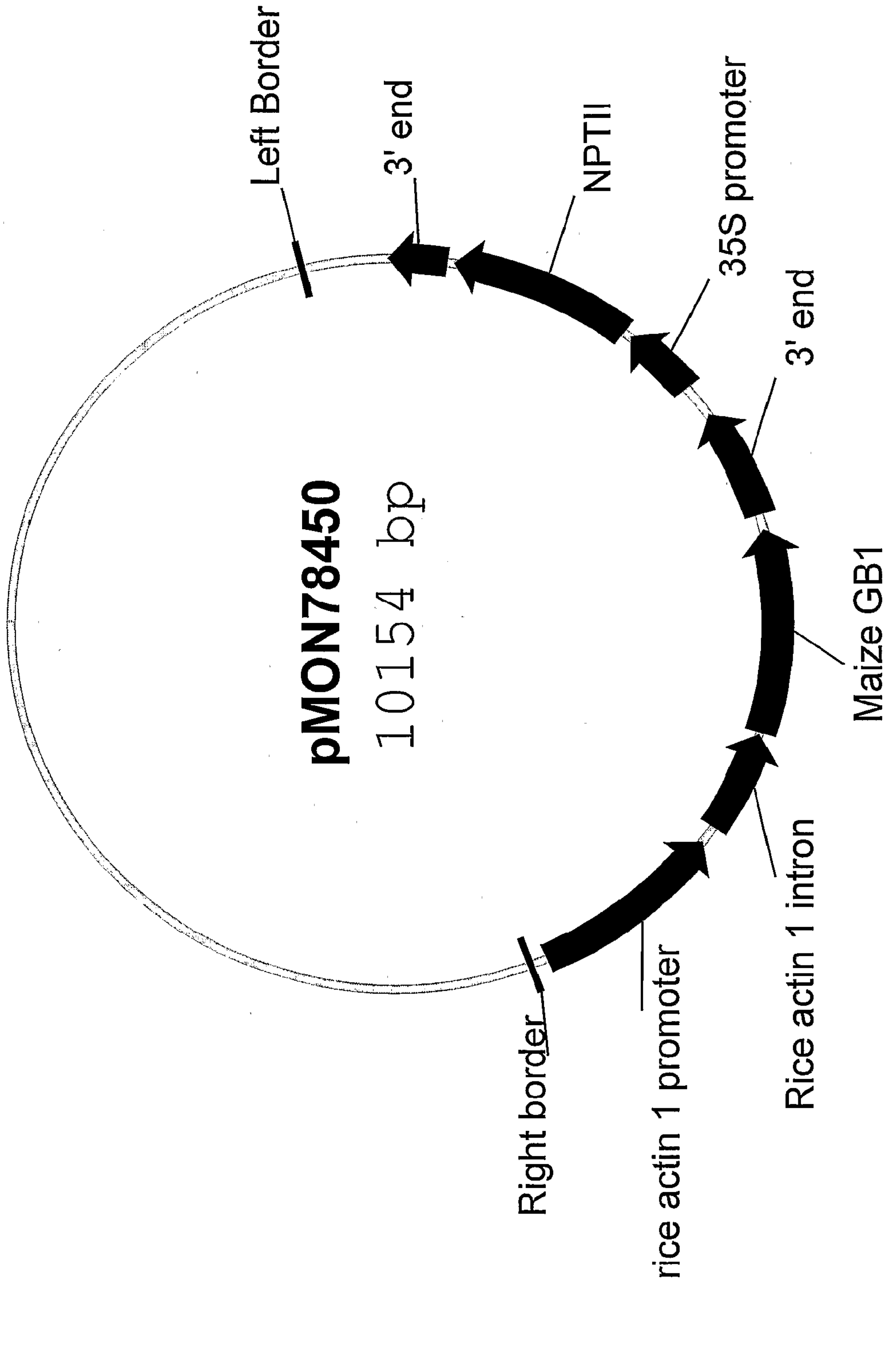

European Patent Office

# EUROPEAN SEARCH REPORT

Application Number
EP 07 11 8354

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | DATABASE EMBL 19 June 2003 (2003-06-19), XP002465351<br>Database accession no. CC607410<br>* abstract *<br>----- | | INV.<br>C12N15/82 |
| A | DATABASE EMBL 30 August 2003 (2003-08-30), XP002465352<br>Database accession no. CG357706<br>* abstract *<br>----- | | |
| P,X | LIU J ET AL: "Plant full length insert polypeptide seqid 52139"<br>GENESEQ, 21 April 2005 (2005-04-21), XP002434803<br>* abstract * | 1 | |
| P,X | -& US 2004/034888 A1 (LIU JINGDONG [US] ET AL) 19 February 2004 (2004-02-19)<br>----- | 1 | |
| | | | TECHNICAL FIELDS SEARCHED (IPC) |
| | | | C12N |

The present search report has been drawn up for all claims

| Place of search | Date of completion of the search | Examiner |
|---|---|---|
| Munich | 21 January 2008 | Schneider, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 07 11 8354

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2008

| Patent document cited in search report | Publication date | Patent family member(s) | Publication date |
|---|---|---|---|
| US 2004034888 A1 | 19-02-2004 | US 2004031072 A1 | 12-02-2004 |
| | | US 2006236419 A1 | 19-10-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 46791003 P
- US 60487273 B
- WO 9901032 A
- US 5952267 A
- US 6310271 B
- WO 9826801 A
- US 20020123118 A1
- US 6281411 B
- US 5888732 A
- US 6277608 B
- US 2001283529 A
- US 2001282319 A
- US 20020007051 A
- US 6437217 B
- US 5641876 A
- US 6426446 B
- US 6429362 B
- US 6232526 B
- US 6177611 B
- US 5322938 A
- US 5352605 A
- US 5359142 A
- US 5530196 A
- US 6433252 B
- US 6429357 B
- US 5837848 A
- US 6294714 B
- US 6140078 A
- US 6252138 B
- US 6175060 B
- US 078972 A
- US 757089 A
- US 200301403641 A
- US 739565 A
- US 547761 P
- US 6084089 A
- US 463974 P
- US 5824857 A
- US 4959317 A
- US 5527695 A
- US 5188642 A
- US 5550318 A
- US 5633435 A
- US 5780708 A
- US 6118047 A
- US 5015580 A
- US 5538880 A
- US 6160208 A
- US 6399861 B
- US 6403865 B
- US 5635055 A
- US 5824877 A
- US 5591616 A
- US 5981840 A
- US 6384301 B
- US 6194636 B
- US 6479733 B
- US 20010051335 A1


### Non-patent literature cited in the description


- **VILARDELL et al.** *Plant Molecular Biology,* 1990, vol. 17 (5), 985-993
- **OULLET, F et al.** *FEBS Letters,* 1998, vol. 423, 324-328
- **KIRCH, H et al.** *Plant Mol Biol,. Mar,* 1997, vol. 33 (5), 897-909
- **SHEN, Q et al.** *Plant Mol Biol., Feb,* 2001, vol. 45 (3), 327-40
- **BAKER, S et al.** *Plant Mol Biol. Mar,* 1994, vol. 24 (5), 701-13
- **WANG H. et al.** *Plant Mol Biol. Jul,* 1995, vol. 28 (4), 605-17